# EUROPEAN PATENT APPLICATION

(11) **EP 1 498 104 A1**
(43) Date of publication of application: **19.01.2005**
(21) Application number: 03717623.7
(22) Date of filing: 17.04.2003
(51) Int. Cl.: A61K 7/06, A61K 45/00, A61P 17/14, C07D 271/08, C07D 313/00, C07D 493/04, C07D 493/06, C07D 493/08, C07D 493/16

(54) **HAIR GROWTH TONIC**

(30) Priority: 17.04.2002 JP 2002115529
(71) Applicant: TAISHO PHARMACEUTICAL CO., LTD, Tokyo 170-8633 (JP)
(72) Inventor: IKEDA, Akiko c/o TAISHO PHARMACEUTICAL CO., LTD., Toshima-ku, Tokyo 170-8633 (JP); SHINONAGA, Hideki TAISHO PHARMACEUTICAL CO., LTD., Toshima-ku, Tokyo 170-8633 (JP); FUJIMOTO, Natsuko TAISHO PHARMACEUTICAL CO., LTD., Toshima-ku, Tokyo 170-8633 (JP); KASAI, Yoko c/o TAISHO PHARMACEUTICAL CO., LTD., Toshima-ku, Tokyo 170-8633 (JP)
(74) Representative: HOFFMANN - EITLE
(86) International application number: PCT/JP2003/004884
(87) International publication number: WO 2003/086334

(57) **Abstract**

A dermal papilla cell growth promoter, a hair growth stimulant and a hair growth tonic containing a compound having an activity of inhibiting the function of WNT-5A.

## Description

### TECHNICAL FIELD

The present invention relates to a dermal papilla cell growth promoter, a hair growth stimulant and a hair growth tonic. More specifically, it relates to a dermal papilla cell growth promoter, a hair growth stimulant and a hair growth tonic comprising a WNT-5A function inhibitor as an active ingredient. Furthermore, the present invention relates to a method of screening a dermal papilla cell growth promoter based on the effect of inhibiting the function of WNT-5A.

### BACKGROUND ART

Human hair follicles are made up of epithelial and mesenchymal dermal cells such as keratinocytes, dermal papilla cells, fibroblasts and sebaceous cells and hair growth cycle (hair cycle) is controlled via interactions among these cells. The hair shaft is formed by the proliferation/differentiation (keratinization) of follicular keratinocytes. Dermal papillae regulate the proliferation, differentiation and apoptosis of these follicular keratinocytes and thus play a key role in the control of the hair cycle. In developing a hair growth stimulant/a hair growth tonic, therefore, it would be important to study effects on dermal papilla cells. However, molecular mechanisms regulating the proliferating ability and hair-cycle controlling ability of dermal papilla cells have been scarcely clarified so far.

On the other hand, WNT-5A is a secretory glycoprotein belonging to the WNT family. The WNT family includes about 20 kinds of molecules which are conserved over a wide range from nematodes to mammals. It is known that these WNTs are important intercellular signaling molecules which regulate axis formation and organ formation during the fetal stage *(Annu. Rev. Cell Dev. Biol.,* 14, 59-88 (1998); *Genes & Dev.* 11, 3286-3305 (1997)). In humans, there are 10 kinds of WNT receptors that are Frizzled seven-pass transmembrane receptors *(Annu. Rev. Cell Dev. Biol.,* 14, 59-88 (1998), *Genes & Dev.,* 11, 3286-3305 (1997)). Depending on WNT-Frizzled bond combinations, there exist 3 kinds of signal transduction pathways (i.e., WNT/β-catenin pathway, PCP pathway and WNT/Ca²⁺ pathway) *(Annu. Rev. Cell Dev. Biol.,* 14, 59-88 (1998)).

In recent years, it is clarified that the WNT/β-catenin pathway is fundamental to the formation of hair follicles *(Genes & Dev.,* 8, 2691-2703 (1994); *Cell,* 95, 605-614 (1998); *Dev. Biol.,* 207, 133-149 (1999); *Genes & Dev.,* 14, 1181-1185 (2000); *Cell,* 105, 533-545 (2001)). In 1988, a stabilized β-catenin transgenic mouse in the skin was constructed. It is reported that this mouse showed increased *de novo* follicular morphogenesis and led to hypertrichosis *(Cell,* 95, 605-614 (1998)). It was reported in 2000 that the WNT/β-catenin signal plays an important role in maintaining the hair-inducing activity of dermal papilla *(Genes & Dev.,* 14, 1181-1185 (2000)).

However, it has been clarified that signal transduction from WNT-5A is mediated not by the WNT/β-catenin pathway but by the Ca²⁺ pathway *(Dev. Biol.,* 182, 114-120 (1997); *Curr. Biol.,* 9, 695-698 (1999)) and nothing has been reported so far with respect to the relationship between WNT-5A and follicle morphogenesis.

It is furthermore reported that duplication of the embryonic axis is induced by injecting *Xenopus laevis* WNT-5A mRNA together with human Frizzled 5 mRNA into an early *of Xenopus laevis* embryo *(Science,* 275, 1652-1654 (1997)) while the axis duplication induced by the injection of WNT-1 or WNT-8 mRNA is inhibited by WNT-5A *(J. Cell Biol.,* 133, 1123-1137 (1996)), and that *Xenopus laevis* WNT-5A binds to rat Frizzled 2 and activates CamKII (Ca²⁺/calmodulin-dependent protein kinase II) and PKC (protein kinase II) via the Ca²⁺ pathway *(Curr. Biol.,* 9, 695-698 (1999)). However, physiological meanings thereof still remain unknown and nothing has been reported so far with respect to the relationship between WNT-5A and hair growth stimulation/hair growth promotion.

### DISCLOSURE OF THE INVENTION

An object of the present invention is to provide a screening method using a molecule regulating the proliferation of dermal papilla cells, a dermal papilla cell growth promoter and a hair growth stimulant and a hair growth tonic based on a novel function.

In order to achieve these objects, the present inventors conducted intensive studies. As a result, they have found that WNT-5A is highly expressed in dermal papilla cells and WNT-5A relates to the proliferating ability of dermal papilla cells. As the results of subsequent studies based on these findings, they have further found that the growth of dermal papilla cells can be remarkably promoted and the hair bulb part of follicles can be enlarged by inhibiting the function of WNT-5A, thereby completing the present invention.

That is, in one embodiment, the present invention provides a dermal papilla cell growth promoter which comprises a compound having an activity of inhibiting the function of WNT-5A.

In another embodiment, the present invention provides a dermal papilla cell growth promoter which comprises a compound having an activity of inhibiting the production of WNT-5A.

In still another embodiment, the present invention provides a compound represented by the following formula (I): 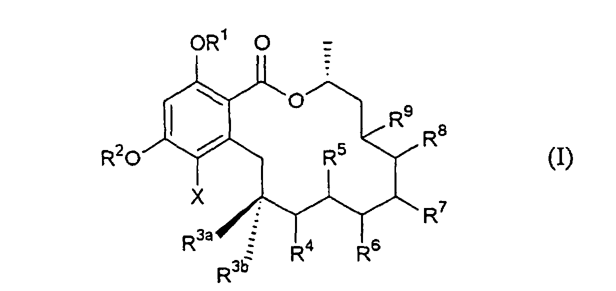 wherein R¹ and R² are the same or different and each represents a hydrogen atom, a C₁₋₆ alkyl group or a C₂₋₆ alkanoyl group;
X represents a hydrogen atom or a halogen atom;
R^{3a} and R^{3b} are the same or different and each represents a hydrogen atom or a hydroxyl group; and
R⁴, R⁵, R⁶, R⁷, R⁸ and R⁹ are the same or different and each represents a hydrogen atom, a hydroxyl group, a halogen atom or a C₂₋₆ alkanoyloxy group, or adjacent groups thereof are taken together to form a π bond or an ether bond, or R⁵ and R⁸ or R⁵ and R⁹ are taken together to form an ether bond.

In still another embodiment, the present invention provides a dermal papilla cell growth promoter which comprises a compound represented by the following formula (II): 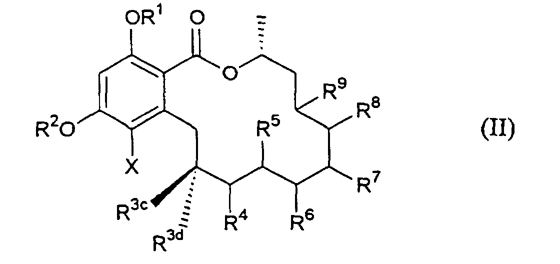 wherein R¹ and R² are the same or different and each represents a hydrogen atom, a C₁₋₆ alkyl group or a C₂₋₆ alkanoyl group;
X represents a hydrogen atom or a halogen atom;
R^{3c} and R^{3d} are the same or different and each represents a hydrogen atom, a hydroxyl group or a C₁₋₆ alkoxy group, or R^{3c} and R^{3d} are taken together to form an oxo group, a hydroxyimino group or a C₁₋₆ alkoxyimino group; and
R⁴, R⁵, R⁶, R⁷, R⁸ and R⁹ are the same or different and each represents a hydrogen atom, a hydroxyl group, a halogen atom or a C₂₋₆ alkanoyloxy group, or adjacent groups thereof are taken together to form a π bond or an ether bond, or R⁵ and R⁸ or R⁵ and R⁹ are taken together to form an ether bond.

In still another embodiment, the present invention provides a dermal papilla cell growth promoter containing a compound represented by the following formula (IX): 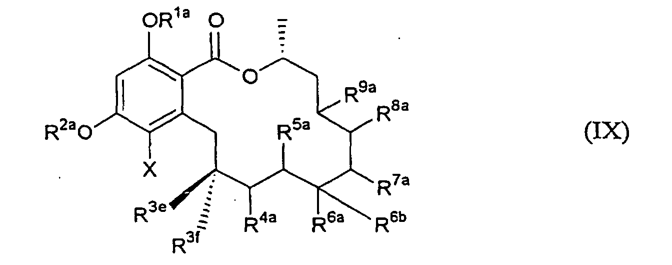 wherein R^{1a} and R^{2a} are the same or different and each represents a hydrogen atom, a C₁₋₆ alkyl group, a C₂₋₆ alkanoyl group,
a group represented by (CH₂)ₚCO-Y-R¹⁰ (wherein Y represents an oxygen atom or a sulfur atom; R¹⁰ represents a hydrogen atom, a C₁₋₆ alkyl group or a substituted or unsubstituted aryl group; and p is 0 or 1),
a group represented by (CH₂)_{q}R¹¹ (wherein R¹¹ represents a substituted or unsubstituted cycloalkyl group or a substituted or unsubstituted C₂₋₁₀ heterocycle; and q is 0 or 1), or
a group represented by COR¹² (wherein R¹² represents a substituted or unsubstituted aryl group or a substituted or unsubstituted C₂₋₁₀ heterocycle);
X represents a hydrogen atom or a halogen atom;
R^{3e} and R^{3f} are the same or different and each represents a hydrogen atom, a hydroxyl group, a C₁₋₆ alkoxy group or a C₁₋₆ alkanoyloxy group, or R^{3e} and R^{3f} are taken together to form an oxo group, a hydroxyimino group or a C₁₋₆ alkoxyimino group; and
R^{4a}, R^{5a}, R^{6a}, R^{7a}, R^{8a} and R^{9a} are the same or different and each represents a hydrogen atom, a hydroxyl group, a halogen atom or
a group represented by Z-R¹³ (wherein Z represents an oxygen atom or a sulfur atom; and R¹³ represents a C₁₋₆ alkyl group, a C₁₋₆ alkanoyl group or a substituted or unsubstituted aryl group), or
adjacent groups thereof are taken together to form a π bond or an ether bond, or
R^{5a} and R^{8a} or R^{5a} and R^{9a} are taken together to form an ether bond; and
R^{6b} represents a hydrogen atom, or is taken together with R^{3e} or R^{3f} to form an ether bond.

In still another embodiment, the present invention provides a hair growth stimulant or a hair growth tonic containing the above-described dermal papilla cell growth promoter as an active ingredient.

In still another embodiment, the present invention provides a method of screening a dermal papilla cell growth promoter, which comprises selecting a substance inhibiting the function of WNT-5A.

In still another embodiment, the present invention provides a method of selecting a substance inhibiting the function of WNT-5A that is a method of screening a dermal papilla cell growth promoter, which comprises the following steps (a) to (c):
(a) a step of culturing human WNT-5A-expressing cells in a medium to which a test compound has been added;
(b) a step of lysing the human WNT-5A-expressing cells cultured in the step (a) to extract RNA and determining the WNT-5A mRNA amount; and
(c) a step of comparing the WNT-5A mRNA amount determined in the step (b).

### BRIEF DESCRIPTION OF THE DRAWINGS

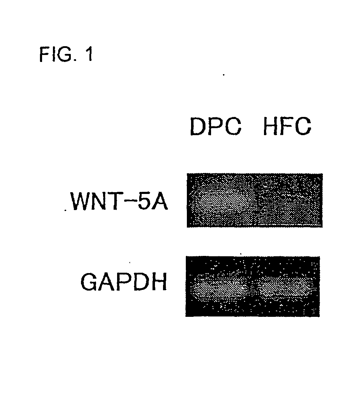

Fig. 1 shows an electrophoresis pattern demonstrating that WNT-5A mRNA is expressed in dermal papilla cells.

Fig. 2 shows an electrophoresis pattern demonstrating that test compounds cause decreases in the WNT-5A mRNA amount in dermal papilla cells.

Fig. 3 shows a graph demonstrating that test compounds have an activity of promoting the growth of dermal papilla cells.

Fig. 4 shows an electrophoresis pattern demonstrating decreases in WNT-5A mRNA amount in monkey skin organ culture.

Fig. 5 shows the results of PCNA-staining in telogen and anagen hair follicles in monkey skin organ culture.

Fig. 6 shows histograms demonstrating that Compound 7 enlarges the hair bulb diameter in monkey skin organ culture.

Fig. 7 shows histograms demonstrating that Compound 3 enlarges the hair bulb diameter in monkey skin organ culture.

Fig: 8 shows photos of the skin before and after application of test compound for 6 months in a hair growth test on a stumptailed macaque *(Macaca arctoides).*

### BEST MODE FOR CARRYING OUT THE INVENTION

Now, the present invention is described in greater detail.

### <Compound inhibiting the function of WNT-5A>

The term "a compound inhibiting the function of WNT-5A" (hereinafter sometimes referred to as " WNT-5A function inhibitor") as used in the present invention means a compound inhibiting the binding of WNT-5A to a WNT-5A receptor or a compound inhibiting the production of WNT-5A, preferably a compound inhibiting the production of WNT-5A.

It has been confirmed that WNT-5A, which is a secretory glycoprotein belonging to the WNT family, is expressed in human, mouse, rat, *Xenopus laevis* and the like. From the viewpoint of using as a drug, a compound inhibiting the function of human WNT-5A (SEQ ID NO: 1) is preferred.

The compound inhibiting the binding of WNT-5A to a WNT-5A receptor means a compound which acts on WNT-5A or the WNT-5A receptor and thus inhibits the binding of WNT-5A to the WNT-5A receptor to thereby regulate the signal transduction by WNT-5A, and is preferably a compound regulating the signal transduction via the Ca²⁺ pathway. Examples thereof include a WNT-5A receptor antagonist. Specific examples of the WNT-5A receptor include human Frizzled 5 (SEQ ID NO:4) and rat Frizzled 2 (SEQ ID NO:6). Although such a compound may be either peptidic or nonpeptidic, a nonpeptidic inhibitor is preferred because of its advantageous of having a longer duration of action. The compound can be selected by a screening system using a labeled WNT-5A and WNT-5A receptor, and has preferably an IC₅₀ of 30 µM or less, and more preferably IC₅₀ of 10 µM or less.

The compound inhibiting the production of WNT-5A means a compound inhibiting the expression of WNT-5A gene. Although such a compound may be either peptidic or nonpeptidic, a nonpeptidic inhibitor is preferred because of its advantageous of having a longer duration of action. The compound can be selected by using a decrease in the amount of WNT-5A protein (SEQ ID NO:1) or the amount of WNT-5A mRNA (SEQ ID NO:2) as an index, and has preferably an IC₅₀ of 30 µM or less, and more preferably 10 µM or less by a nucleic acid probe assay in accordance with the method of Hartley *et al. (Drug Metabolism and Disposition,* 28(5), 608-616 (2000); Test Example 4 described below).

Compounds represented by formulae (I), (II) and (IX) are particularly preferred.

In the compounds represented by formulae (I), (II) and (IX), the C₁₋₆ alkyl group means a linear or branched alkyl group having from 1 to 6 carbon atoms, and examples thereof include a methyl group, an ethyl group, a propyl group, an isopropyl group, a butyl group, an isobutyl group, a *tert*-butyl group, a pentyl group, a 2-ethylpropyl group, a hexyl group, and the like.

The C₁₋₆ alkanoyl group means a linear or branched alkanoyl group having from 1 to 6 carbon atoms, and examples thereof include an acetyl group, a propionyl group, a butyryl group, a t-butyryl group and the like.

The C₁₋₆ alkoxy group means a linear or branched alkoxy group having from 1 to 6 carbon atoms, and examples thereof include a methoxy group, an ethoxy group, a propoxy group, an isopropoxy group, a butoxy group, an isobutoxy group, a *sec*-butoxy group, a *tert*-butoxy group, a pentyloxy group, an isopentyloxy group, a neoisopentyloxy group, a tert-pentyloxy group, a 1-methylbutoxy group, a 2-methylbutoxy group, a 1,2-dimethylpropoxy group, a hexyloxy group, an isohexyloxy group and the like.

The C₁₋₆ alkoxyimino group means a linear or branched alkoxyimino group having from 1 to 6 carbon atoms, and examples thereof include an *N*-methoxyimino group, an *N*-ethoxyimino group, an *N*-propxyimino group, an *N*-isopropoxyimino group, an *N*-butoxyimino group, an *N*-isobutoxyimino group, an *N*-pentyloxyimino group, an *N*-hexyloxyimino group, and the like.

The C₁₋₆ alkanoyloxy group means a linear or branched alkanoyloxy group having from 1 to 6 carbon atoms, and examples thereof include an acetoxy group, a propionyloxy group, a pivaloyloxy group, and the like.

The C₃₋₁₀ cycloalkyl group means a cycloalkyl group having from 3 to 10 carbon atoms, and examples thereof include a cyclopropyl group, a cyclobutyl group, a cyclopentyl group, a cyclohexyl group, a cycloheptyl group, a cyclooctyl group, a cyclononyl group, and the like.

Examples of the substituted C₃₋₁₀ cycloalkyl group include a cycloalkyl group wherein a hydrogen atom(s) in the cycloalkyl group is/are substituted with at least one group selected from the group consisting of a halogen atom, an substituted or unsubstituted C₁₋₁₀ alkyl group, a hydroxyl group, a C₁₋₅ hydroxyalkyl group, a carboxyl group, a mercapto group, an indolyl group, a C₁₋₅ alkylthio group, an amino group, an amido group and a C₁₋₅ alkoxy group.

Examples of the aryl group include a phenyl group, a naphthyl group, an anthracene group and the like.

Examples of the substituted aryl group include aryl groups wherein a hydrogen atom(s) in an aryl group is/are substituted with at least one group selected from the group consisting of a substituted or unsubstituted C₁₋₁₀ alkyl group, a hydroxyl group, a C₁₋₅ hydroxyalkyl group, a carboxyl group, a mercapto group, an indazolyl group, an indolyl group, a C₁₋₅ alkylthio group, a cyano group, a nitro group, an amino group, an amido group, an acetylamino group and a C₁₋₅ alkoxy group.

Examples of the C₂₋₁₀ heterocycle include furan, pyrrole, thiophene, oxazole, isoxazole, thiazole, imidazole, pyrazole, pyran, pyridine, piperidine, pyridazine, pyrimidine, pyrazine, quinoline and the like.

Examples of the substituted C₂₋₁₀ heterocycle include a heterocycle wherein a hydrogen atom(s) in the heterocycle is/are substituted with at least one group selected from the group consisting of a halogen atom, a substituted or unsubstituted C₁₋₁₀ alkyl group, a hydroxyl group, a C₁₋₅ hydroxyalkyl group, a carboxyl group, a mercapto group, an indazolyl group, an indolyl group, a C₁₋₅ alkylthio group, a cyano group, a nitro group, an amino group, an amido group and a C₁₋₅ alkoxy group.

The ether bond means -O-, the formula -(CH₂)ₘ-O-(CH₂)ₙ- (wherein m and n each represents an integer of 1 to 3 and the alkylene groups in the formula may be substituted with an alkyl group(s)), or the formula -O-(CH₂)ₘ-O- (wherein m is an integer of 1 to 3 and the alkylene groups in the formula may be substituted with an alkyl group(s)).

Examples of the combination of the adjacent groups which form a π bond or an ether bond include (R⁴ with R⁵), (R⁵ with R⁶), (R⁶ with R⁷), (R⁷ with R⁸) and (R⁸ with R⁹) (the same applies to R^{4a} to R^{9a} in formula (IX)).

Among the compounds represented by formula (IX), a compound represented by formula (I) is preferred, and a compound wherein R^{3a} is a hydrogen atom and R^{3b} is a hydroxyl group is more preferred. From the viewpoint of the stability of compound, compounds represented by formulae (IIa), (IIb) and (IIc) are preferred.

Compound of formula (II) wherein (R⁴ and R⁵) and (R⁶ and R⁷) each form a π bond: 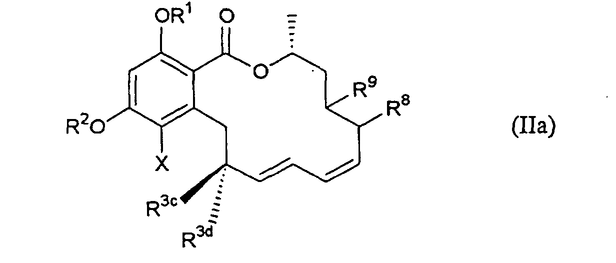 (wherein R¹, R², R^{3c}, R^{3d}, R⁸; R⁹ and X have the same meanings as described above).

Compound of formula (II) wherein (R⁴ and R⁵) form a π bond and R⁶ and R⁷ each represents a hydrogen atom: 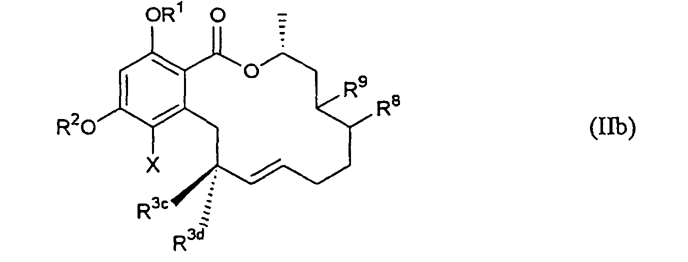 (wherein R¹, R², R^{3c}, R^{3d} , R⁸, R⁹ and X have the same meanings as described above).

Compound of formula (II) wherein R⁴, R⁵, R⁶ and R⁷ each represents a hydrogen atom: 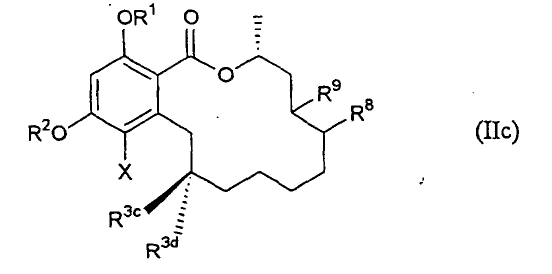 (wherein R¹, R², R^{3c}, R^{3d}, R⁸, R⁹ and X have the same meanings as described above).

Among the compounds represented by formula (IX), a compound wherein R^{3e} and R^{6b} are taken together to form an ether bond and (R^{3f} and R^{4a}) and (R^{5a} and R^{6a}) each form a π bond (e.g., Compound 77, etc.) are preferred since they effectively inhibit the expression of WNT-5A mRNA in dermal papilla cells and promote the growth of the dermal papilla cells.

Among the compounds represented by formula (IX), a compound wherein R^{1a} and R^{2a} each represents (CH₂)ₚCO-Y-R¹⁰ (wherein Y represents an oxygen atom or a sulfur atom; R¹⁰ represents a hydrogen atom, a C₁₋₆ alkyl group or a substituted or unsubstituted aryl group; and p represents 0 or 1) (e.g., Compound 52, etc.) and a compound wherein R^{1a} and R^{2a} represent each COR₁₂ (wherein R₁₂ represents a substituted or unsubstituted aryl group or a substituted or unsubstituted heterocycle) (e.g., Compound 58, *etc.)* are preferred since they effectively inhibit the expression of WNT-5A mRNA in dermal papilla cells and promote the growth of the dermal papilla cells.

Also, a compound wherein R^{7a} represents Z-R¹³ (wherein Z represents an oxygen atom or a sulfur atom; and R¹³ represents a C₂₋₆ alkanoyl group or a substituted or unsubstituted aryl group) (e.g., Compound 53, *etc.)* is preferred since they effectively inhibit the expression of WNT-5A mRNA in dermal papilla cells and promote the growth of the dermal papilla cells.

A compound represented by formula (II) can be produced by, for example, combining the following production methods. 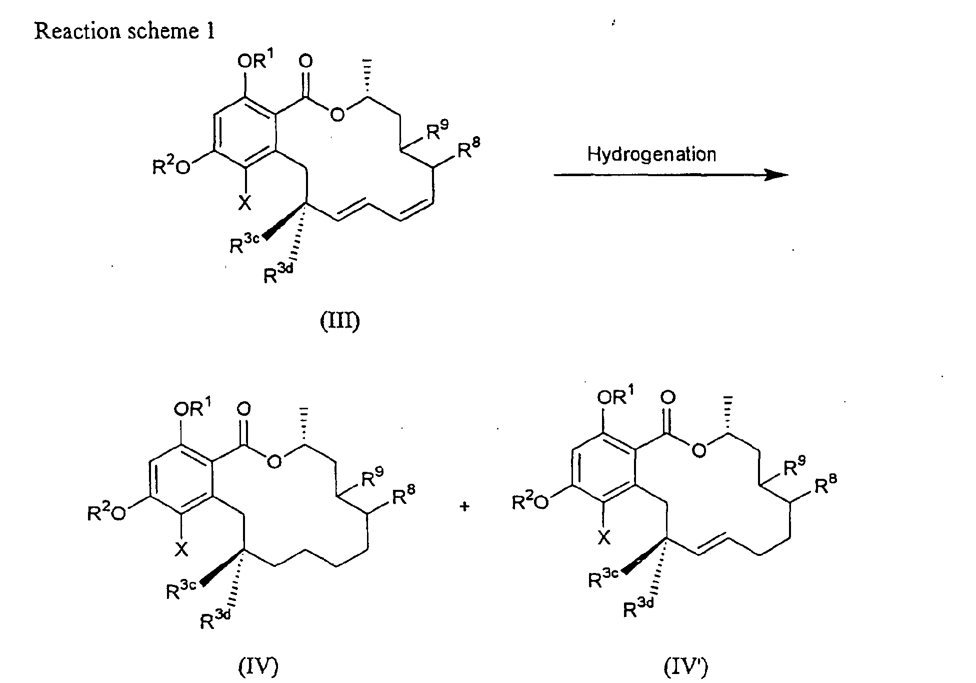 (wherein R¹, R², R^{3c}, R^{3d}, R⁸, R⁹ and X have the same meanings as described above).

The compound represented by formula (III) is subjected to a hydrogenation reaction in the presence of a catalyst such as palladium carbon in an organic solvent (e.g., ethyl acetate, tetrahydrofuran, diethyl ether, ethyl alcohol, methyl alcohol, etc.) to give the compound represented by formula (IV) or (IV') or a mixture of these compounds. The compounds of formulae (IV) and (IV') can be separated and purified by a separation method commonly employed such as column chromatography. 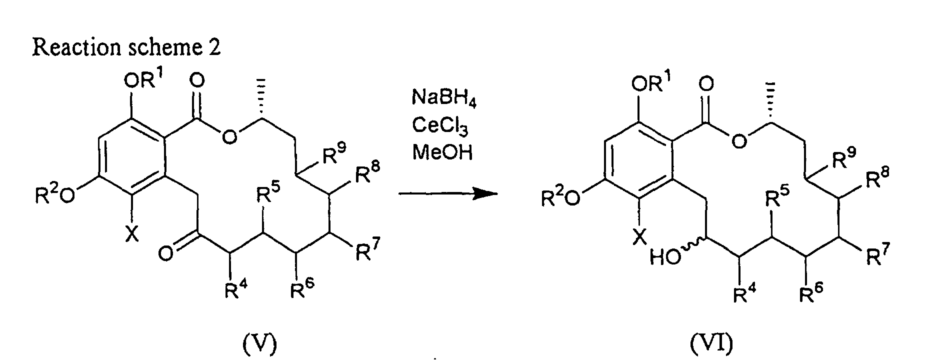 (wherein R¹, R², R⁴, R⁵, R⁶, R⁷, R⁸, R⁹ and X have the same meanings as described above).

The compound represented by formula (V) is allowed to react with a reducing agent such as potassium boron hydride, sodium boron hydride or lithium boron hydride, if necessary, in the coexistence of a salt such as LiCl, MgCl₂, CeCl₃, CaCl₂ or NiCl in an organic solvent (e.g., tetrahydrofuran, diethyl ether, ethyl alcohol, methyl alcohol, *etc.*) at -20 to 100°C, preferably at 0 to 20°C, to give the compound represented by formula (VI). The compound represented by formula (VI) can be separated and purified by a separation method commonly employed such as column chromatography. 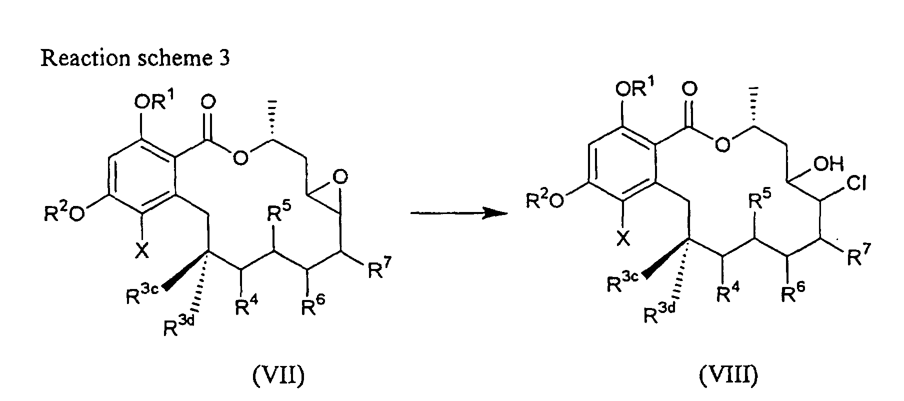 (wherein R¹, R², R^{3c}, R^{3d}, R⁴, R⁵, R⁶, R⁷ and X have the same meanings as described above).

The compound represented by formula (VII) is treated with an acid or a base in an appropriate organic solvent (e.g., tetrahydrofuran, diethyl ether, ethanol, methanol, *etc.)* to give the compound represented by formula (VIII).

The compounds represented by formula (IX) (1, 60 to 78) are produced by culturing *Pochonia chalamydosporia* var. *chlamydosporia* TF-0480 strain in a medium containing various nutrients basically in accordance with methods commonly employed for producing fermentation products.

A liquid medium is mainly used as the medium, the medium contains a carbon source, a nitrogen source and an inorganic salt optionally together with vitamins, precursors and a defoaming agent, and the pH is adjusted to around 7. The carbon source includes glucose, dextrin, glycerine, starch and the like, which may be used alone or as a mixture thereof. The nitrogen source includes meat extract, oatmeal, yeast extract, soybean flour, polypeptone, corn steep liquor, urea, an ammonium salt and the like, which may be used alone or as a mixture thereof. The inorganic salt includes monopotassium phosphate, magnesium sulfate, sodium chloride, calcium carbonate and the like, which may be used alone or as a mixture thereof. The defoaming agent which can be used include Adekanol or a silicone compound. As the culture method, aerobic culture methods such as shaken culture and aeration-agitation culture are suitable. The culture is carried out at pH 4 to 10 and at 25 to 35°C for 2 to 5 days, preferably at 25 to 28°C for 3 days.

Compounds 1 and 60 to 78 thus produced by the culture can be isolated in accordance with methods commonly employed for collecting fermentation products. For example, it is effective to employ the following methods. Namely, after the completion of the culture, a culture filtrate is obtained by centrifugation or filtration. Then, it is adsorbed by a polystyrene resin such as DIAION HP-20 (trade name, manufactured by Mitsubishi Chemical) and eluted with an organic solvent such as lower alcohol or acetone. Cells are extracted with an organic solvent such as lower alcohol or acetone. Next, the cell extract and the eluate from the adsorbent resin are combined together and concentrated under reduced pressure. To the remaining water phase, an organic solvent such as ethyl acetate, chloroform or n-butanol is added for extraction, and the extract is concentrated. The resulting residue is re-dissolved in an organic solvent such as benzene, ethyl acetate, acetone, methanol or chloroform and then applied to silica gel column chromatography, gel filtration column chromatography, column chromatography packed with ODS for reversed phase partition and high performance liquid chromatography to thereby purify and isolate the compound according to the present invention.

The strain producing the active ingredient according to the present invention is a novel strain that has been newly isolated from soil by the present inventors. The microorganism was named *"Pochonia chlamydosporia* var. *chlamydosporia* TF-0480" and has been deposited to International Patent Organism Depository, National Institute of Advanced Industrial Science and Technology as "FERM BP-8332".

The microbiological properties of this strain are as follows.

### 1) Growth on medium

The microorganism grows well in various agar plate media and well or moderately forms spores in tested media. Table 1 summarizes characteristics of colonies formed after culturing at 26°C for 2 weeks on various media. Colors are indicated in accordance with the color codes proposed by A. Kornerup *et al., Dizionario dei colori,* Musterschmidt (1978).

**Table 1**

| Medium | Results of observation on colonies with naked eye |
|---|---|
| PDA^{*-1} | Showing relatively well growth and sporulation. Colony diameter:23-28 mm. Wooly and somewhat domed colonies with pannose center and slight radial wrinkles. Somewhat irregular periphery (colony tip). Secreting a pale light yellow [3A6] pigment into the medium. Colony surface color: white [1A1] at the periphery and pale yellow [4A3] at the center. Back face color: reddish yellow [4A6] at the periphery and yellowish brown [5D8] at the center. Agar medium being irregularly or radially fractured. |
| OMA^{*-2} | Showing well growth and sporulation. Colony diameter: 48-50 mm. Somewhat thin and wooly colonies with domed center and intermediate part. Relatively regular-shaped periphery. No pigment secretion. Colony surface color: white [1A1] at the periphery and light yellow [3A4] at the center. Back face color: light yellow [3A4] similar to the surface. |
| CMA^{*-3} | Showing well growth and moderate sporulation. Colony diameter: 47-51 mm. Thin and pannose colonies. Relatively regular-shaped periphery. No pigment secretion into the medium. Colony surface/back face color: both white [1A1] to yellowish white [1A2]. |
| MA^{*-4} | Showing relatively well growth and sporulation. Colony diameter: 26-27 mm. Thick and wooly colonies with domed center. Relatively regular-shaped periphery. Secreting a slightly reddish yellow [4A7] pigment into the medium. Colony surface color: white [1A1] at the periphery and pale yellow [4A3] at the center. Back face color: light orange [5A4] at the periphery, brown [7E8] at the center and orange [6B7] at the intermediate part. |
| SA^{*-5} | Showing relatively well growth and sporulation. Colony diameter: 23-28 mm. Thick and wooly colonies with domed center. Somewhat irregular periphery with slight radial wrinkles. Secreting a reddish yellow [4A7] pigment into the medium. Colony surface color: white [1A1] at the periphery and orange white [5A2] in other parts. Back face color: light orange [5A4] at the periphery and center and brown [7E8] at the intermediate part. Agar medium being irregularly or radially fractured. |
| LCA^{*-6} | Showing well growth and moderate sporulation. Colony diameter: 42-44 mm. Thin pannose to slightly wooly colonies. Relatively regular-shaped periphery. No secretion of pigment into the medium. Colony surface/back face color: both white [1A1]. |

| | |
|---|---|
| *-1: Potato dextrose agar medium (E-MF21, manufactured by Eiken Chemical Co., Ltd.). | |
| *-2: 1/2-Diluted oatmeal agar medium (manufactured by Difco, prepared by 1/2 diluting Bacto oatmeal agar and adding agar to give a final agar concentration of 2%). | |
| *-3: Corn meal agar medium (manufactured by Difco, Corn meal agar). | |
| *-4: Malt extract agar medium (Nakase, T., 6th ed., pp.617, Japan Collection of Microorganisms, the Institute of Physical and Chemical Research, Saitama, 1995) | |
| *-5: Sabouraud agar medium (manufactured by Eiken Chemical Co., Ltd., E-MF03) | |
| *-6: Miura agar medium (Miura, K. and M. Kudo, *Trans. Mycol. Soc. Japan,* 11, 116-118 (1970)) | |

### 2) Morphology

Colonies formed after culturing the strain on a corn meal agar plate at 26°C for 14 days are observed under an optical microscope. As a result, the formation of phialo-conidia was observed. A single conidium-forming cell (phialide) or 2 to 4 verticillate phialides are formed directly on a hypha. Conidiophorer cannot be clearly distinguished from vegetative hyphae. A plural number of conidia are formed at the front edge of a phialide to form a viscous mass. A phialide has a tapered cylindrical shape and smooth and colorless surface. It is from 13 to 15 µm in length and 0.8 to 1.7 µm in thickness at the base and 0.5 to 0.8 µm in thickness at the end. Conidia are in an elliptical shape or a semi-spherical shape (rare) and most of them have slightly projecting base. The conidia have smooth and colorless surface and are 2.2 to 5.0 × 1.8 to 2.8 µm in size. A single pale yellow and multicellular chlamydospore having netted wall (dictyochlamydospore) is formed on the medium surface or at the end of a short hypha sprouting on the medium. Dictyochlamydospores are also formed in the medium in a small amount. These dictyochlamydospores are 14 to 20 × 12 to 22 µm in size. After prolonging the culture over one month or longer, no teleomorph is observed.

### 3) Physiological properties

### (1) Growth temperature range and optimum temperature

In Sabouraud liquid medium at pH 6.0, this strain grows within a temperature range of 15 to 31°C and the optimum temperature is from 25 to 27°C.

### (2) Growth pH range and optimum pH value

In YpSs liquid medium at 26°C, this strain grows within a pH of 3 to 10 and the optimum pH is from 5 to 7.

### 4) Aerobic/anaerobic: aerobic.

Based on the above-described morphological characteristics and culture properties, the characteristics of this strain are compared with various known species reported in (1) K.H. Domsch *et al., Compendium of soil fungi,* Vol. 1, IHW- Verlag (1980) and (2) G.L. Barron, *The Genera of Hyphoomycetes from soil,* Williams & Wilkins (1968) and the like. As a result, this strain is closely similar to an incomplete species known under a classification name *Verticillium chlamydosporia* or *Diheterospora chlamydosporia* and, therefore, seemingly belongs to the same species. In (3) R. Zare, W. Gams and H.C. Evans, *A revision of Verticillium section Prostrata. V. The genus Pochonia, with notes on Rotiferophthora,* 73, 1-2, p51-86 (2001), this species is molecular systematically re-examined and, in its turn, referred to as belonging to the genus *Pochonia* as the proper genus. Moreover, it is suggested to refer such a species forming non-linked Verticillium-type conidia as the strain of the present invention as to a variant *Pochonia chlamydosporia* var. *chlamydosporia.*

Based on these facts, this strain is named *"Pochonia chlamydosporia* var. *chlamydosporia* TF-0480".

### <Dermal papilla cell growth promoter>

The term "dermal papilla cell growth promoter" as used in the present invention means a medicament or a reagent having an effect of increasing the number of dermal papilla cells.

The dermal papilla cell growth promoter according to the present invention is characterized by being based on the effect of inhibiting the function of WNT-5A. Since WNT-5A inhibits the functions of WNT-1 class molecules (WNT-1, WNT-8, *etc.*) which are activators of WNT/β-catenin pathway, the growth of dermal papilla cells is controlled by regulating the function of WNT-5A or regulating the expression of WNT-5A. Therefore, even compounds having completely different structures (e.g., Compound 24, Compound 79, Compound 7, *etc.)* have an excellent effect of promoting the growth of dermal papilla cells, so long as these compounds have an excellent effect of inhibiting the function of WNT-5A.

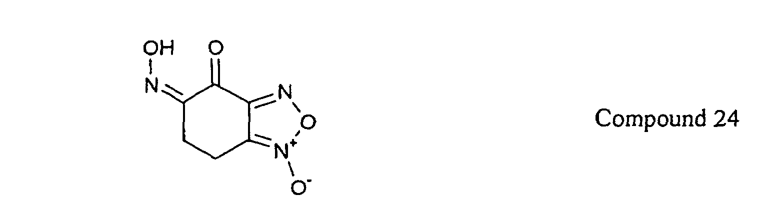

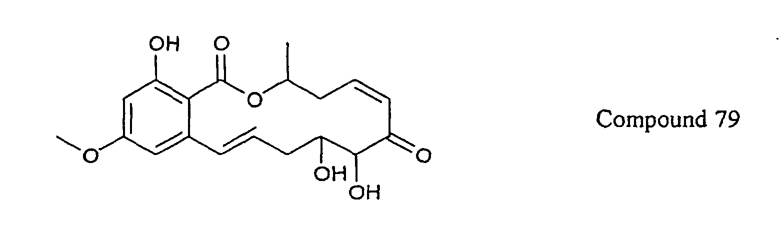

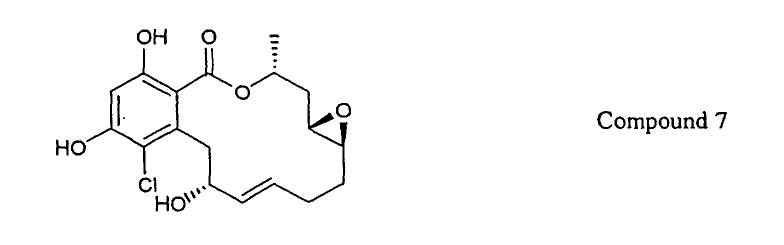

The growth of cells can be measured by a method publicly known by a person skilled in the art. Examples of the measuring method include counting of viable cells using an appropriate color-developing substrate and the [³H]-thymidine uptake method. As the color-developing substrate, tetrazolium salts such as MTT, MTS and XTT and Alamar Blue are preferably used.

### <Hair growth stimulant/hair growth tonic>

The term "hair growth stimulant or hair growth tonic" as used in the present invention means products to be used for inducing hair growth, promoting hair growth, preventing alopecia and the like. In the case of using the hair growth stimulant and hair growth tonic according to the present invention as medicaments, the subject to be applied includes improvement or prevention of alopecia areata and male pattern alopecia.

The effects of the hair growth stimulant and hair growth tonic according to the present invention are achieved by the effect of promoting the growth of dermal papilla cells by inhibiting the function of WNT-5A. Based on this function mechanism, the cell growing ability, which has been depressed in dermal papillae in an alopecia site, is enhanced and dermal well-developed papilla tissue is formed. It is therefore expected that the hair growth stimulant/hair growth tonic according to the present invention are efficacious against symptoms on which little effects can be established by the existing hair growth stimulants and hair growth tonics.

It is furthermore expected that synergistic effects could be obtained by combining the hair growth stimulant and hair growth tonic according to the present invention with other hair growth stimulants and hair growth tonics having different function points.

The hair growth stimulant and hair growth tonic according to the present invention can be administered in various doses and various dosage forms depending on individual compounds.

In the case of administering the compound represented by formula (IX) by application (lotion, ointment, a gel, *etc.),* for example, it can be administered at a dose of 0.0001 to 10% by weight, preferably 0.001 to 5% by weight and more preferably 0.001 to 1% by weight, although the dose varies depending on the type and dosage form of the hair growth tonic. In the case of orally administering (dust, tablet or capsule) the compound represented by formula (IX) to a male adult, the daily dose preferably ranges from 1 to 100 mg/kg.

Although the dosage forms of the hair growth stimulant and hair growth tonic according to the present invention are not particularly restricted, it is preferable to provide the hair growth stimulant/hair growth tonic containing a WNT-5A production inhibitor (e.g., the compound represented by formula (IX)) as an active ingredient in the form of a water-soluble composition in the case of external application. Such a water-soluble composition can be produced by using various additives (humectant, thickener, preservative, antioxidant, aromatic, coloring agent, etc.) commonly employed in producing drugs, quasi drugs or cosmetics, so long as the effects of the present invention are not damaged thereby. The hair growth stimulant and hair growth tonic according to the present invention can be provided as hair-care compositions such as hair tonic, hair oil, hair moose or gel, or ointments.

In the case of using hair growth stimulant and hair growth tonic according to the present invention as liquid preparations, such a preparation is produced as an aqueous solution, a non-aqueous solution, a suspension, liposomes or an emulsion prepared by dissolving a WNT-5A production inhibitor (e.g., the compound represented by formula (IX)) in purified water, an appropriate buffer such as a phosphate buffer, a physiological salt solution such as physiological saline, Ringer solution or Locke solution, ethanol or glycerol appropriately combined with a surfactant, etc. commonly employed, followed by sterilization. It is topically administered as a liquid preparation for scalp. The liquid preparation may be applied directly to the scalp. Alternatively, it may be applied by using a jetting nozzle such as a spray.

In the case of using hair growth stimulant and hair growth tonic according to the present invention as semi-solid preparations, a WNT-5A production inhibitor (for example, the compound represented by formula (IX)) is mixed with a fat, an oil, lanolin, vaseline, paraffin, wax, a plaster, a resin, plastic, glycerine, higher alcohol, glycerol, water, an emulsifier, a suspending agent or the like to give an external preparation (ointment, cream, etc.) which can be topically administered.

In the case of using hair growth stimulant and hair growth tonic according to the present invention as solid preparations, a WNT-5A production inhibitor (e.g., the compound represented by formula (IX)) is mixed with appropriate additive(s) to give an external preparation such as a dust or a powder. Alternatively, it is possible to produce a solid preparation which is dissolved or suspended in a solvent before using and applied to the scalp.

In the case of orally using, it is preferable that a WNT-5A production inhibitor (e.g., the compound represented by formula (IX)) is blended with a pharmaceutically acceptable carrier (filler, binder, disintegrating agent, flavor, corrigent, emulsifier, etc.), a diluent, a dissolution aid or the like and the obtained medicinal composition is processed in a conventional manner to give a tablet, a capsule, granules, a dust, a syrup, a suspension, a solution and the like.

These preparations can be produced by using formulation techniques commonly employed.

### <Screening method>

The present invention further relates to a method of screening a dermal papilla cell growth promoter, which comprises selecting a compound inhibiting the function of WNT-5A.

Selecting a compound inhibiting the function of WNT-5A (hereinafter sometimes referred to as "WNT-5A function inhibitor") means, for example, selecting (i.e., screening) a WNT-5A production inhibitor, or selecting a WNT-5A receptor antagonist.

As test materials to be subjected to the screening method according to the present invention, any materials can be used. That is, the test materials are not restricted in kind, and they may be low-molecular weight compounds, compounds contained in a natural extract or synthetic peptides. They may also be a compound library or a combinatorial library. The compound library can be constructed by a method publicly known by one of the skilled in the art. Alternatively, a commercially available compound library can be used. From the viewpoint of using as a medicament, the compound which is subjected to screening preferably has a molecular weight of 3000 or less. From the viewpoint of enabling application/oral administration, a low-molecular weight compound having a molecular weight of 600 or less is preferred.

### (1) Method of screening WNT-5A receptor antagonist

In a method of screening a WNT-5A receptor antagonist, whether or not a bond between the WNT-5A protein and the WNT-5A receptor is formed can be examined by using a labeled WNT-5A protein and a WNT-5A receptor and detecting or measuring the label. Examples of the label include a radioisotope (³²P, ³³P, ¹³¹I, ¹²⁵I, ³H, ¹⁴C, ³⁵S, *etc.),* an enzyme (alkali phosphatase, hoarse radish peroxidase, etc.), a fluorescent substance (fluorescein isothiocyanate, *etc.*) and the like. They can be commercially available and the labeling can be made by a known method.

A specific example of an *in vitro* assay system is performed in a cell-free system. More specifically, one of the WNT-5A protein and the WNT-5A receptor is bound to a support and the other and a test material are added thereto. After incubating, the reaction mixture is washed and the binding of the other protein to the protein having been bound to the support is detected or measured.

Examples of the support to which the protein is bound include insoluble polysaccharides such as agarose, dextran and cellulose; synthetic resins such as polystyrene, polyacrylamide and silicone; and the like. More specifically, commercially available beads and plates obtained by these materials can be employed.

### (2) Method of screening compound inhibiting WNT-5A production

A compound inhibiting WNT-5A production can be screened by using the amount of WNT-5A mRNA or the amount of WNT-5A protein as an index. It is also possible to bind a reporter gene to the promoter region of a WNT-5A gene and detect the expression amount. As the promoter of the WNT-5A gene, one represented by SEQ ID NO:3 is preferably used.

Examples of the reporter gene include a GFP (green fluorescent protein) gene, a GUS (β-glucuronidase) gene, an LUC (luciferase) gene, and a CAT (chloramphenicol acetyltransferase) gene.

When the expression amount of WNT-5A mRNA is used as an index, for example, human WNT-5A-expressing cells are used and a test material is added thereto. After culturing in an incubator for several hours at 37°C in a 5% CO₂-95% air atmosphere, the cells are lysed and RNAs are extracted. Then, the amount of the WNT-5A mRNA is measured by, for example, RT-PCR. Thus, a material having an activity of decreasing the WNT-5A mRNA amount can be searched. The primers used in PCR are not particularly limited, so long as they are specific to WNT-5A mRNA, and they can be designed from the WNT-5A mRNA sequence. Preferred examples include a forward primer AATGTCTTCC AAGTTCTTCC TAGTGGC (SEQ ID NO:8) and a reverse primer GATGTCGGAA TTGATACTGG CA (SEQ ID NO:9).

When the WNT-5A protein amount is used as an index, for example, human WNT-5A-expressing cells are used and a test material is added thereto. After culturing in an incubator for several hours at 37°C in a 5% CO₂-95% air atmosphere, the cells are lysed by using the culture medium and proteins are extracted. Then, the amount of the WNT-5A protein is measured by ELISA (enzyme-linked immunosorbent assay) or the like. Thus, a substance having an activity of decreasing the expression amount of the WNT-5A mRNA can be searched.

Next, the present invention is described in detail by reference to Test Examples below; however, the present invention is not limited thereto.

### Example 1 (Compounds 3 and 4)

Radicicol (Compound 1: 10.8 g) was dissolved in ethyl acetate (140 ml) and 5% palladium carbon (wet type) (255 mg) was added thereto, followed by hydrogen replacement (1 atm) and stirring at room temperature for 3 hours. After filtering the palladium carbon, the filtrate was distilled off under reduced pressure. The resulting residue was purified by silica gel chromatography and eluted with n-hexane:ethyl acetate (2: 1) to give target Compound 4 (3.43 g) was obtained, or eluted with n-hexane:ethyl acetate (3:2) to give target Compound 3 (4.14 g).

### Example 2 (Compounds 7 and 8)

Compound 3 (602.5 mg) was dissolved in methanol (13 ml) and cerium (III) chloride heptahydride (2.14 g) was added thereto, followed by stirring at room temperature for 30 minutes. Next, sodium boron hydride (180 mg) was slowly added to the solution under ice cooling, followed by stirring at room temperature for 5 minutes. After adding saturated disodium hydrogen phosphate (40 ml) to the reaction solution, the mixture was diluted with water (40 ml) and the organic solvent was distilled off under reduced pressure. The residual aqueous layer was extracted with ethyl acetate (300 ml × 2). The resulting ethyl acetate layer was dried over anhydrous sodium sulfate and then the ethyl acetate was distilled off under reduced pressure. Then, the residue was crudely purified by silica gel preparative thin layer chromatography (20 cm × 20 cm, 1.0 mm in thickness, developed with chloroform:methanol = 93:7, eluted with ethyl acetate). The resulting crude purified product was then purified by high performance liquid chromatography (20 φ × 250 mm, YMC-Pack Pro C18, eluted with water (to which acetic acid had been added, pH 3.5):acetonitrile = 65:35) to give the target compounds (Compound 7: 144.9 mg, Compound 8: 13.8 mg).

### Example 3 (Compounds 9 and 10)

Compound 4 (26.5 mg) was dissolved in methanol (5 ml) and cerium (III) chloride heptahydride (100 mg) was added thereto, followed by stirring at room temperature for 30 minutes. Next, sodium boron hydride (60 mg) was slowly added to the solution under ice cooling, followed by stirring at room temperature for 30 minutes. After adding saturated disodium hydrogen phosphate (12 ml) to the reaction solution, the mixture was diluted with water (20 ml) and the organic solvent was distilled off under reduced pressure. The residual aqueous layer was extracted with ethyl acetate (30 ml x 2). The resulting ethyl acetate layer was dried over anhydrous sodium sulfate and then the ethyl acetate was distilled off under reduced pressure. Then, the residue was purified by silica gel preparative thin layer chromatography (20 cm x 20 cm, 0.25 mm in thickness, developed with chloroform:methanol = 94:6, eluted with ethyl acetate) to give the target compounds (Compound 9: 5.7 mg, Compound 10: 8.8 mg).

### Example 4 (Compound 5)

Radicicol (91.5 mg) was dissolved in methanol (5 ml) and cerium (III) chloride heptahydride (88 mg) was added thereto, followed by stirring at room temperature for 10 minutes. Next, sodium boron hydride (60 mg) was slowly added to the solution under ice cooling, followed by stirring at room temperature for 30 minutes. After adding saturated disodium hydrogen phosphate (20 ml) to the reaction solution, the mixture was diluted with water (20 ml) and the organic solvent was distilled off under reduced pressure. The residual aqueous layer was extracted with ethyl acetate (50 ml x 3). The resulting ethyl acetate layer was dried over anhydrous sodium sulfate and then the ethyl acetate was distilled off under reduced pressure. Then, the residue was purified by silica gel preparative thin layer chromatography (20 cm x 20 cm, 0.5 mm in thickness, developed with chloroform:methanol = 9:1, eluted with ethyl acetate) to give target Compound 5 (27.2 mg).

### Example 5 (Compound 2)

Radicicol (15.3 mg) was dissolved in pyridine (1.5 ml) and acetic anhydride (4 ml) was added thereto, followed by stirring at room temperature for 6.5 hours. After adding ice water (20 ml) to the reaction solution, the mixture was extracted with ethyl acetate (20 ml). The ethyl acetate layer was washed with water (20 ml x 2) and dried over anhydrous sodium sulfate and then the ethyl acetate was distilled off under reduced pressure. Then, the residue was purified by silica gel preparative thin layer chromatography (20 cm × 20 cm, 0.5 mm in thickness, developed with chloroform:methanol = 95:5, eluted with ethyl acetate) to give target Compound 2 (18.5 mg).

### Example 6 (Compound 11)

Radicicol (19.0 mg) was dissolved in dimethyl sulfoxide (1 ml), and potassium carbonate (3 mg) and methyl iodide (4 ml) were added thereto, followed by stirring at room temperature for 6.5 hours. After adding water (20 ml) to the reaction solution, the mixture was extracted with ethyl acetate (20 ml). The ethyl acetate layer was dried over anhydrous sodium sulfate and then the ethyl acetate was distilled off under reduced pressure. Then, the residue was purified by silica gel preparative thin layer chromatography (20 cm × 20 cm, 0.5 mm in thickness, developed with chloroform:methanol = 95:5, eluted with ethyl acetate) to give target Compound 11 (15.3 mg).

### Example 7 (Compounds 16, 17, 19 and 23)

Radicicol (930 mg) was dissolved in 1,4-dioxane (14 ml) and 1N hydrochloric acid (12 ml) was added thereto, followed by stirring at room temperature for 2 hours. After diluting with water (40 ml), the mixture was extracted with ethyl acetate (100 ml). The ethyl acetate layer was dried over anhydrous sodium sulfate and then the ethyl acetate was distilled off under reduced pressure. Then, the residue was crudely purified by silica gel preparative thin layer chromatography (20 cm x 20 cm, 1.0 mm in thickness, developed with chloroform:methanol:n-hexane = 5:1:5, eluted with ethyl acetate). The resulting crude purified product was then purified by high performance liquid chromatography (20 φ × 250 mm, YMC-Pack Pro C18, eluted under gradient with water (to which acetic acid had been added, pH 3.5):acetonitrile = 70:30 to 40:60) to give the target compounds (Compound 16: 11.4 mg, Compound 17: 19.4 mg, Compound 19: 32.6 mg, Compound 23: 103.7 mg).

### Example 8 (Compounds 12 and 13)

Radicicol (232.6 mg) was dissolved in 1,4-dioxane (4 ml) and 1N hydrochloric acid (1 ml) was added thereto, followed by stirring at room temperature for 30 minutes. After neutralizing with 1N sodium hydroxide, the solvent was distilled off under reduced pressure. Then, the concentrate was dissolved with 20 ml of methanol. The resultant solution was filtered through a cotton plug and the methanol was distilled off under reduced pressure. The resulting residue was then purified by high performance liquid chromatography (20 φ × 250 mm, YMC-Pack Pro C18, eluted with water (to which acetic acid had been added, pH 3.5):acetonitrile = 65:35) to give the target compounds (Compound 12: 42.6 mg, Compound 13: 10.4 mg).

### Example 9 (Compound 18)

Into 5 ml of dimethylformamide, 1 ml of phosphorus oxychloride was dropped under ice cooling and then the mixture was stirred at room temperature for 30 minutes. The resultant solution was slowly added to a dimethylformamide solution (4 ml) of Radicicol (98.5 mg) under ice cooling followed by stirring at room temperature for 24 hours. Then, the reaction solution was diluted with ethyl acetate (100 ml) and washed with water (100 ml × 3). The ethyl acetate layer was dried over anhydrous sodium sulfate and then the ethyl acetate was distilled off under reduced pressure. Then, the resulting residue was purified by silica gel preparative thin layer chromatography (20 cm × 20 cm, 0.5 mm in thickness, developed with chloroform:methanol = 94:6, eluted with ethyl acetate) to give the target compound (Compound 18: 61.8 mg).

### Example 10 (Compounds 14 and 15)

Radicicol (378 mg) was dissolved in 1,4-dioxane (4 ml) and 1N hydrochloric acid (1 ml) was added thereto, followed by stirring at room temperature for 20 minutes. After neutralizing with 1N sodium hydroxide, the solvent was distilled off under reduced pressure. Then, the concentrate was dissolved with 20 ml of methanol. The resultant solution was filtered through a cotton plug and the methanol was distilled off under reduced pressure. The resulting residue was then purified by high performance liquid chromatography (20 φ × 250 mm, YMC-Pack Pro C18, eluted with water (to which acetic acid had been added, pH 3.5):acetonitrile = 70:30) to give the target compounds (Compound 14: 10.7 mg, Compound 15: 9.9 mg) simultaneously with Compound 12 (40.6 mg).

### Example 11 (Compounds 20, 21 and 22)

Compound 3 (96.3 mg) was dissolved in 1,4-dioxane (2 ml) and 1N hydrochloric acid (2.5 ml) was added thereto, followed by stirring at room temperature for 16 hours. After neutralizing with 1N sodium hydroxide, the solvent was distilled off under reduced pressure. Then, the concentrate was dissolved with 20 ml of methanol. The resultant solution was filtered through a cotton plug and the methanol was distilled off under reduced pressure. The resulting residue was then purified by high performance liquid chromatography (20 φ × 250 mm, YMC-Pack Pro C18, eluted with water (to which acetic acid had been added, pH 3.5):acetonitrile = 45:55) to give the target compounds (Compound 20: 9.3 mg, Compound 21: 22.0 mg, Compound 22: 26.2 mg).

### Example 12 (Compounds 25 and 26)

Compound 7 (34 mg) was dissolved in *N,N* dimethylformamide (2 ml), and potassium hydroxide (3 mg) and methyl iodide (500 µl) were added thereto, followed by stirring at room temperature for 4.5 hours. After adding water (20 ml) to the reaction solution, the mixture was extracted with ethyl acetate (20 ml). The ethyl acetate layer was dried over anhydrous sodium sulfate and the ethyl acetate was distilled off under reduced pressure. The resulting residue was dissolved in a small amount of acetone and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (38:62) as the mobile phase to obtain two fractions. Each fraction was concentrated under reduced pressure to give Compound 25 (6.4 mg, 39 min) and Compound 26 (14.5 mg, 29 min).

### Example 13 (Compound 27)

Compound 7 (55 mg) was dissolved in pyridine (1 ml), and acetic anhydride (3 ml) was added thereto, followed by stirring at room temperature for 17 hours. After adding water (50 ml) to the reaction solution, the mixture was extracted with ethyl acetate (50 ml). The ethyl acetate layer was dried over anhydrous sodium sulfate and the ethyl acetate was distilled off under reduced pressure. The resulting residue was dissolved in a small amount of acetone and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (60:40) as the mobile phase. The resulting fraction was concentrated under reduced pressure to give Compound 27 (64.7 mg, 18 min).

### Example 14 (Compounds 28 and 29)

Compound 8 (131 mg) was dissolved in N,N-dimethylformamide (2 ml), and potassium hydroxide (3 mg) and methyl iodide (2 ml) were added thereto, followed by stirring at room temperature for 7 hours. After adding water (40 ml) to the reaction solution, the mixture was extracted with ethyl acetate (40 ml). The ethyl acetate layer was dried over anhydrous sodium sulfate and the ethyl acetate was distilled off under reduced pressure. The resulting residue was dissolved in a small amount of acetone and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (38:62) as the mobile phase. Thus 2 fractions were obtained and respectively concentrated under reduced pressure to give Compound 28 (14.2 mg, 52 min) and Compound 29 (31.7 mg, 37 min).

### Example 15 (Compound 30)

Compound 8 (72 mg) was dissolved in pyridine (2 ml), and acetic anhydride (2 ml) was added thereto, followed by stirring at room temperature for 17 hours. After adding water (50 ml) to the reaction solution, the mixture was extracted with ethyl acetate (50 ml). The ethyl acetate layer was dried over anhydrous sodium sulfate and the ethyl acetate was distilled off under reduced pressure. ' The resulting residue was dissolved in a small amount of acetone and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (53:47) as the mobile phase. The obtained fraction was concentrated under reduced pressure to give Compound 30 (45.5 mg, 30 min).

### Example 16 (Compound 31)

Radicicol (10.8 g) was dissolved in ethyl acetate (140 ml) and 5% palladium carbon (wet type) (255 mg) was added thereto, followed by hydrogen replacement (1 atm) and stirring at room temperature for 3 hours. After filtering the palladium carbon, the filtrate was concentrated under reduced pressure. The resulting residue was purified by silica gel chromatography [Silica Gel 60 (trade name, manufactured by Merck)] and eluted with n-hexane:ethyl acetate (2:1) to give Compound 4 (3.43 g), or eluted with n-hexane:ethyl acetate (3:2) to give Compound 3 (4.14 g). A portion (500 mg) of a fraction (3.08 g) between Compound 3 and Compound 4 was dissolved in a small amount of N,N-dimethylformamide and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (38:62) as the mobile phase. Thus 2 fractions were obtained and respectively concentrated under reduced pressure to give Compound 3 (177.8 mg) together with Compound 31 (20.7 mg, 23 min).

### Example 17 (Compounds 32, 33 and 34)

Radicicol (11.9 g) was dissolved in ethyl acetate (160 ml) and 5% palladium carbon (wet type) (300 mg) was added thereto. After purging with hydrogen (1 atm), the mixture was stirred at room temperature for 3 hours. After filtering the palladium carbon, the filtrate was concentrated under reduced pressure. The resulting residue was purified by silica gel chromatography [Silica Gel 60 (trade name, manufactured by Merck)] and eluted with n-hexane:ethyl acetate (2:1) to give Compound 4, or eluted with n-hexane:ethyl acetate (3:2) to give Compound 3. Furthermore, a fraction eluted with n-hexane:ethyl acetate (1:1) was concentrated under reduced pressure to give a crude purified product (936 mg). The resulting crude purified product was dissolved in a small amount of acetone and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (34:66) as the mobile phase to obtain three fractions. Each fraction was concentrated under reduced pressure to give Compound 32 (17.4 mg, 17 min), Compound 33 (49.4 mg, 18 min) and Compound 34 (347.4 mg, 21 min).

### Example 18 (Compound 35)

Radicicol (210 mg) was dissolved in pyridine (4 ml), and hydroxylamine hydrochloride (180 mg) was added thereto at room temperature, followed by heating 40°C and stirring for 2.5 hours. After cooling to room temperature, water (100 ml) was added thereto and the mixture was extracted with ethyl acetate (80 ml). The ethyl acetate layer was dried over anhydrous sodium sulfate and then the ethyl acetate was distilled off under reduced pressure. The resulting residue was purified by preparative silica gel chromatography [Silica Gel 60 F254 (trade name, manufactured by Merck), 0.5 mm in thickness] using chloroform-methanol (90:10) as the developing solvent. A fraction showing an Rf value of 0.65 was collected, extracted with ethyl acetate and then concentrated under reduced pressure to give Compound 35 (40.0 mg).

### Example 19 (Compounds 36, 37, 38 and 39)

Radicicol (11.9 g) was dissolved in ethyl acetate (160 ml), and 5% palladium carbon (wet type) (300 mg) was added thereto, followed by hydrogen replacement (1 atm) and stirring at room temperature for 3 hours. After filtering the palladium carbon, the filtrate was concentrated under reduced pressure. The resulting residue was purified by silica gel chromatography [Silica Gel 60 (trade name, manufactured by Merck)] and eluted with n-hexane:ethyl acetate (2:1). The resulting fraction was concentrated under reduced pressure to give a fraction (1537 mg) comprising Compound 4 as the main component. This fraction was dissolved in methanol (21 ml)/tetrahydrofuran (7 ml), and cerium (III) chloride heptahydride (2130 mg) was added thereto, followed by stirring at room temperature for 30 minutes. Next, sodium boron hydride (790 mg) was slowly added to the solution under ice cooling, followed by stirring at room temperature for 10 minutes. The reaction solution was diluted with water (30 ml) and the pH value was adjusted to be neutral by adding a 1N aqueous hydrochloric acid solution, followed by further dilution with water (30 ml). The aqueous layer was extracted with ethyl acetate (100 ml × 2). The resulting ethyl acetate layer was dried over anhydrous sodium sulfate and then the ethyl acetate was distilled off under reduced pressure to give a gummy substance (1.46 g). The obtained substance was dissolved in a solvent mixture of chloroform-methanol (4:1) and adsorbed by a silica gel [Silica gel 60 (trade name, manufactured by Merck)] column (400 ml) prepared by moistening with n-hexane. Then, elution was successively carried out with a solvent mixture of n-hexane-ethyl acetate. After eluting Compound 4 with n-hexane-ethyl acetate (2: 1), the residue was eluted with n-hexane-ethyl acetate (3:2) to give each of fraction (1) (190 mg) and fraction (2) (398 mg) by concentration under reduced pressure. Fraction (1) was dissolved in a small amount of tetrahydrofuran and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (35:65) as the mobile phase to obtain three fractions. Each of the fraction was concentrated under reduced pressure to give Compound 9 (87.2 mg) together with Compound 36 (11.3 mg, 20 min) and Compound 38 (10.1 mg, 25 min). Fraction (2) was dissolved in a small amount of tetrahydrofuran and purified by preparative high performance liquid chromatography under the same conditions as in fraction (1) to obtain two fractions. Each of the fractions was concentrated under reduced pressure to give Compound 37 (19.8 mg, 25 min) and Compound 39 (23.2 mg, 27 min).

### Example 20 (Compounds 40 and 41)

Compound 7 (61 mg) was dissolved in N,N-dimethylformamide (3 ml), and potassium hydroxide (5 mg) and 1-bromobutane (1 ml) were added thereto, followed by stirring at room temperature for 3.5 hours. After adding water (20 ml) to the reaction solution, the mixture was extracted with ethyl acetate (30 ml). The ethyl acetate layer was dried over anhydrous sodium sulfate and then the ethyl acetate was distilled off under reduced pressure. The resulting residue was dissolved in a small amount of acetone and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) under gradient of (60:40) to (90: 10) as the mobile phase to obtain two fractions. Each of the fractions was concentrated under reduced pressure to give Compound 40 (25.6 mg, 33 min) and Compound 41 (21.4 mg, 40 min).

### Example 21 (Compounds 42 and 43)

Compound 7 (67 mg) was dissolved in N,N-dimethylformamide (3 ml), and potassium hydroxide (5 mg) and 1-bromohexane (1 ml) were added thereto, followed by stirring at room temperature for 3.5 hours. After adding water (20 ml) to the reaction solution, the mixture was extracted with ethyl acetate (30 ml). The ethyl acetate layer was dried over anhydrous sodium sulfate and then the ethyl acetate was distilled off under reduced pressure. The resulting residue was dissolved in a small amount of acetone and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) under gradient of (60:40) to (90:10) as the mobile phase to obtain two fractions. Each of the fractions was concentrated under reduced pressure to give Compound 42 (24.9 mg, 38 min) and Compound 43 (32.9 mg, 53 min).

### Example 22 (Compound 44)

Compound 7 (62 mg) was dissolved in N,N-dimethylformamide (3 ml), and potassium hydroxide (5 mg) and 1-bromopropane (1 ml) were added thereto, followed by stirring at room temperature for 1.5 hours. After adding water (20 ml) to the reaction solution, the mixture was extracted with ethyl acetate (30 ml). The ethyl acetate layer was dried over anhydrous sodium sulfate and then the ethyl acetate was distilled off under reduced pressure. The resulting residue was dissolved in a small amount of acetone and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) under gradient of (45:55) to (85:15) as the mobile phase. The obtained fraction was concentrated under reduced pressure to give Compound 44 (45.3 mg, 38 min).

### Example 23 (Compound 45)

Compound 7 (64 mg) was dissolved in N,N-dimethylformamide (3 ml), and potassium hydroxide (5 mg) and 2-(bromomethyl)cyclohexane (2 ml) were added thereto, followed by stirring at room temperature for 7 hours. After adding water (20 ml) to the reaction solution, the mixture was extracted with ethyl acetate (30 ml). The ethyl acetate layer was dried over anhydrous sodium sulfate and then the ethyl acetate was distilled off under reduced pressure. The resulting residue was dissolved in a small amount of acetone and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) under gradient of (65:35) to (85:15) as the mobile phase. The obtained fraction was concentrated under reduced pressure to give Compound 45 (24.7 mg, 70 min).

### Example 24 (Compound 46)

Compound 7 (200 mg) was dissolved in N,N-dimethylformamide (10 ml), and hydrobromic acid (5 ml) was added thereto, followed by stirring at 110°C for 6 hours. After adding ethyl acetate (150 ml) to the reaction solution, the mixture was washed with sodium hydrogen carbonate (150 ml x 2). The ethyl acetate layer was dried over anhydrous sodium sulfate and then the ethyl acetate was distilled off under reduced pressure. The resulting residue was dissolved in a small amount of methanol and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) under gradient of (38:62) to (50:50) as the mobile phase. The obtained fraction was concentrated under reduced pressure to give Compound 46 (17.0 mg, 18 min).

### Example 25 (Compound 47)

Compound 62 (154 mg) was dissolved in ethyl acetate (5 ml), and 5% palladium carbon (wet type) (30 mg) was added thereto, followed hydrogen replacement (1 atm) and stirring at room temperature for 90 minutes. After filtering the palladium carbon, the filtrate was distilled off under reduced pressure to give Compound 47 (134.8 mg).

### Example 26 (Compound 48)

Compound 61 (211 mg) was dissolved in ethyl acetate (11 ml), and 5% palladium carbon (wet type) (42 mg) was added thereto, followed by hydrogen replacement (1 atm) and stirring at room temperature for 90 minutes. After filtering the palladium carbon, the filtrate was distilled off under reduced pressure. The resulting residue (184 mg) was purified by preparative thin layer chromatography [Silica Gel 60 F254 (trade name, manufactured by Merck), 20 cm × 20 cm, 0.5 mm in thickness] using chloroform-methanol (95:5) as the developing solvent. A part with an Rf value of 0.5 was collected, extracted with ethyl acetate and then concentrated under reduced pressure to give Compound 48 (159.7 mg).

### Example 27 (Compounds 49 and 50)

Compound 61 (233 mg) was dissolved in methanol (10 ml)/tetrahydrofuran (2 ml), and cerium (III) chloride heptahydride (754 mg) was added thereto, followed by stirring at room temperature for 30 minutes. Next, sodium boron hydride (236 mg) was slowly added to the solution under ice cooling, followed by stirring at room temperature for 5 minutes. After diluting the reaction solution with water (30 ml), a 1N aqueous hydrochloric acid solution (3 ml) was added to adjust the pH value to be neutral, and the mixture was further diluted with water (30 ml). The aqueous layer was extracted with ethyl acetate (100 ml × 2). The resulting ethyl acetate layer was dried over anhydrous sodium sulfate and then the ethyl acetate was distilled off under reduced pressure to give a white powder (267 mg). Then, the obtained powder was dissolved in a small amount of water and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (45:55) as the mobile phase to obtain two fractions. Each of the fractions was concentrated under reduced pressure to give Compound 49 (49.3 mg, 19 min) and Compound 50 (184.6 mg, 21 min).

### Example 28 (Compound 51)

Compound 62 (191 mg) was dissolved in methanol (10 ml)/tetrahydrofuran (4 ml), and cerium (III) chloride heptahydride (641 mg) was added thereto, followed by stirring at room temperature for 30 minutes. Next, sodium boron hydride (216 mg) was slowly added to the solution under ice cooling, followed by stirring at room temperature for 10 minutes. After diluting the reaction solution with water (30 ml), a 1N aqueous hydrochloric acid solution (3 ml) was added to adjust the pH value to be neutral, and the mixture was further diluted with water (30 ml). The aqueous layer was extracted with ethyl acetate (100 ml × 2). The resulting ethyl acetate layer was dried over anhydrous sodium sulfate and then the ethyl acetate was distilled off under reduced pressure to give a colorless oily substance (182 mg). Then, the obtained oil was dissolved in a small amount of methanol and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (30:70) as the mobile phase. The obtained fraction was concentrated under reduced pressure to give Compound 51 (137.3 mg, 29 min).

### Example 29 (Compound 52)

Radicicol (205 mg) was dissolved in acetone (10 ml), and S-chloroacetylthiophenol (330 mg) and potassium carbonate (310 mg) were added thereto, followed by stirring at room temperature for 1.5 hours. Then, water was added to the reaction solution, followed by extraction with ethyl acetate. After washing with a saturated aqueous sodium chloride solution and drying over anhydrous magnesium sulfate, the extract was dried under reduced pressure. Then, the residue was crudely purified by silica gel column chromatography (20 g, ethyl acetate:n-hexane = 1:2). The resulting crude purified product was then purified by high performance liquid chromatography (20 φ × 250 mm, YMC-Pack Pro C18, eluted with water (to which acetic acid had been added, pH 3.5):acetonitrile = 65:35) to give Compound 52 (105 mg).

### Example 30 (Compound 53)

Radicicol (406 mg) was dissolved in N,N-dimethylformamide (6 ml), and triethylamine (57.6 mg) and thiophenol (260 mg) were added thereto, followed by stirring under ice cooling overnight. Then, dilute hydrochloric acid was added to the reaction solution, followed by extraction with ethyl acetate. After washing successively with water and a saturated aqueous sodium chloride solution and drying over anhydrous magnesium sulfate, the extract was dried under reduced pressure. A 238 mg portion of the resulting residue was then purified by high performance liquid chromatography (20 φ × 250 mm, YMC-Pack Pro C18, eluted with water (to which acetic acid had been added, pH 3.5):acetonitrile = 65:35) to give Compound 53 (46.0 mg).

### Example 31 (Compounds 54 and 55)

Radicicol (378 mg) was dissolved in N,N-dimethylformamide (6 ml), and triethylamine (56.4 mg) and thioacetic acid (163 mg) were added thereto under ice cooling, followed by stirring under ice cooling overnight. Then, dilute hydrochloric acid was added to the reaction solution, followed by extraction with ethyl acetate. After washing successively with water and a saturated aqueous sodium chloride solution and drying over anhydrous magnesium sulfate, the extract was dried under reduced pressure. A 238 mg portion of the resulting residue was then purified by high performance liquid chromatography (20 φ × 250 mm, YMC-Pack Pro C18, eluted with water (to which acetic acid had been added, pH 3.5):acetonitrile = 65:35) to give Compound 54 (49.0 mg) and Compound 55 (15.7 mg).

### Example 32 (Compound 56)

Radicicol (131 mg) was dissolved in acetone (10 ml), and methyl bromoacetate (219 mg) and potassium carbonate (183 mg) were added thereto, followed by stirring at room temperature for 3 hours. Then, dilute hydrochloric acid was added to the reaction solution, followed by extraction with ethyl acetate. After washing with a saturated aqueous sodium chloride solution and drying over anhydrous magnesium sulfate, the extract was dried under reduced pressure. Then, the resulting residue was recrystallized from ethyl acetate to give Compound 56 (81.8 mg).

### Example 33 (Compound 57)

Compound 7 (116 mg) was dissolved in acetone (10 ml), and methyl I bromoacetate (330 mg) and potassium carbonate (276 mg) were added thereto, followed by stirring at room temperature for 3 days. Then, dilute hydrochloric acid was added to the reaction solution, followed by extraction with ethyl acetate. After washing with a saturated aqueous sodium chloride solution and drying over anhydrous magnesium sulfate, the extract was dried under reduced pressure. Then, the resulting residue was recrystallized from ethyl acetate to give Compound 57 (27.7 mg).

### Example 34 (Compound 58)

Radicicol (103 mg) was dissolved in N,N-dimethylformamide (6 ml), and nicotinic acid (190 mg), 1-ethyl-3-(3-dimethylaminopropyl)carbodiimide hydrochloride (270 mg) and 4-dimethyl pyridine (37.7 mg) were added thereto, followed by stirring at room temperature for 3.5 hours. Then, water was added to the reaction solution, followed by extraction with ethyl acetate. After washing with a saturated aqueous sodium chloride solution and drying over anhydrous magnesium sulfate, the extract was dried under reduced pressure. Then, the resulting residue was recrystallized from acetone-water to give Compound 58 (94.5 mg).

### Example 35 (Compound 59)

Compound 56 (312 mg) was dissolved in methanol (60 ml), and adding potassium hydroxide (206 mg)-water (20 ml) was added thereto, followed by stirring at room temperature for 15 minutes. Then, dilute hydrochloric acid was added to the reaction solution, followed by extraction with ethyl acetate. After washing with a saturated aqueous sodium chloride solution and drying over anhydrous magnesium sulfate, the extract was dried under reduced pressure. Then, the resulting residue was then purified by high performance liquid chromatography (20 φ × 250 mm, YMC-Pack Pro C18, eluted with water (to which acetic acid had been added, pH 3.5):acetonitrile = 65:35) to give Compound 59 (10.6 mg).

### Example 36 (Compounds 60, 61, 62, 63, 68 and 70)

An aseptic liquid medium (pH 6) containing 2.0% glucose, 4.0% mannitol, 2.0% oatmeal, 0.4% yeast extract, 0.001% iron (II) sulfate heptahydrate, 0.001% zinc (II) sulfate heptahydrate, 0.001% manganese (II) sulfate tetra-pentahydrate and 0.0005% copper (II) sulfate pentahydrate was inoculated with *Pochonia chlamydosporia* var. *chlamydosporia* TF-0480 strain which was then cultured therein under shaking at 26°C for 72 hours. Using a 50 L jar, an aseptic medium having the same composition as one employed in the seed culture was inoculated with 300 ml of the seed culture medium which was then cultured therein under aeration-agitation at 26°C for 144 hours. After the completion of the culturing, the culture was centrifuged to thereby separate the cells from the supernatant. The supernatant was adsorbed by 1.5 L of HP-20 (manufactured by Mitsubishi Chemical), washed with water and then eluted with 3 L of methanol. The eluate was concentrated under reduced pressure and the obtained aqueous layer was extracted with ethyl acetate. The ethyl acetate layer was dehydrated over anhydrous sodium sulfate and concentrated under reduced pressure to give a brown syrupy substance (60 g).

A portion (30 g) of the culture extract was dissolved in a solvent mixture of chloroform-methanol (4:1) and adsorbed by a silica gel [Silica Gel 60 (trade name, manufactured by Merck)] column (1600 ml) prepared by moistening with n-hexane. Next, elution was successively carried out with n-hexane-ethyl acetate mixtures to give a fraction (130 mg) eluted with a solvent mixture of n-hexane-ethyl acetate (4:1), (2:1)-eluted fractions (1) (1.88 g) and (2) (780 mg), and a (1:2)-eluted fraction (3.56 g). The n-hexane-ethyl acetate (4:1)-eluted fraction (130 mg) was dissolved in a small amount of acetone and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (55:45) as the mobile phase to obtain two fractions. Each of the fractions was concentrated under reduced pressure to give Compound 63 (23.5 mg, 20 min) and Compound 70 (4.8 mg, 24 min). The n-hexane-ethyl acetate (2:1)-eluted fraction (1.88 g) was recrystallized from acetone (30 ml) to give Compound 61 (277 mg). The eluted fraction (2) (780 mg) was purified by preparative thin layer chromatography [Silica Gel 60 F254 (trade name, manufactured by Merck), 20 cm × 20 cm, 1 mm in thickness] using n-hexane-acetone (3:2) as the developing solvent. A part with an Rf value of 0.4 was collected, extracted with ethyl acetate and then concentrated under reduced pressure to give Compound 60 (80.3 mg). Next, the n-hexane-ethyl acetate (1:2)-eluted fraction (3.56 g) was dissolved in a solvent mixture of chloroform-methanol (4:1) and adsorbed by a silica gel [Silica Gel 60 (trade name, manufactured by Merck)] column (880 ml) prepared by moistening with chloroform. Next, elution was successively carried out with a solvent mixture of chloroform-methanol to give a chloroform-methanol (98:2)-eluted fraction (926 mg). Then, it was recrystallized from acetone-methanol (1 ml/3 ml) to give Compound 62 (339 mg). A chloroform-methanol (97:3)-eluted fraction (1.42 g) was purified by column chromatography using Sephadex LH-20 (trade name, manufactured by Amersham Pharmacia Biotech) (methanol, 900 ml), followed by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (30:70) as the mobile phase. The obtained fraction was concentrated under reduced pressure to give Compound 68 (4.5 mg, 21 min).

### Example 37 (Compounds 64, 65, 71, 72 and 76)

A portion (30 g) of a culture extract obtained as in Example 36 was dissolved in a solvent mixture of chloroform-methanol (4:1) and adsorbed by a silica gel [Silica Gel 60 (trade name, manufactured by Merck)] column (700 ml) prepared by moistening with chloroform. Next, elution was successively carried out with a solvent mixture of chloroform-methanol. A chloroform-methanol (93:7)-eluted fraction was divided into 2 portions, i.e., fractions (1) (156 mg) and (2) (164 mg). Fraction (1) was dissolved in a small amount of methanol and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (30:70) as the mobile phase to obtain four fractions. Each of the fractions was concentrated under reduced pressure to give Compound 64 (35.4 mg, 21 min), Compound 72 (13.7 mg, 27 min), Compound 71 (6.6 mg, 29 min) and Compound 65 (4.1 mg, 33 min). Fraction (2) was purified by preparative high performance liquid chromatography under the same conditions, and the obtained fractions were collected and concentrated under reduced pressure to give Compound 71 (3.7 mg) together with Compound 76 (4.5 mg, 20 min).

### Example 38 (Compounds 69 and 74)

A culture extract (39.5 g) obtained as in Example 36 was dissolved in n-hexane (300 ml x 2). The precipitate thus formed was filtered and the solvent was removed. The obtained precipitate (8.1 g) was dissolved in a solvent mixture of chloroform-methanol (4:1) and adsorbed by a silica gel [Silica Gel 60 (trade name, manufactured by Merck)] column (650 ml) prepared by moistening with chloroform. Next, elution was successively carried out with a solvent mixture of chloroform-methanol to give a chloroform-methanol (88:12)-eluted fraction (318 mg). This fraction was divided in a small amount of methanol and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (30:70) as the mobile phase to obtain two fractions. Each of the fractions was concentrated under reduced pressure to give Compound 74 (15.3 mg, 19 min) and Compound 69 (8.0 mg, 38 min).

### Example 39 (Compound 66)

A culture extract (39.5 g) obtained as in Example 36 was dissolved in n-hexane (300 ml × 2). The precipitate thus formed was filtered and the solvent was removed. The obtained precipitate (8.1 g) was dissolved in a solvent mixture of chloroform-methanol (4:1) and adsorbed by a silica gel [Silica Gel 60 (trade name, manufactured by Merck)] column (650 ml) prepared by moistening with chloroform. Next, elution was successively carried out with a solvent mixture of chloroform-methanol to give a chloroform-methanol (100:0)-eluted fraction (1.5 mg). Next, it was applied to silica gel column (620 ml) using a solvent mixture of n-hexane-ethyl acetate to give an n-hexane-ethyl acetate (2:1)-eluted fraction (321 mg). The obtained fraction was dissolved in a small amount of acetone and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (under gradient of 45:55 to 70:30) as the mobile phase to give Compound 66 (14.4 mg, 34 min).

### Example 40 (Compounds 73, 77 and 78)

A culture extract (500 g) obtained as in Example 36 was dissolved in a solvent mixture of chloroform-methanol (4:1) and adsorbed by a silica gel [Silica Gel 60 (trade name, manufactured by Merck)] column (6000 ml) prepared by moistening with chloroform. Next, elution was successively carried out with a solvent mixture of chloroform-methanol. Thus, a chloroform-methanol (95:5)-eluted fraction (57 g) was obtained. A portion (18 g) of this fraction was further subjected to silica gel column (800 ml) using a solvent mixture of n-hexane-ethyl acetate. An n-hexane-ethyl acetate (2:1)-eluted fraction was divided into 2 portions, i.e., fractions (1) (126 mg) and (2) (985 mg). Fraction (1) was dissolved in a small amount of acetone and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (55:45) as the mobile phase. The obtained fraction was concentrated under reduced pressure to give Compound 78 (13.4 mg, 26 min). On the other hand, fraction (2) was purified by preparative high performance liquid chromatography using acetonitrile-water (pH 3.5, acetic acid) (45:55) as the mobile phase to obtain the two fractions. Each of the fractions was concentrated under reduced pressure to give Compound 73 (38.1 mg, 10 min) and Compound 77 (67.2 mg, 26 min).

### Example 41 (Compounds 67 and 75)

A culture extract (500 g) obtained as in Example 36 was dissolved in a solvent mixture of chloroform-methanol (4:1) and adsorbed by a silica gel [Silica Gel 60 (trade name, manufactured by Merck)] column (6000 ml) prepared by moistening with chloroform. Next, elution was successively carried out with a solvent mixture of chloroform-methanol. Thus, a chloroform-methanol (95:5)-eluted fraction (57 g) was obtained. A portion (18 g) of this fraction was further subjected to silica gel column (800 ml) using a solvent mixture of n-hexane-ethyl acetate to obtain an n-hexane-ethyl acetate (1:2)-eluted fraction. This fraction was dissolved in a small amount of acetone and purified by preparative high performance liquid chromatography [column: YMC-Pack Pro C18 AS-343 (20 φ × 250 mm), detection: UV absorption at 254 nm, flow rate: 10 ml/min] using acetonitrile-water (pH 3.5, acetic acid) (40:60) as the mobile phase to obtain two fractions. Each of the fractions was concentrated under reduced pressure to give Compound 67 (17.4 mg, 11 min) together with a semi-pure sample (23.9 mg). This semi-pure sample was purified by preparative thin layer chromatography [Silica Gel 60 F254 (trade name, manufactured by Merck), 20 cm × 20 cm, 0.5 mm in thickness] using chloroform-methanol (90:10) as the developing solvent. A part with an Rf value of 0.5 was collected, extracted with ethyl acetate and then concentrated under reduced pressure to give Compound 75 (4.3 mg).

Table 2 shows the compounds synthesized and purified in the above Examples and data thereof.

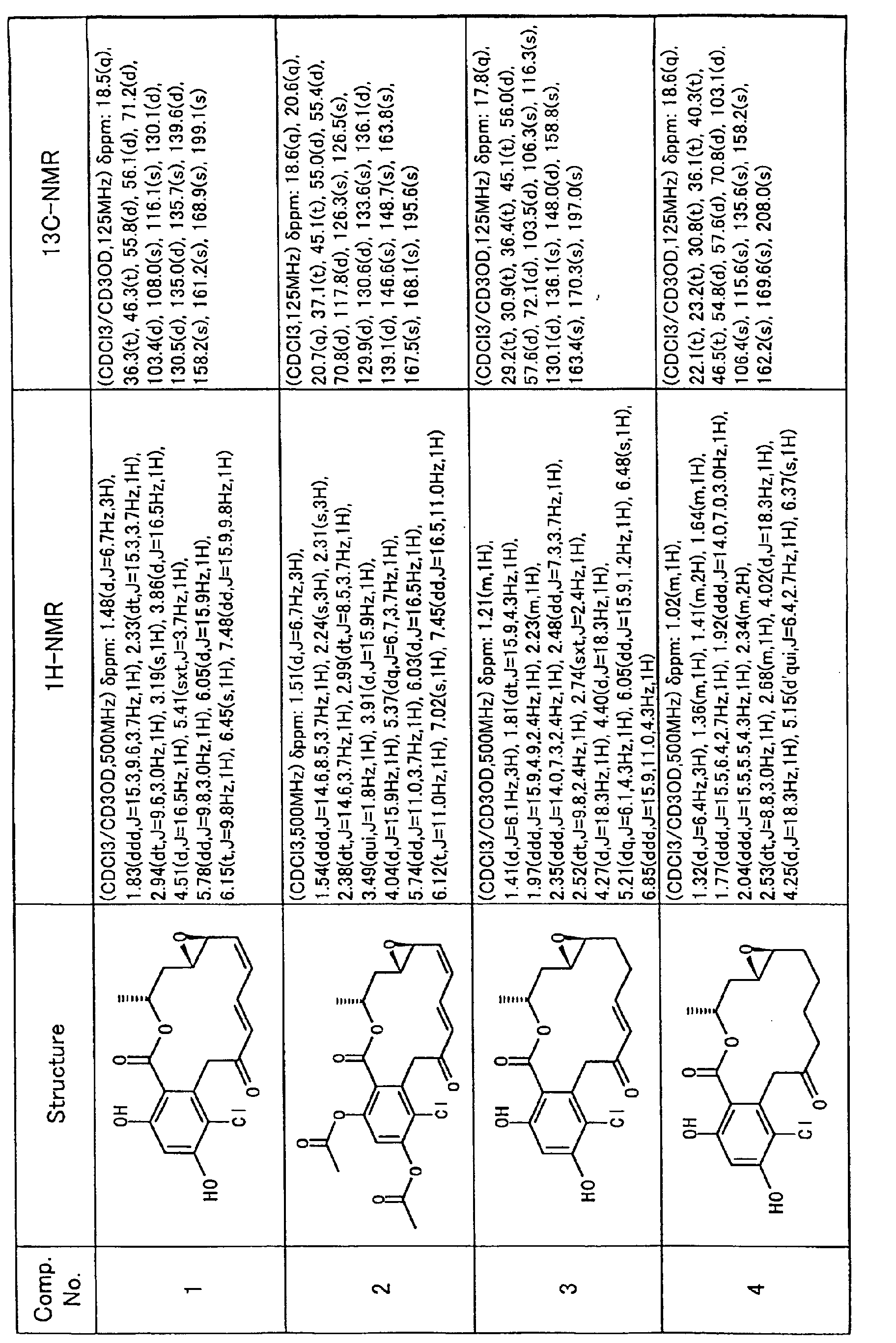

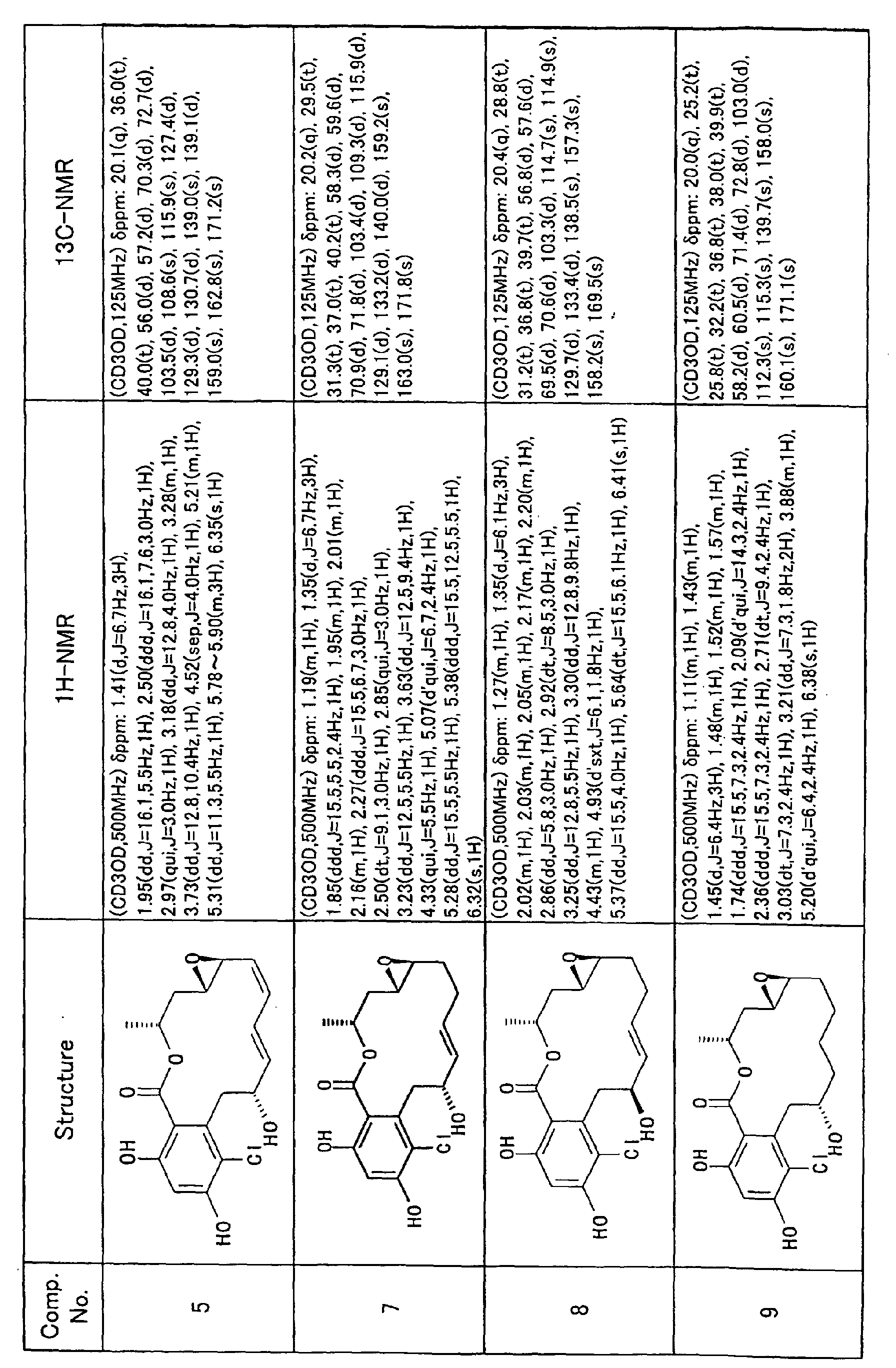

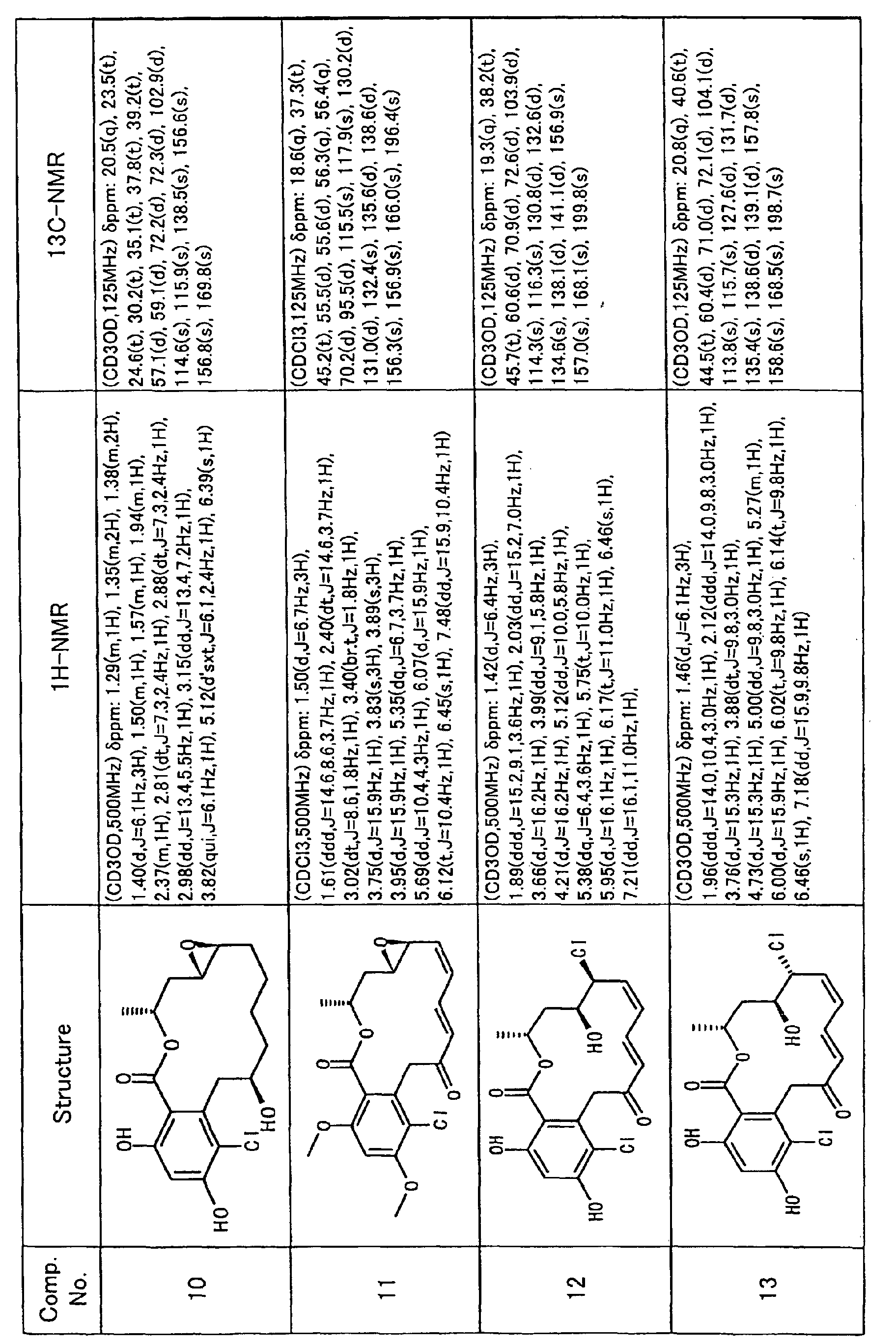

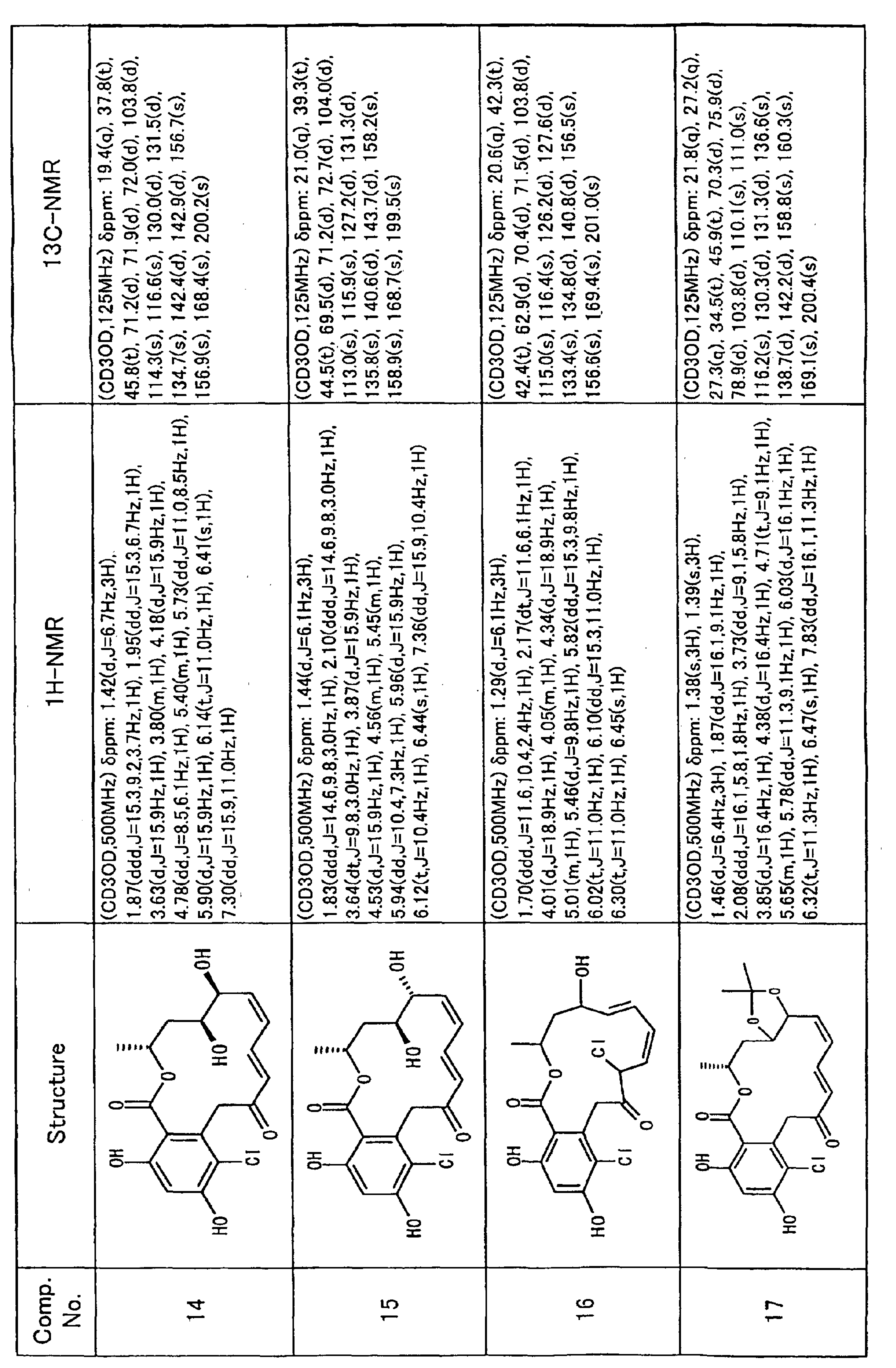

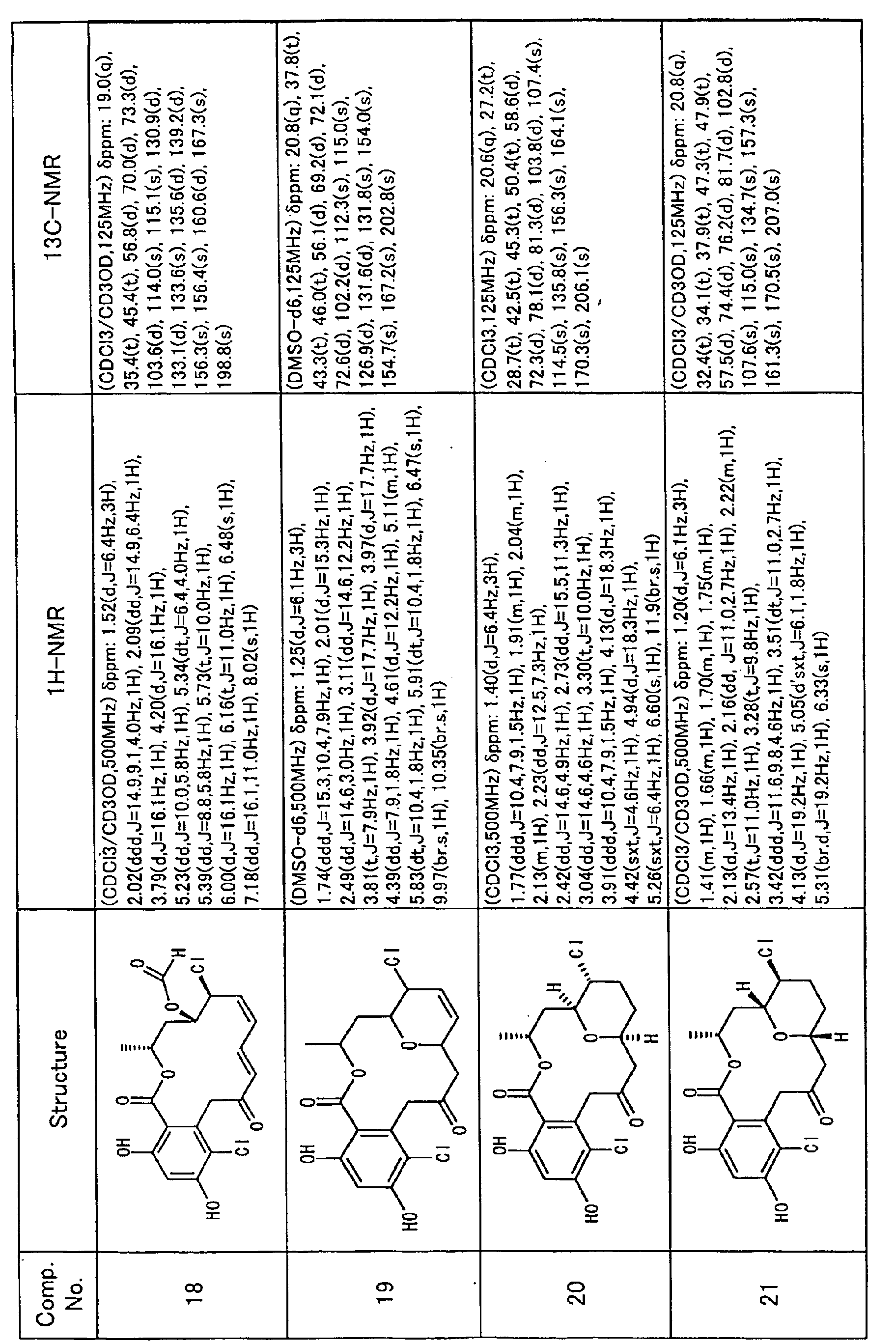

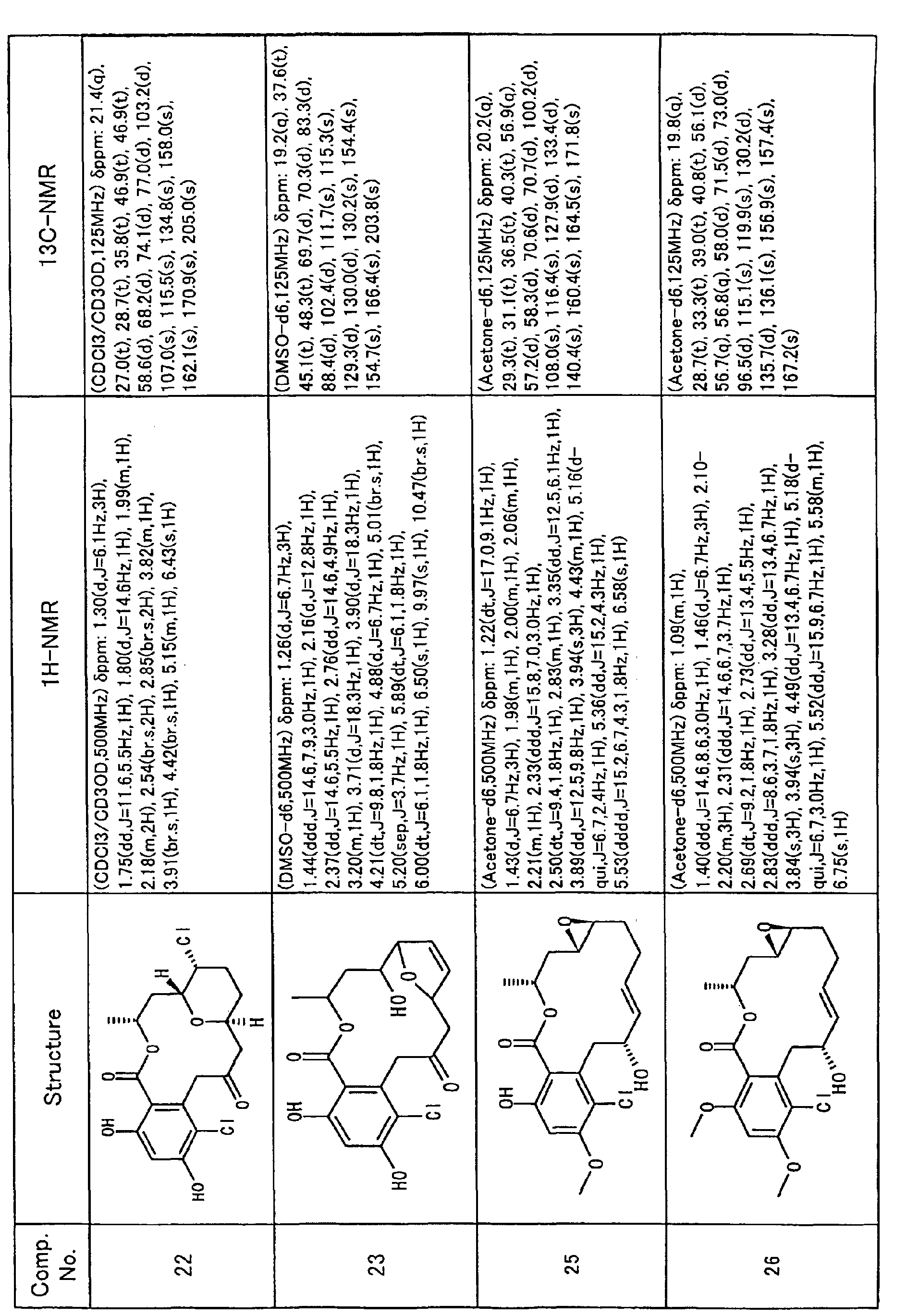

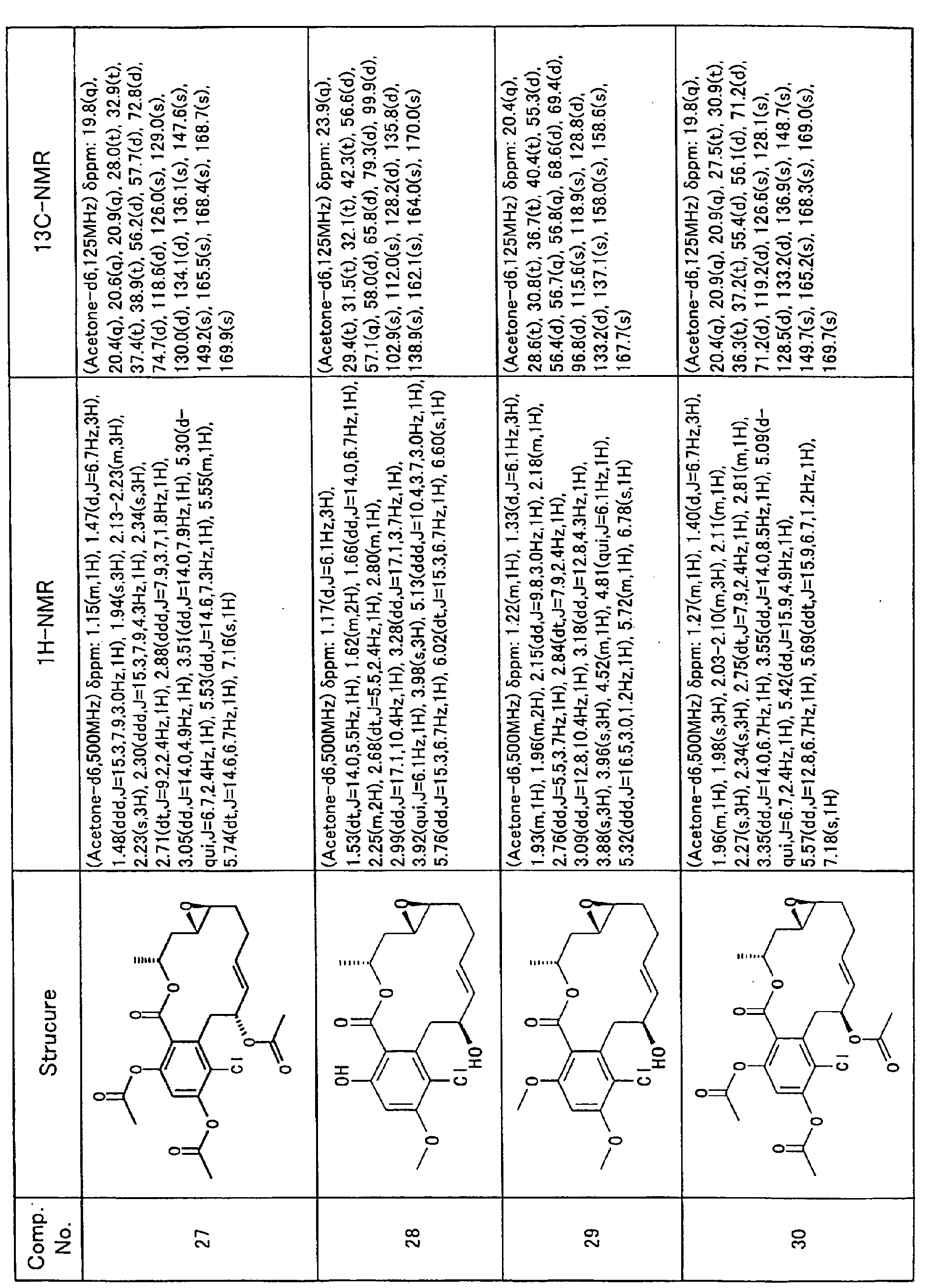

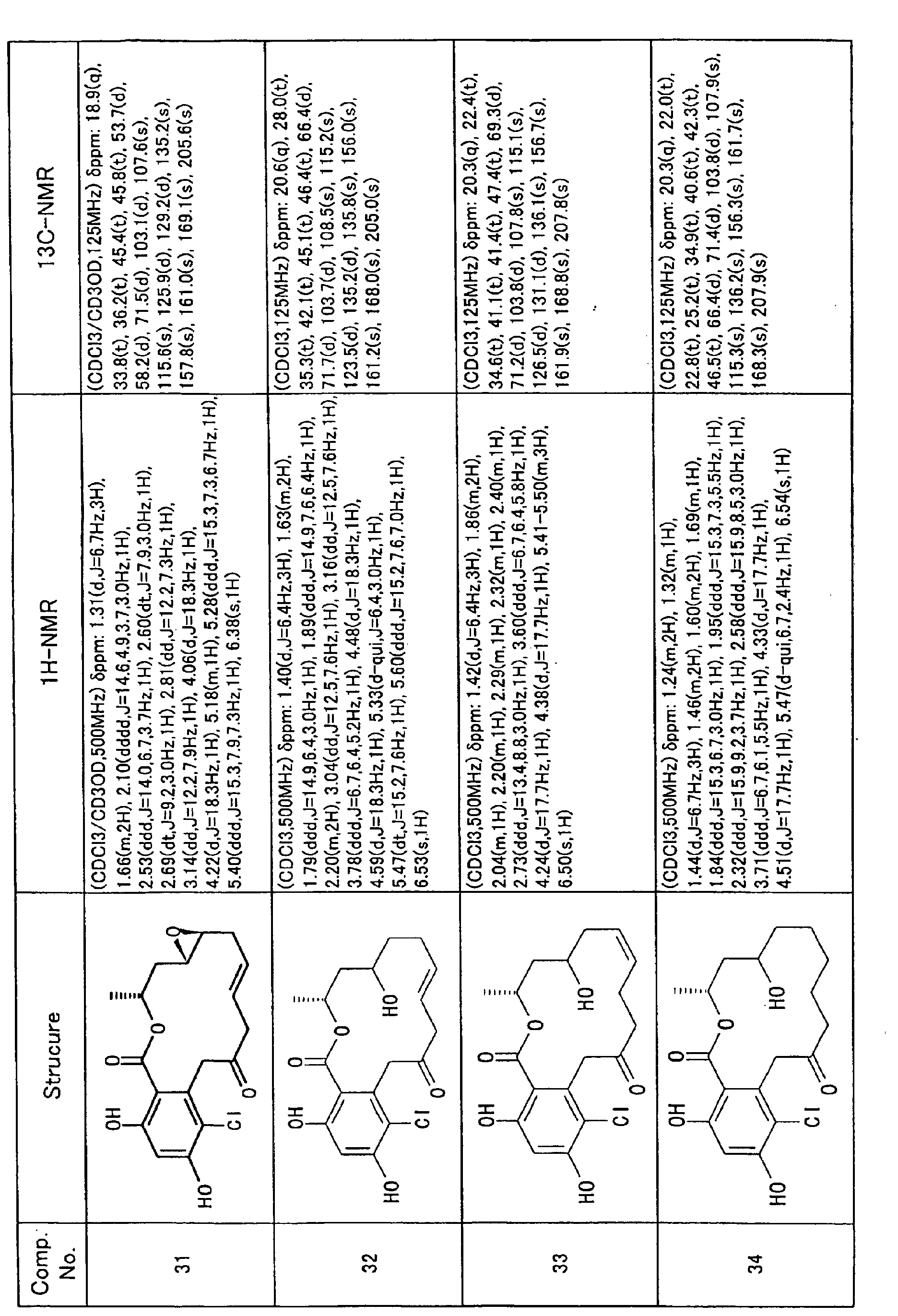

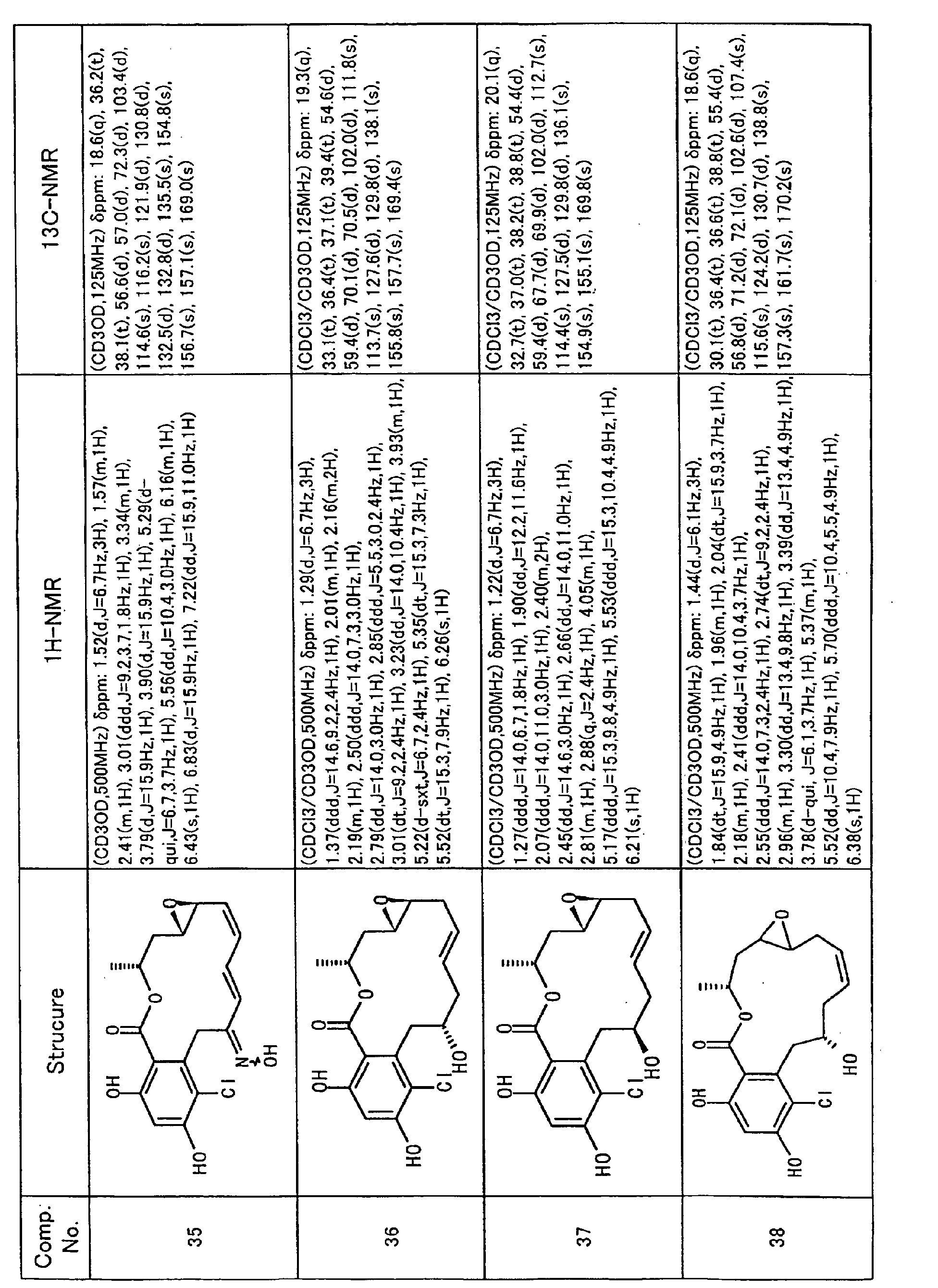

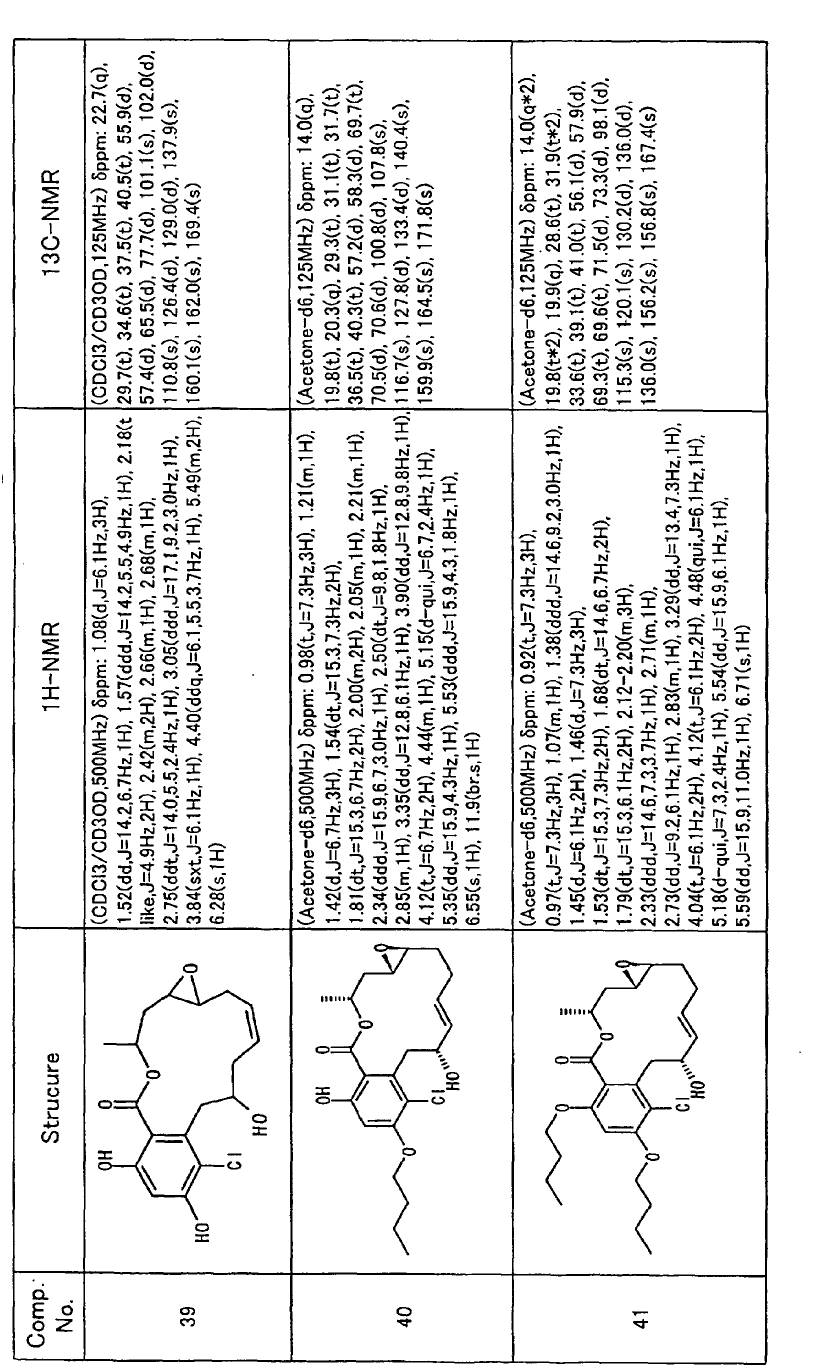

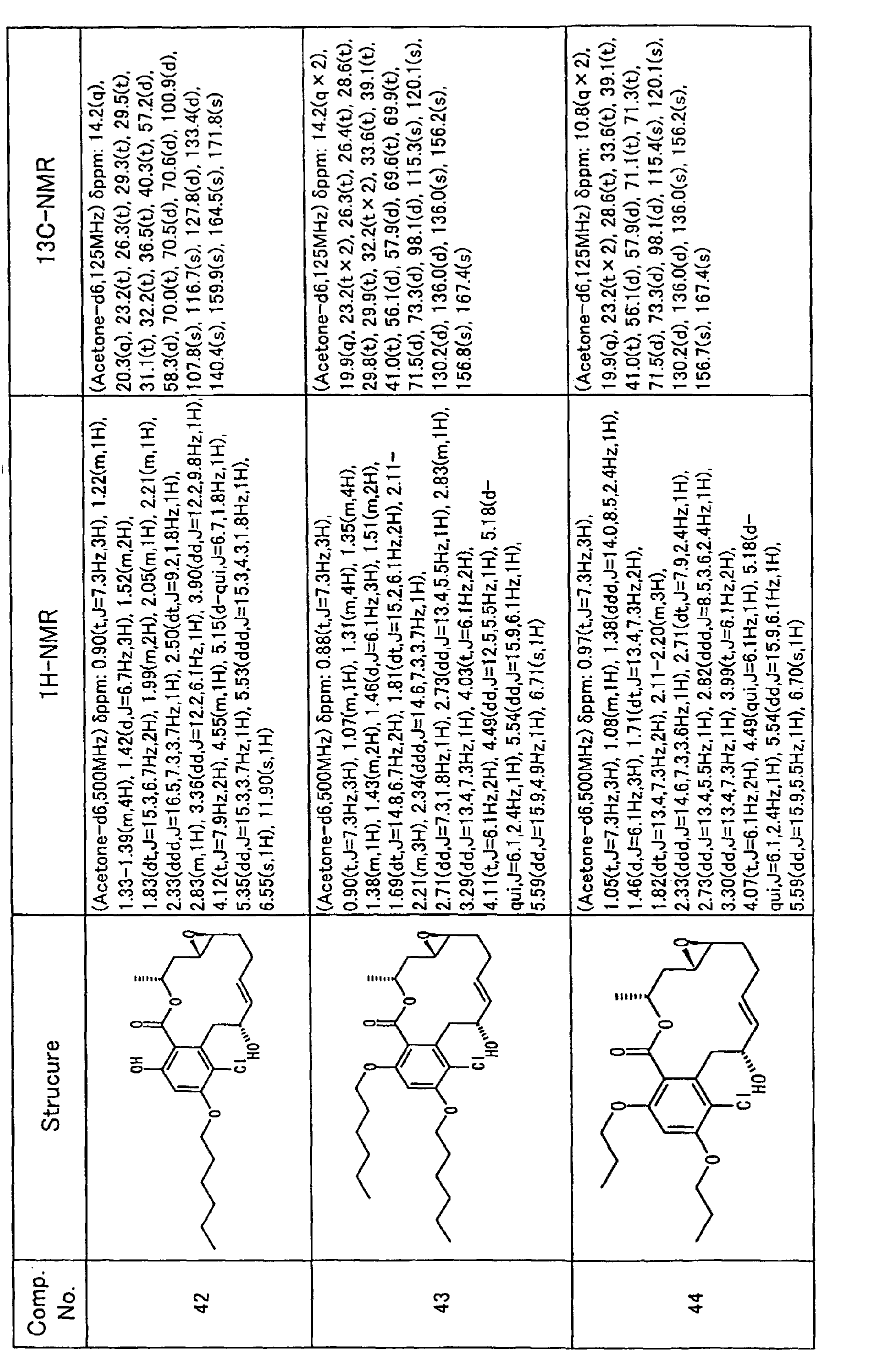

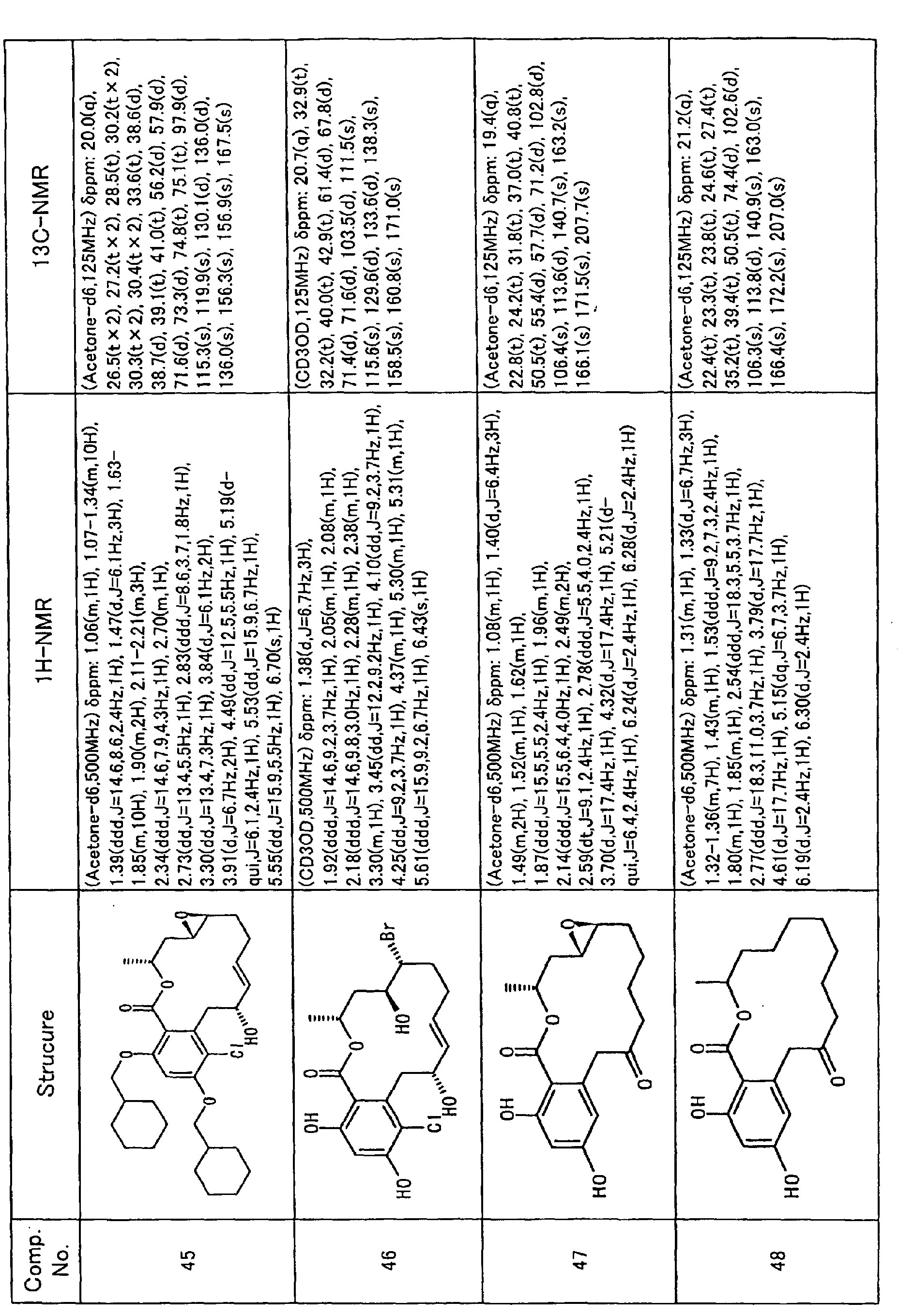

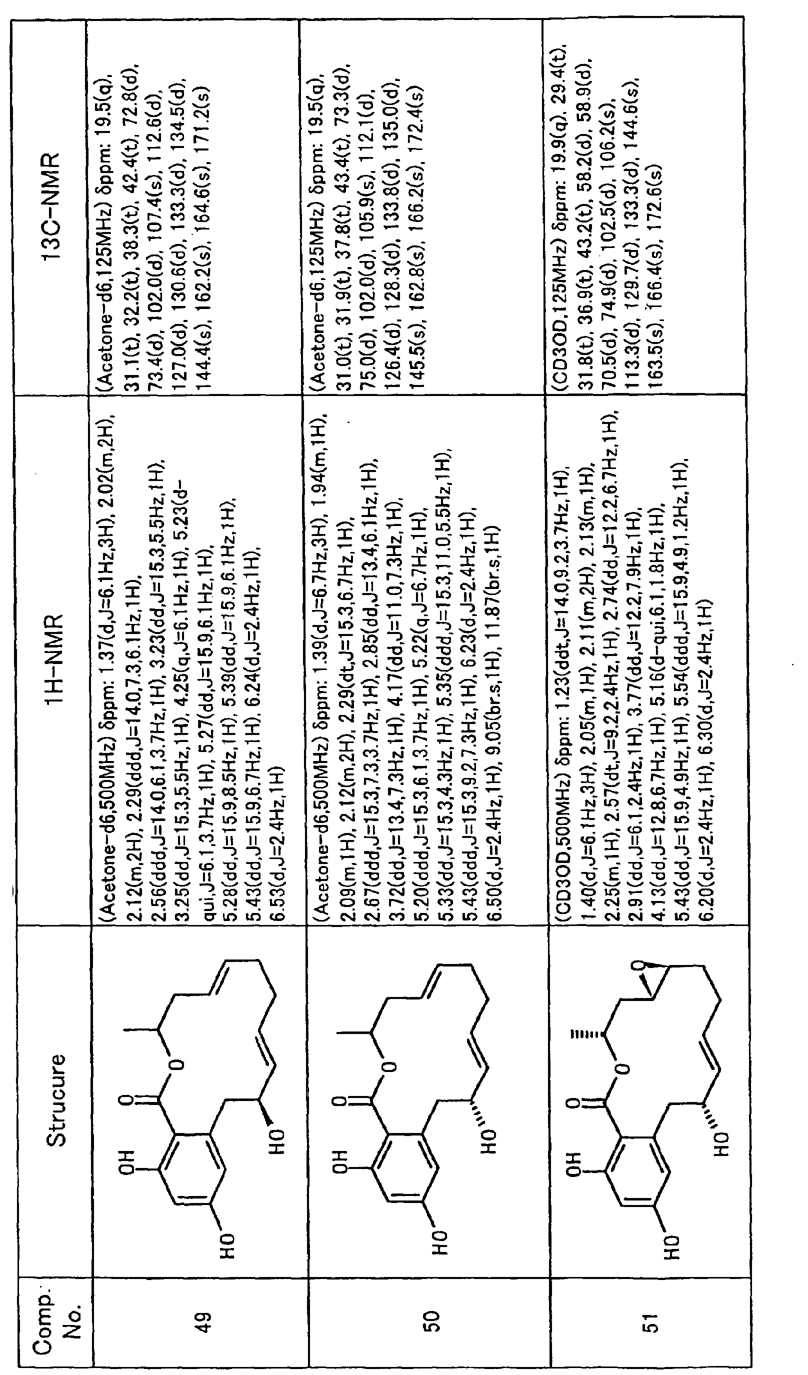

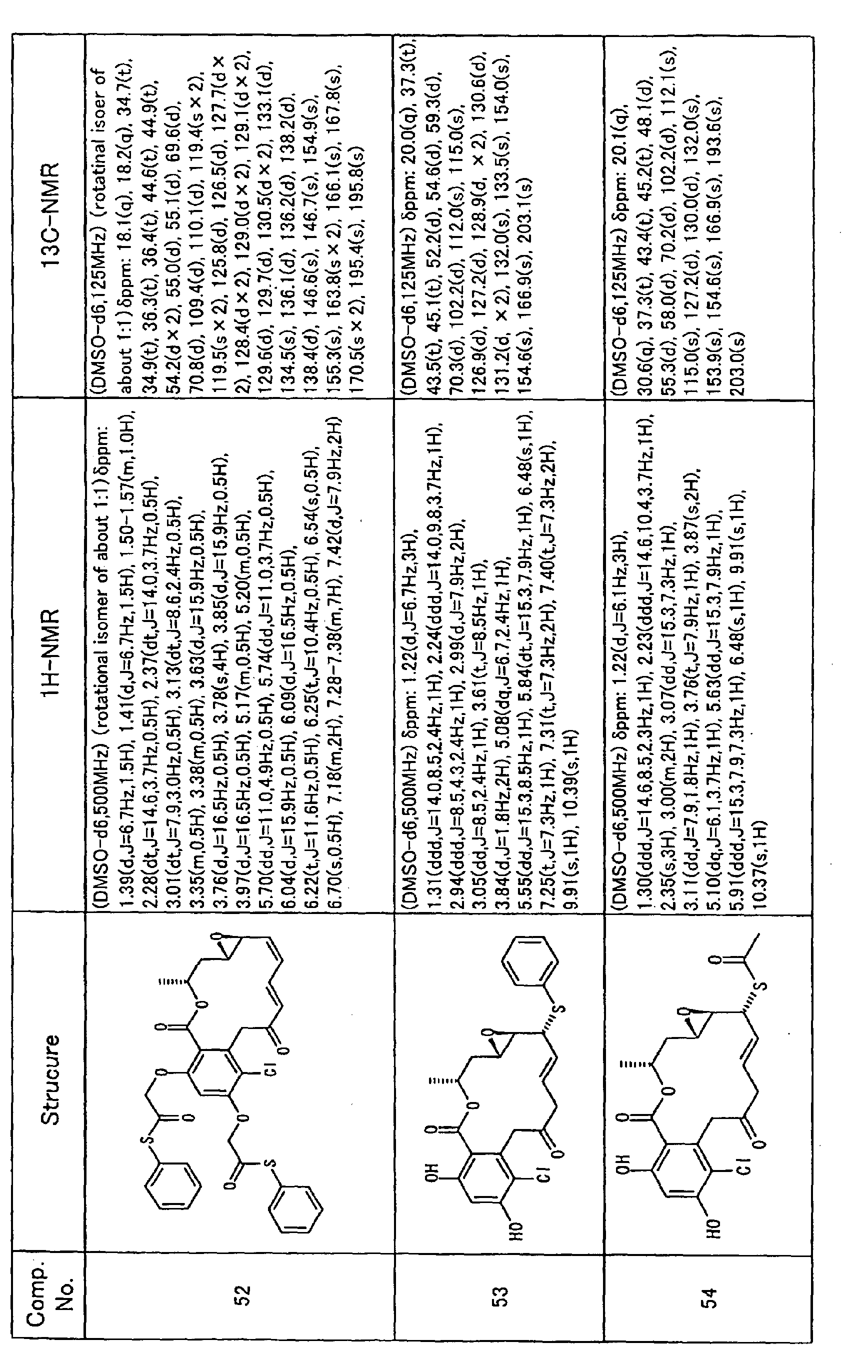

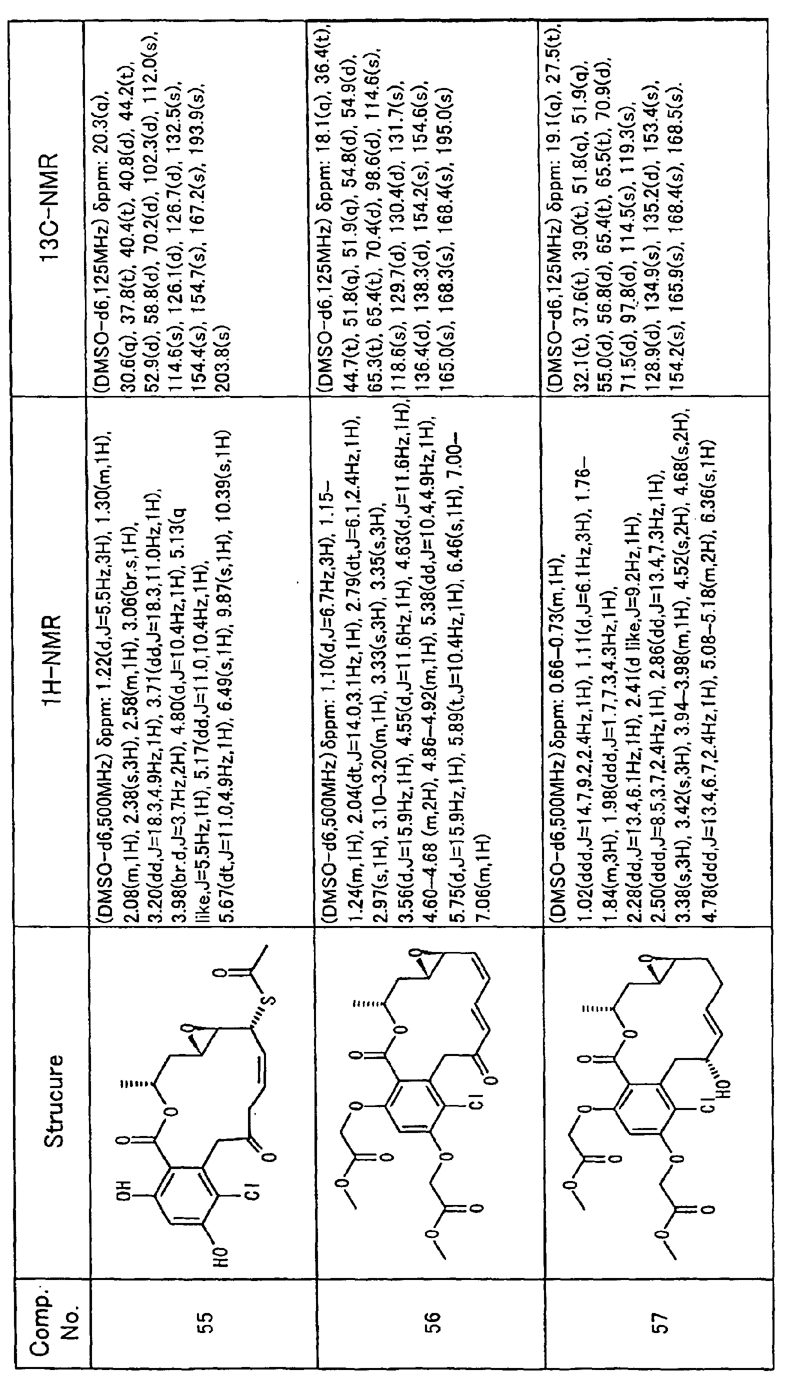

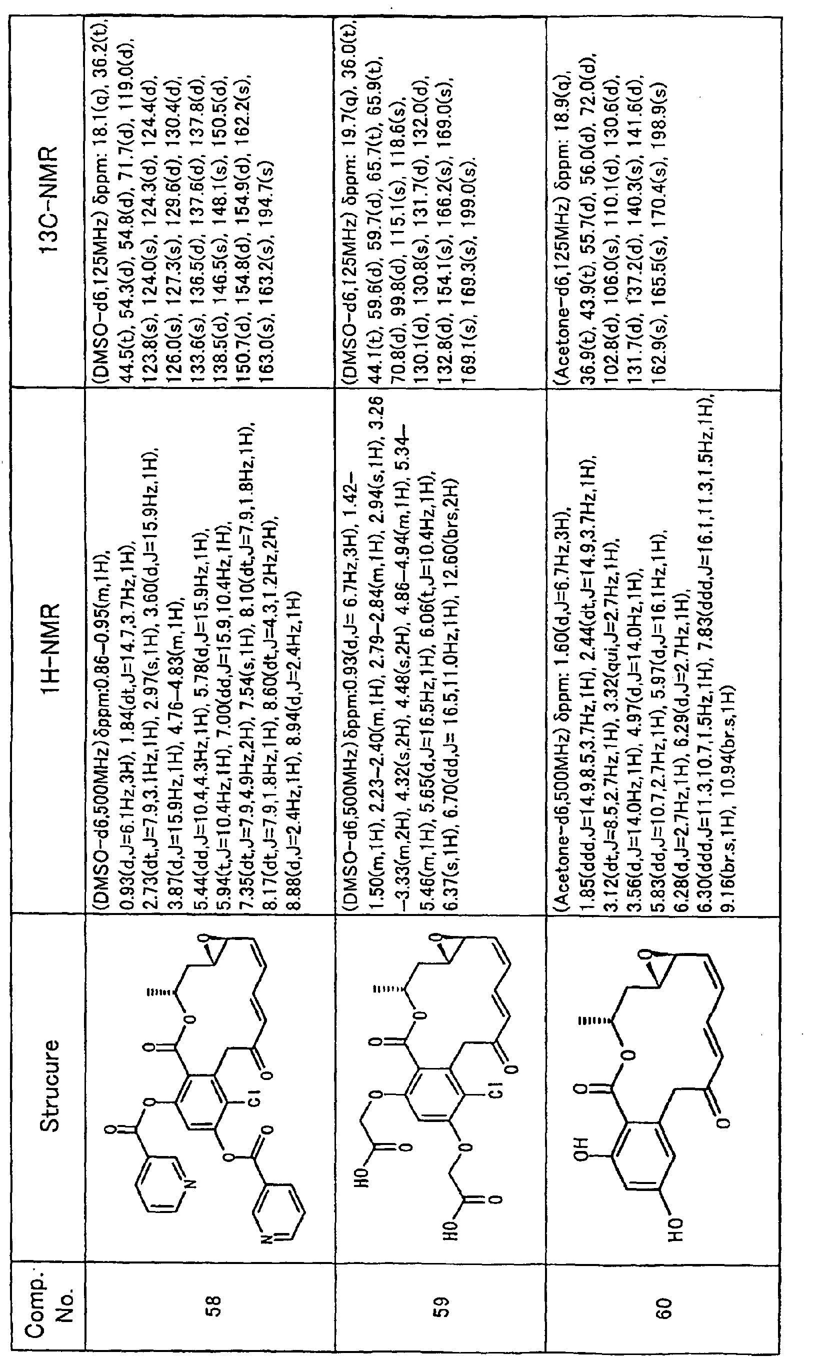

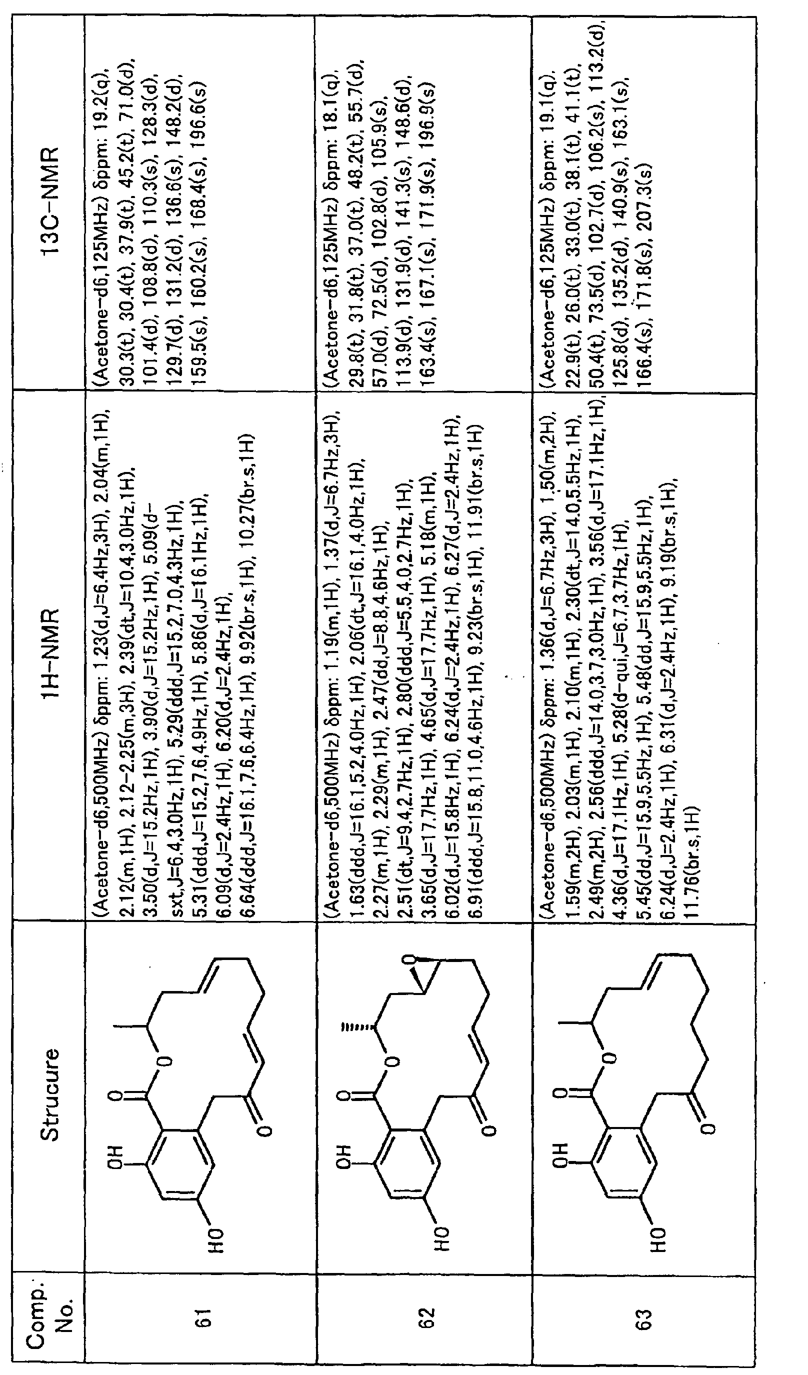

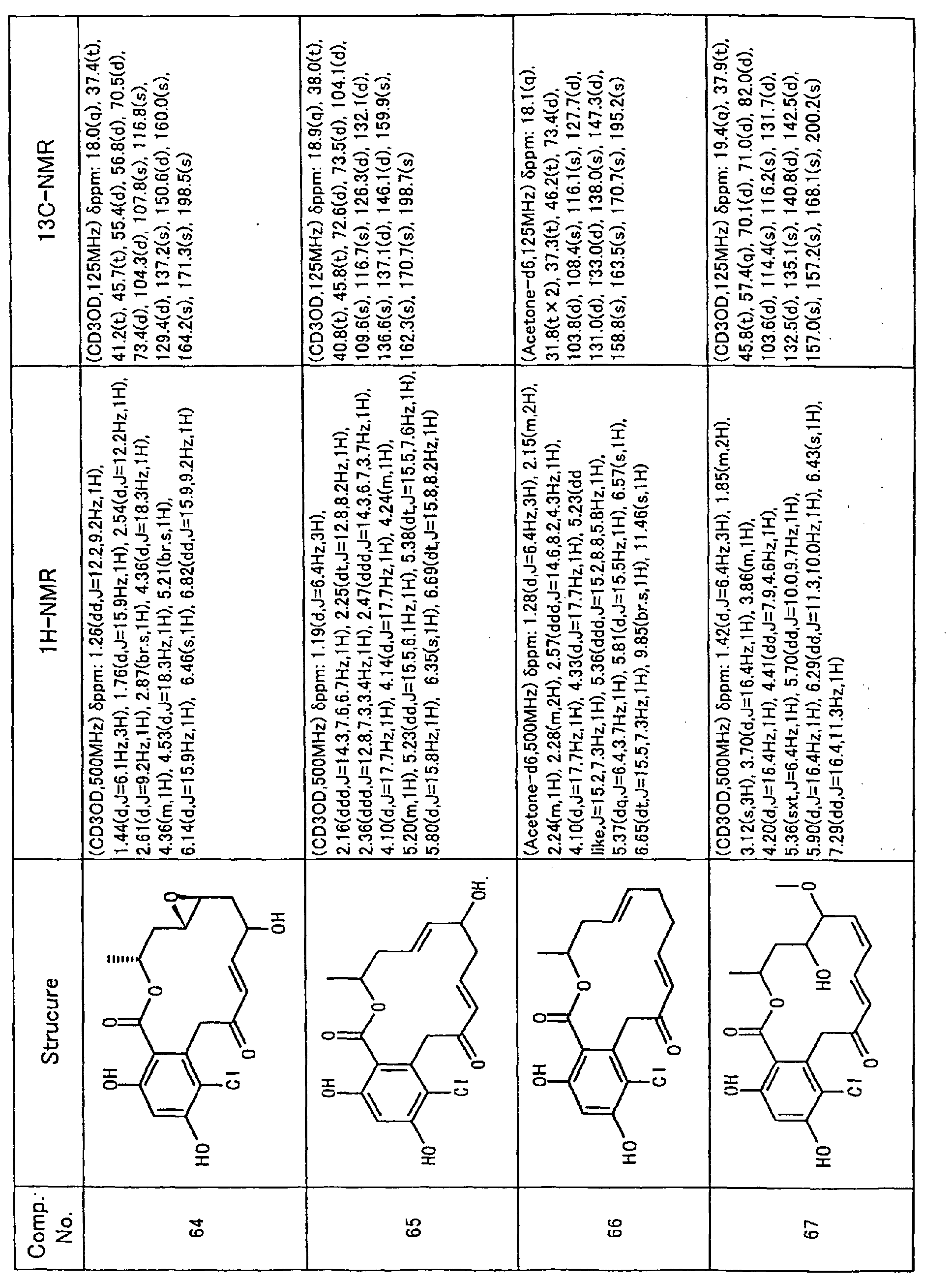

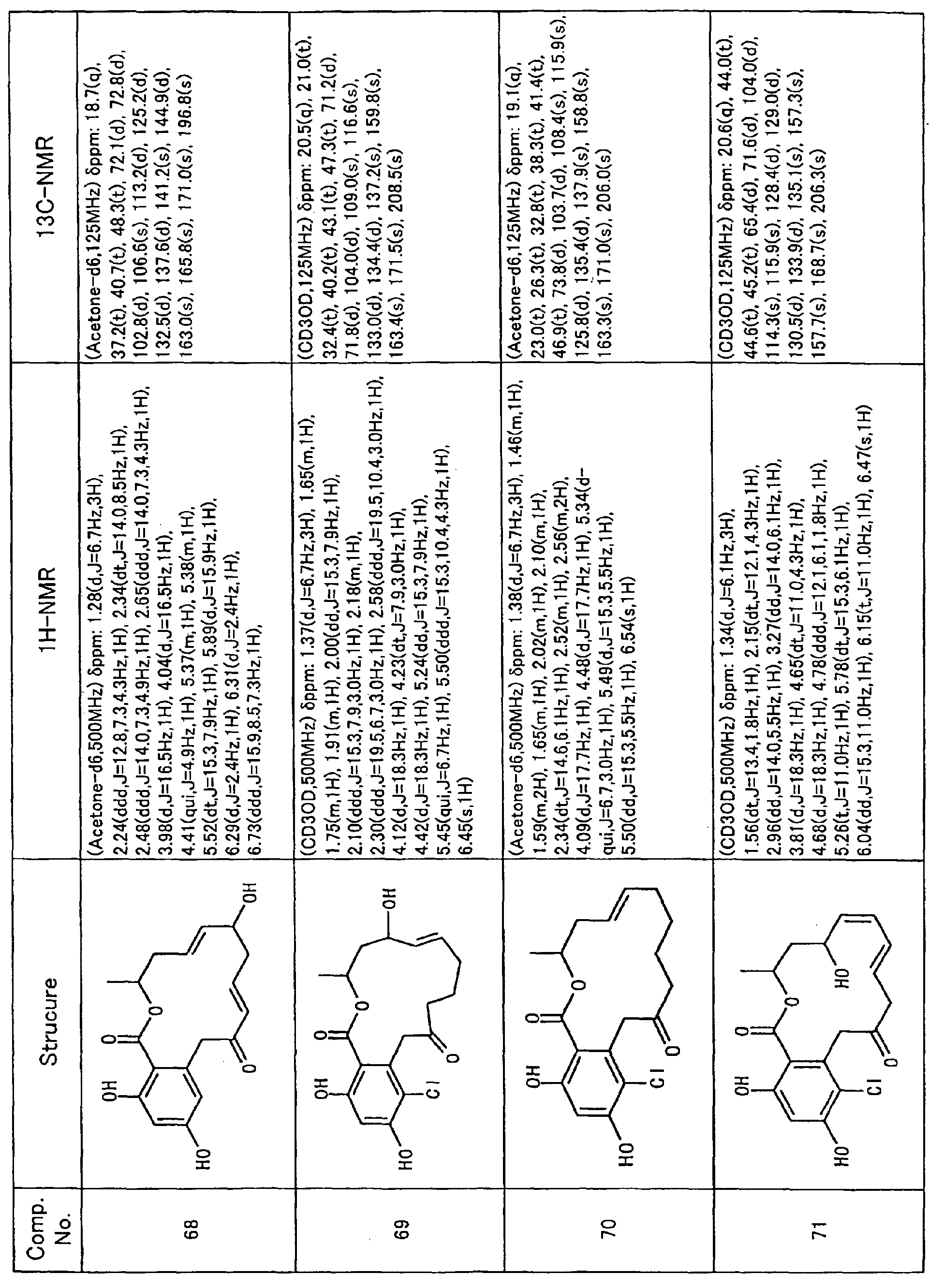

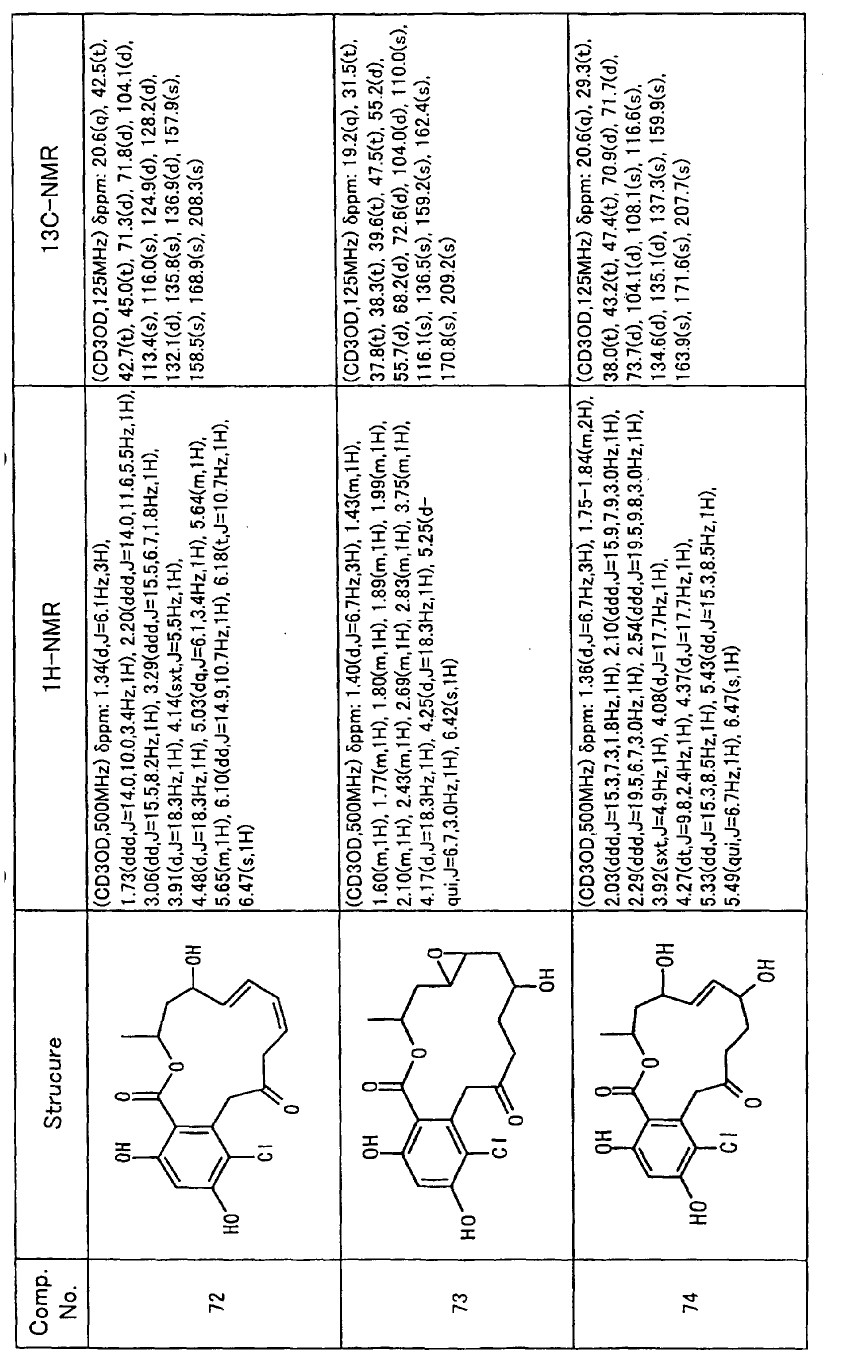

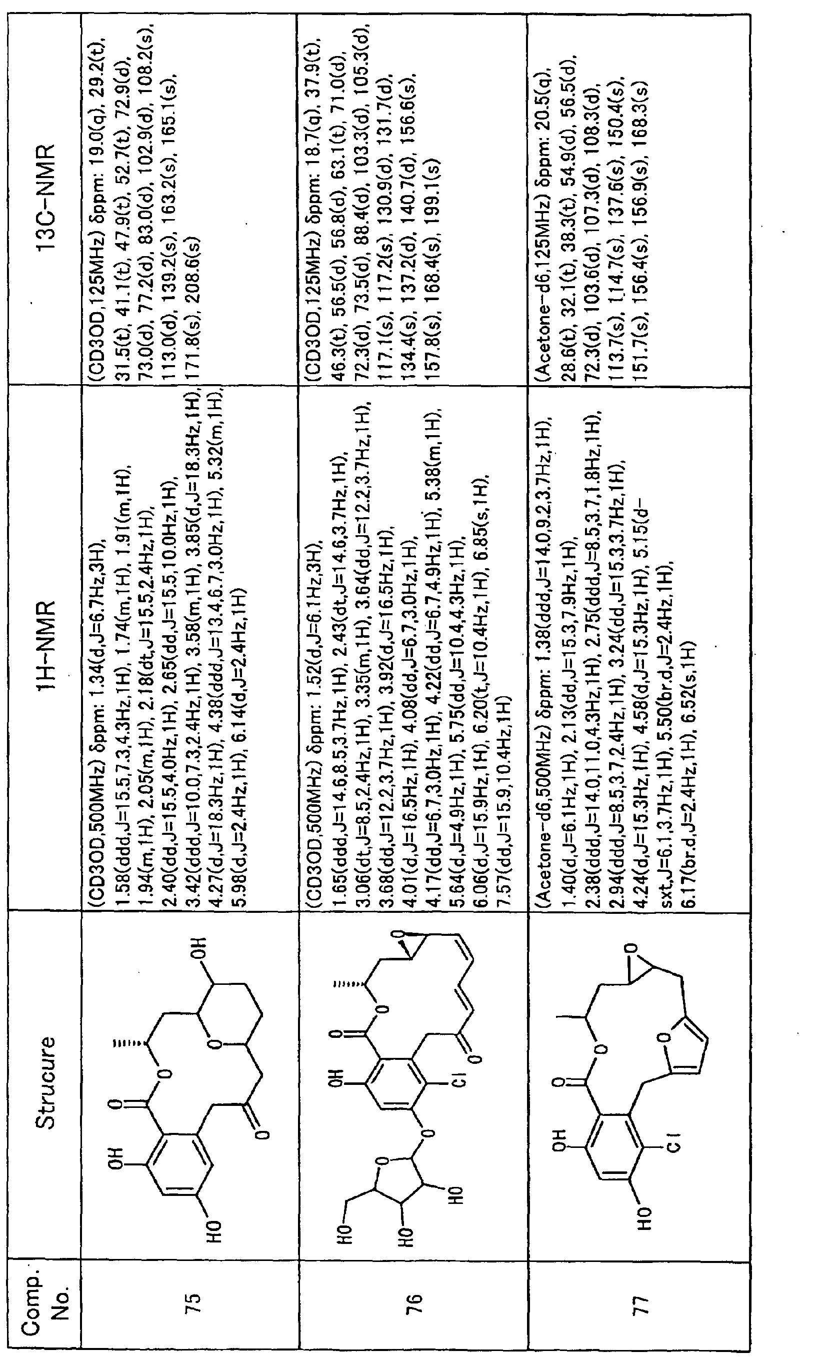

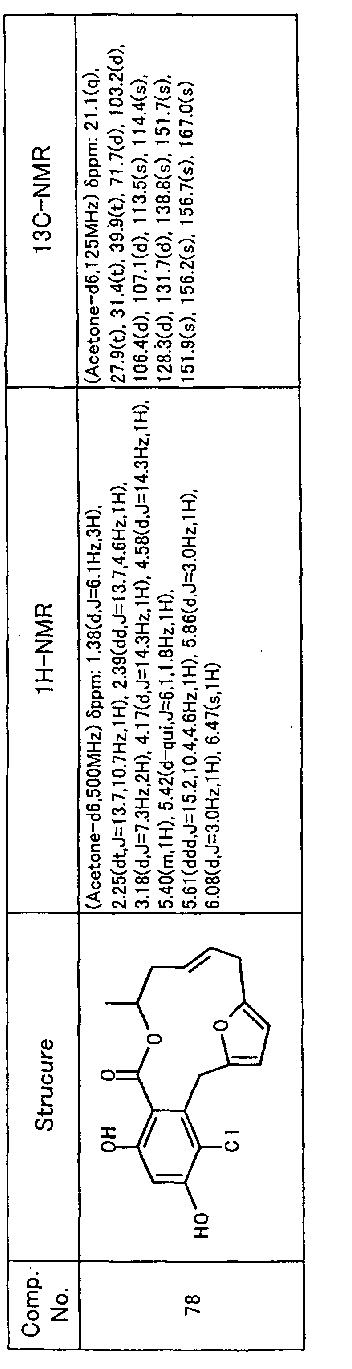

### Test Example 1

### Expression of WNT-5A mRNA in dermal papilla cells:

Human dermal papilla cells, which were purchased from Toyobo, were cultured in MEM (Invitrogen) containing 12% FBS. Human hair follicle cells were separated from plucked hairs in accordance with the method of Arase *et al. (J. Dermatol. Sci.,* 2, 66-70 (1991)) and cultured by using KGM-2 (Sanko Junyaku).

The dermal papilla cells in passage 5 and the hair follicle cells in passage 2 were sowed in a 10 cm dish to give a density of 2×10⁶ cells/well and cultured over 2 nights. After removing the medium, the cells were washed with PBS(-) and total RNAs were extracted by using TRIzol reagent (Invitrogen). Using 50 ng of the total RNAs, 0.4 µM primers specific to WNT-5A or glyceraldehyde-3-phosphate dehydrogenase (GAPDH) [WNT-5A forward: AATGTCTTCC AAGTTCTTCC TAGTGGC (SEQ ID NO:8), WNT-5A reverse: GATGTCGGAA TTGATACTGG CA (SEQ ID NO:9), GAPDH forward: ACCACAGTCC ATGCCATCAC (SEQ ID NO: 10) and GAPDH reverse: TCCACCACCC TGTTGCTGTA (SEQ ID NO:11)] and SUPERSCRIPT One-Step RT-PCT with PLATINUM Taq (Invitrogen), RT-PCR was carried out in accordance with the protocol attached to SUPERSCRIPT One-Step RT-PCT with PLATINUM Taq. In brief, first strand synthesis was carried out at 50°C for 30 minutes in a reaction system of 25 µl in total volume. After heating to 94°C for 2 minutes, each cDNA fragment was amplified in 23 or 20 cycles with each cycle consisting of 30 seconds at 94°C, 30 seconds at 55°C and 30 seconds at 72°C.

Each reaction solution was electrophoresed in a 1.5% agarose gel and stained with ethidium bromide. Fig. I shows the results.

In the case of using the dermal papilla cell (DPC)-derived RNA, a remarkable amplification of the cDNA fragment was observed in the 23-cycle PCR using the WNT-5A-specific primers. In the case of using the hair follicle, cell (HFC)-derived RNA, no amplified product was observed under the same conditions.

### Test Example 2

### Measurement of activity of decreasing WNT-5A mRNA amount:

Human dermal papilla cells, which were purchased from Toyobo, were cultured in MEM (Invitrogen) containing 12% FBS.

The dermal papilla cells in passage 5 were sowed in a 12-well plate to give a density of 1.6×10⁵ cells/well and cultured overnight. Then, the medium was replaced by a medium to which no compound was added or a medium containing Compound 1, 2, 3, 4, 5, 7, 9 or 24 and the culture was continued for additional 24 hours. After the completion of the culture, each medium was removed and the cells were washed with PBS(-). Then total RNAs were extracted by using TRIzol reagent (Invitrogen). Using 50 ng of the total RNAs, 0.4 µM of primers specific to WNT-5A or GAPDH [WNT-5A forward: AATGTCTTCC AAGTTCTTCC TAGTGGC (SEQ ID NO:8), WNT-5A reverse: GATGTCGGAA TTGATACTGG CA (SEQ ID NO:9), GAPDH forward: ACCACAGTCC ATGCCATCAC (SEQ ID NO:10) and GAPDH reverse: TCCACCACCC TGTTGCTGTA (SEQ ID NO: 11)] and SUPERSCRIPT One-Step RT-PCT with PLATINUM Taq (Invitrogen), RT-PCR was carried out in accordance with the protocol attached to SUPERSCRIPT One-Step RT-PCT with PLATINUM Taq. In brief, first strand synthesis was carried out at 50°C for 30 minutes in a reaction system of 25 µl in total volume. After heating to 94°C for 2 minutes, the cDNA fragment of WNT-5A or GAPDH was amplified in 23 or 20 cycles with each cycle consisting of 30 seconds at 94°C, 30 seconds at 55°C and 30 seconds at 72°C.

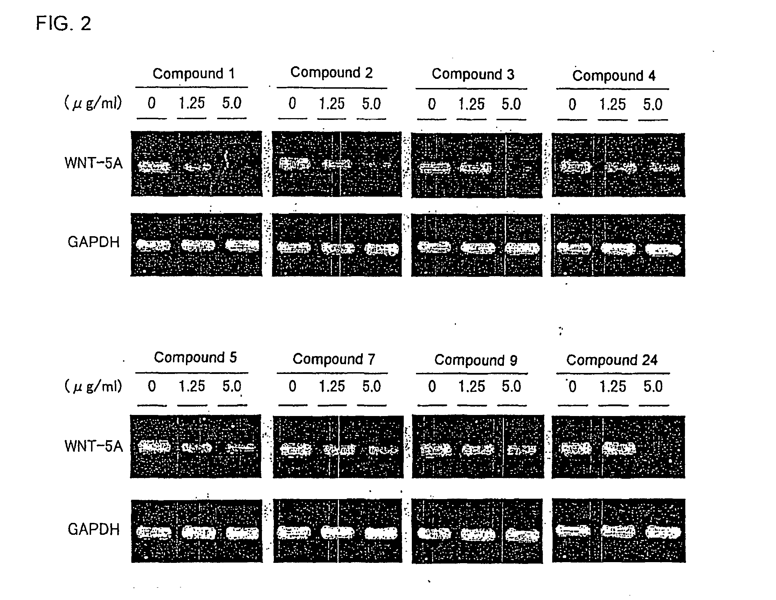

Each reaction solution was electrophoresed in a 1.5% agarose gel and stained with ethidium bromide. Fig. 2 shows the results.

In the 23-cycle PCR using the WNT-5A-specific primers, the amplified WNT-SA cDNA fragments was remarkably decreased in the cultures with the compounds, compared with the culture using the medium to which no compound was added.

In the 20-cycle PCR using the GAPDH-specific primers, on the other hand, no change was observed in the amount of amplified cDNA fragment regardless of the presence or absence of the compounds.

### Test Example 3

### Dermal papilla cell growth promotion activity test:

Human dermal papilla cells, which were purchased from Toyobo, were cultured in MEM (Invitrogen) containing 12% FBS.

The dermal papilla cells in passage 5 were sowed in a 96-well plate for spheroid culture to give a density of 1.5×10⁴ cells/well and cultured overnight. Then, the medium was replaced by a medium to which no compound was added or a medium containing Compound 1, 2, 3, 4, 5, 7, 9 or 24 and the culture was continued for additional 72 hours. After the completion of the culture, cells were counted by using Cell Counting Kit (Wako Pure Chemicals). Five hours before the completion of the culture, WST-1 reagent at 1/10 amount per medium was added to each medium and then the optical density (O.D. 450 nm/620 nm) of the medium was measured at the completion of the culture. A positive correlation was observed between the cell number and optical density within the range of 0.25 to 4×10⁴ cells/well.

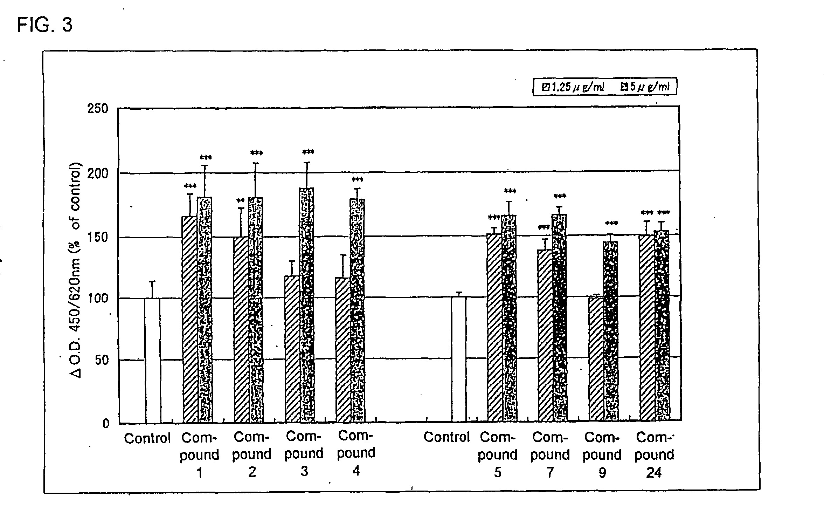

As a result, it is clarified that a compound having an activity of decreasing WNT-5A mRNA amount has an activity of promoting the growth of dermal papilla cells (Fig. 3). In this figure, each numerical value means an average of 6 wells (control group) or 3 wells (test groups). To compare the control group to the test groups, Student's t-test was employed:
*: P<0.05, **: P<0.01, ***: P<0.001.

The above-described Test Examples indicates that WNT-5A is expressed in human dermal papilla cells, the amount of WNT-5A mRNA in dermal papilla cells is decreased by the compound according to the present invention, and a compound having an activity of decreasing WNT-5A mRNA amount has an activity of promoting the growth of dermal papilla cells.

### Test Example 4

### Activity of compounds (Table 2) of decreasing WNT-5A mRNA amount (quantification of mRNA by QuantiGene method)

Human dermal papilla cells, which were purchased from Toyobo, were cultured in MEM (Invitrogen) containing 12% FBS.

The dermal papilla cells in passage 5 were sowed in a 96-well plate to give a density of 1×10⁴ cells/well and cultured overnight. Then, the medium was replaced by a medium to which no compound was added or a medium containing the compounds and the culture was continued for additional 24 hours. After the completion of the culture, WNT-5A or GAPDH mRNA amount was measured by using QuantiGene High Volume Kit (Bayer Medical) by the branched DNA (bDNA) signal amplification method *(Drug Metabolism and Disposition,* 28(5), 608-616(2000)). In accordance with the protocol attached to QuantiGene High Volume Kit, cells were lysed by using Lysis Mixture and the lysis solution was added to capture plate. Next, a set of probes specific to WNT-5A or GPDH was added and allowed to react at 53°C for 20 hours. After washing the plate by using 0.1×SSC containing 0.03% lauryl sulfate, an amplification probe comprising bDNA was added and allowed to react at 46°C for 1 hour. After washing the plate, a labeling probe labeled with alkaline phosphatase was added and allowed to react at 46°C for 1 hour. After washing the plate, a substrate Lumi-Phos Plus was added and allowed to react at 46°C for 30 minutes. Then, the luminescence was measured by using WALLAC 1420ARVOₛₓ.

The WNT-5A-specific probe set was designed based on the sequence of the coding region of human WNT-5A mRNA. As capture extenders (CEs), 10 probes (SEQ ID NOs:12 to 21) were used; as label extenders (LEs), 31 probes (SEQ ID NOs:22 to 52) were employed; and as blockers, 9 probes (SEQ ID NOs:53 to 60) were employed.

As GAPDH-specific probe set, use was made of bDNA probe set for human GAPDH (Xeno Tech LLC, B0960).

The amounts of WNT-5A and GAPDH mRNAs are expressed in relative values (%) to the control case of adding of no compound and the concentration of a compound at which the mRNA amount is halved (IC₅₀) is calculated as an index of WNT-5A mRNA decreasing activity of the corresponding compound.

Table 3 shows the effects of the compounds on the WNT-5A and GDPH mRNA amounts. Each numerical value in the table shows the average of 2 wells. These compounds decreased the WNT-5A mRNA amount in dermal papilla cells. The IC₅₀ value of the most active compound was 0.12µM. At the IC₅₀ concentration of each compound for WNT-5A mRNA suppression, no decrease was observed in the GAPDH mRNA amount measured at the same time.

**Table 3**

| | IC₅₀ (µM) | |
|---|---|---|
| Compound No. | WNT-5A | GAPDH |
| 1 | 0.20 | 28.25 |
| 2 | 0.39 | 26.41 |
| 3 | 3.16 | 57.95 |
| 4 | 3.46 | >30 |
| 5 | 3.47 | ' >30 |
| 7 | 4.07 | >120 |
| 18 | 0.63 | 6.58 |
| 24 | 4.74 | 21.07 |
| 35 | 0.46 | 4.29 |
| 38 | 6.42 | >120 |
| 52 | 0.12 | 13.47 |
| 53 | 0.12 | 45.45 |
| 54 | 2.37 | >30 |
| 55 | 0.74 | >30 |
| 58 | 0.50 | 79.95 |
| 65 | 8.28 | 109.16 |
| 68 | 10.69 | 50.63 |
| 73 | 9.37 | >120 |
| 76 | 8.56 | 55.94 |
| 77 | 8.13 | >120 |
| 79 | 0.90 | 38.16 |

### Test Example 5

### Dermal papilla cell growth promoting activity of compounds (Table 2):

Human dermal papilla cells, which were purchased from Toyobo, were cultured in MEM (Invitrogen) containing 12% FBS.

The dermal papilla cells in passage 5 were sowed in a 96-well plate for spheroid culture to give a density of 1.5×10⁴ cells/well and cultured overnight. Then, the medium was replaced by a medium to which no compound was added or a medium containing the compound and the culture was continued for additional 72 hours. After the completion of the culture, cells were counted by using a Cell Counting Kit (Wako Pure Chemicals). Five hours before the completion of the culture, WST-1 reagent at 1/10 amount per medium was added to each medium and then the optical density (O.D. 450 nm/620 nm) of the medium was measured at the completion of the culture. A positive correlation was observed between the cell number and optical density within the range of 0.25 to 4×10⁴ cells/well.

Table 4 shows the effects of the compounds having an activity of decreasing the WNT-5A mRNA amount on the growth of dermal papilla cells. In this table, each numerical value means an average of 6 wells (control group) or 3 wells (test groups). To compare the control group to the test groups, Student's t-test was employed:
*: P<0.05, **: P<0.01, ***: P<0.001.

Each of the compounds having an activity of decreasing the WNT-5A mRNA amount showed a remarkable activity of promoting the growth of dermal papilla cells at 16 µM. Compounds 1, 2, 18, 35, 52, 53 and 58, each showing a potent activity of decreasing the WNT-5A mRNA amount (i.e., IC₅₀ being lower than 1 µM), also showed a significant growth promoting activity at 1 µM.

**Table 4**

| Compound No. | ΔO.D. 450 nm/620 nm (% of control) | | |
|---|---|---|---|
| | 1 | 4 | 16 (µM) |
| 1 | 132.9 ± 11.6*** | 183.7 ± 12.1*** | 144.7 ± 13.0*** |
| 2 | 142.6 ± 10.1*** | 167.3 ± 8.5*** | 241.0 ± 25.3*** |
| 3 | 92.9 ± 1.6 | 92.9 ± 9.9 | 244.2 ± 33.4*** |
| 4 | 97.4 ± 24.4 | 148.2 ± 4.9*** | 180.2 ± 6.9*** |
| 5 | 110.2 ± 45.0 | 174.7 ± 29.2*** | 236.4 ± 26.4*** |
| 7 | 82.6 ± 3.9 | 169.7 ± 10.4*** | 194.8 ± 12.7*** |
| 18 | 115.6 ± 9.0 * | 147.1 ± 11.3*** | 210.9 ± 25.9*** |
| 24 | 108.2 ± 6.9 | 104.3 ± 10.5 | 119.7 ± 3.7** |
| 35 | 160.8 ± 3.5*** | 181.6 ± 3.7*** | 158.6 ± 18.3*** |
| 38 | 103.5 ± 2.6 | 89.6 ± 7.3 | 124.9 ± 5.1** |
| 52 | 124.5 ± 1.0*** | 143.2 ± 9.5*** | 197.0 ± 5.4*** |
| 53 | 126.6 ± 14.3** | 149.5 ± 12.8*** | 184.4 ± 3.6*** |
| 54 | 80.0 ± 2.4 | 97.1 ± 5.6 | 135.0 ± 0.9*** |
| 55 | 89.0 ± 1.8 | 126.9 ± 7.2*** | 175.6 ± 9.8*** |
| 58 | 123.3 ± 1.8*** | 150.4 ± 9.4*** | 217:4 ± 14.5*** |
| 65 | 92.1 ± 6.7 | 87.6 ± 8.7 | 141.0 ± 5.3*** |
| 73 | 91.7 ± 5.7 | 81.2 ± 7.8 | 205.6 ± 9.2*** |
| 76 | 80.0 ± 5.6 | 77.1 ± 7.0 | 120.7 ± 6.3 * |
| 77 | 81.6 ± 4.4 | 86.3 ± 5.0 | 180.8 ± 21.7*** |
| 79 | 87.5 ± 9.9 | 98.6 ± 3.6 | 120.0 ± 4.1** |

| | | | |
|---|---|---|---|
| *: p<0.05, | | | |
| **: p<0.01, | | | |
| ***: p<0.001 | | | |

### Test Example 6

### Decrease in WNT-5A mRNA in monkey skin organ culture

The dorsal skin of a Cynomolgus monkey *(Macaca fascicularis)* (male, 6 years old) was collected and divided into pieces (5 mm x 8 mm) under a stereoscopic microscope. Then each skin tissue piece was cultured in William's Medium E (Invitrogen) to which no compound was added or Compound 7 or Compound 3 was added and which contained 10 µg/ml insulin (Sigma) and 10 ng/ml hydrocortisone (Kurabo).

On the 11th day of the culturing, hair follicles were separated from each skin tissue piece and total RNAs were extracted by using TRIzol reagent (Invitrogen). Using 200 ng portions of the total RNAs, 0.4 µM portions of primers specific to WNT-5A or glyceraldehyde-3-phosphate dehydrogenase (GAPDH) [WNT-5A sense: AATGTCTTCC AAGTTCTTCC TAGTGGC (SEQ ID NO:8), WNT-5A antisense: GATGTCGGAA TTGATACTGG CA (SEQ ID NO:9), GAPDH sense: ACCACAGTCC ATGCCATCAC (SEQ ID NO:10) and GAPDH antisense: TCCACCACCC TGTTGCTGTA (SEQ ID NO: 11)] and SUPERSCRIPT One-Step RT-PCT with PLATINUM Taq (Invitrogen), RT-PCR was carried out in accordance with the protocol attached to SUPERSCRIPT One-Step RT-PCT with PLATINUM Taq. In brief, first strand synthesis was carried out at 50°C for 30 minutes in a reaction system of 25 µl in total volume in accordance with the protocol attached to SUPERSCRIPT One-Step RT-PCT with PLATINUM Taq. After heating to 94°C for 2 minutes, the WNT-5A or GPDH cDNA fragment was amplified in 40 or 30 cycles with each cycle consisting of 30 seconds at 94°C, 30 seconds at 55°C and 30 seconds at 72°C.

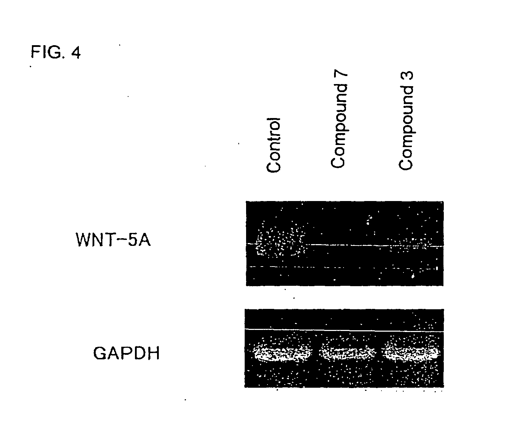

Each reaction solution was electrophoresed in a 1.5% agarose gel and stained with Cyber Green (Takara). Fig. 4 shows the results.

In the 40-cycle PCR using the WNT-5A-specific primers, the amplified WNT-5A cDNA fragment was remarkably decreased in the culture with the addition of Compound 7 or Compound 3, compared with the culture to which no compound was added.

In the 30-cycle PCR using the GAPDH-specific primers, on the other hand, no change was observed in the amount of amplified cDNA fragment regardless of the presence or absence of the compounds.

### Test Example 7

### Increase in proliferating cell nuclear antigen (PCNA)-positive cells in monkey skin organ culture:

The dorsal skin of a Cynomolgus monkey *(Macaca fascicularis)* (male, 6 years old) was collected and divided into pieces (5 mm × 8 mm) under a stereoscopic microscope. Then each skin tissue piece was cultured in William's Medium E (Invitrogen) to which no compound was added or Compound 7 or Compound 3 was added and which contained 10 µg/ml of insulin (Sigma) and 10 ng/ml of hydrocortisone (Kurabo).

On the 30th day of the culturing, the skin tissue pieces were each fixed by using 10% neutralized formalin at room temperature. A section embedded in paraffin was prepared. After adding 1% BSA-containing PBS, it allowed to stand at room temperature for 30 minutes and then boiled in a 0.1 M citrate buffer solution. Then it was reacted with anti-PCNA antibody (Dako Japan, diluted 200-fold) at 4°C overnight and then reacted with biotinylated antimouse IgG antibody. Further, it was reacted with ABC standard kit (vector) and subjected to color development using AEC substance (Sigma).

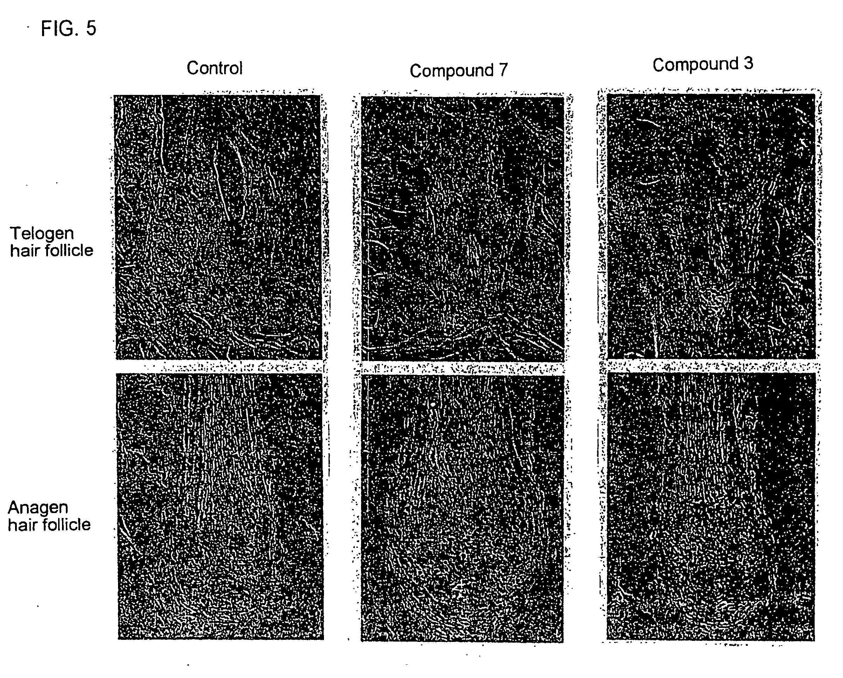

As a result, a positive reaction was observed in cells in hair follicles and the epidermal basal layer. In follicles, remarkable increases in the PCNA-positive cell count are observed in dermal papillae and dermal sheath both in the telogen and anagen hair follicles (Fig. 5).

### Test Example 8

### Enlargement in hair bulb diameter in monkey skin organ culture:

The dorsal skin of a Cynomolgus monkey *(Macaca fascicularis)* (male, 6 years old) was collected and divided into pieces (5 mm × 8 mm) under a stereoscopic microscope. Then each skin tissue piece was cultured in William's Medium E (Invitrogen) to which no compound was added or Compound 7 or Compound 3 was added and which contained 10 µg/ml of insulin (Sigma) and 10 ng/ml of hydrocortisone (Kurabo).

On the 30th day of the culturing, all follicles contained in each skin tissue piece were separated and the diameter of bulbs was measured under a stereoscopic microscope.

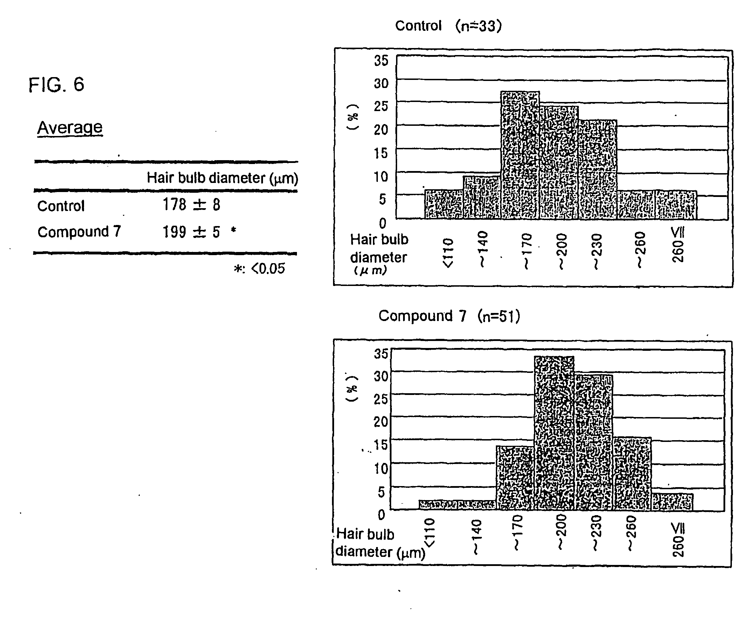

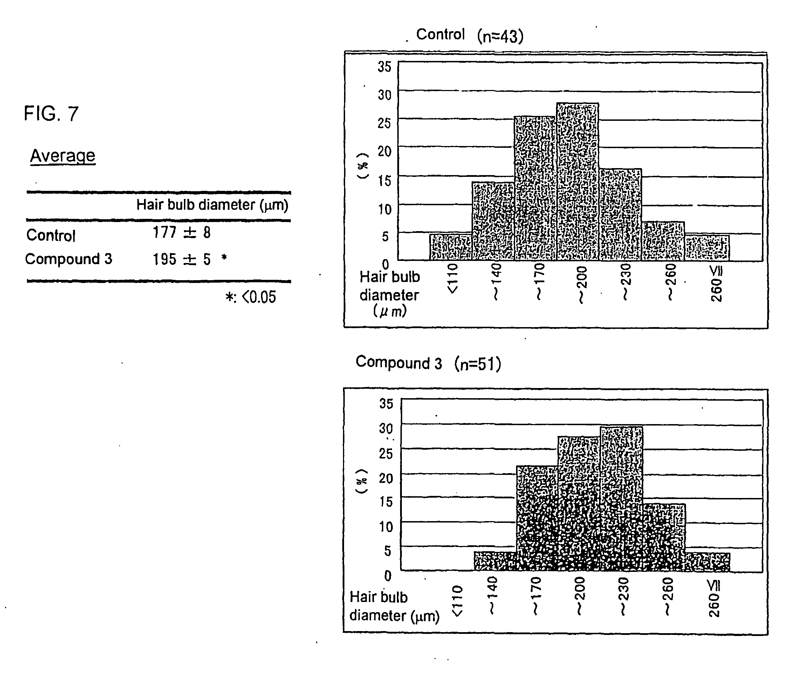

Figs. 6 and 7 show the results. In these figures, each numerical value means mean ± S.E. of bulb diameters of all follicles contained in each skin tissue piece. To compare the control group with the test groups, Student's t-test was employed:
*: P<0.05.

These figures also present histograms showing the bulb diameter distribution. All follicles contained in each skin tissue piece are divided into bulb diameter groups as indicated in the figures and follicle count in each group is expressed in the relative value (%) to the total follicle count separated from the tissue piece. Fig. 6 shows the effect achieved after culturing in the presence of Compound 7. After culturing in the presence of Compound 7 for 30 days, the average bulb diameter of the follicles contained in the skin piece was significantly higher than that of the control group to which no compound was added. As shown by the distribution, the control group to which no compound was added showed the largest follicle count in the group of diameter of 140 µm or longer but shorter than 170 µm, whereas the group to which Compound 7 was added showed the largest follicle count in the group of diameter of 170 µm or longer but shorter than 200 µm. The ratio of follicles of 170 µm or longer in bulb diameter in the group to which no compound was added was 57.6%, whereas the ratio in the group to which Compound 7 was elevated to 82.4%.

On the other hand, Fig. 7 shows the effect achieved after culturing in the presence of Compound 3. Similar to the case of Compound 7, after culturing in the presence of Compound 3 for 30 days, the average bulb diameter of the follicles contained in the skin piece was significantly higher than that of the control group to which no compound was added. As shown by the distribution, the control group to which no compound was added showed the largest follicle count in the group of diameter of 140 µm or longer but shorter than 170 µm, whereas the group to which Compound 3 was added showed the largest follicle count in the group of diameter of 200 µm or longer but shorter than 230 µm. The ratio of follicles of 170 µm or longer in bulb diameter in the group to which no compound was added was 55.8%, whereas the ratio in the group to which Compound 7 was elevated to 74.5%.

### Test Example 9

### Hair growth test in stumptailed macaque:

To the forehead of a stumptailed macaque *(Macaca arctoides)* (male, 14 years old) suffering from alopecia, 1 mL of a lotion containing 0.3% Compound 7 was applied once a day 5 times per week for 6 months. Before the initiation of the application, the alopecia site in the forehead was marked by tattoo. The skin part including the tattoo was photographed before the initiation of the application and after applying for 6 months.

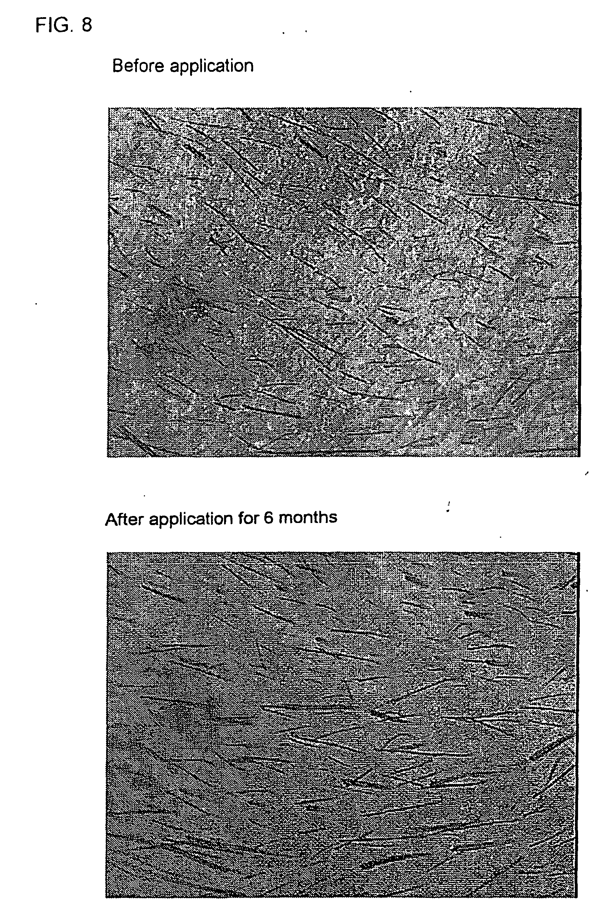

Fig. 8 provides enlarged photos of the skin before and after application of the lotion containing Compound 7 for 6 months. Before the application, many thin vellus hairs were observed. In the same site after applying for 6 months, in contrast, vellus hairs decreased and thick terminal hairs increased. That is, it is recognized that the alopecia in the forehead of the stumptailed macaque was clearly ameliorated by applying the lotion containing Compound 7 for 6 months.

The results of the above Test Examples indicate that WNT-5A is expressed in human dermal papilla cells, the amount of WNT-5A mRNA in human dermal papilla cells is decreased by the compound according to the present invention, and a compound having an activity of decreasing WNT-5A mRNA amount has an activity of promoting the growth of dermal papilla cells.

It is also indicated that the compound according to the present invention causes a decrease in the WNT-5A mRNA amount in monkey skin organ culture too, the compound causes a remarkable increase in the PCNA-staining positive proliferative cells both in the telogen and anagen hair follicles, and the compound enlarges the bulb size of follicles.

It is furthermore indicated that the compound according to the present invention has an effect of ameliorating alopecia of a stumptailed macaque which is a model animal of male pattern alopecia.

That is to say, a compound promoting the growth of dermal papilla cells would enhance the cell growing ability, which has been lowered in the dermal papillae in follicles in a part with alopecia, and thus contributes to the formation of well-developed dermal papilla tissues, thereby promoting hair growth.

### INDUSTRIAL APPLICABILITY

A WNT-5A function inhibitor, for example, a compound having an activity of decreasing WNT-5A mRNA amount or a pharmaceutically acceptable salt thereof has an activity of promoting the growth of dermal papilla cells and, therefore, is useful as a hair growth stimulant/hair growth tonic with a novel function mechanism.

The present invention provides a completely novel idea of using a compound inhibiting the function of WNT-5A as an agent ameliorating or preventing alopecia, which has never been known hitherto, and screening of such a compound inhibiting the function of WNT-5A is useful in developing a novel hair growth stimulant/hair growth tonic.

## Claims

1. A dermal papilla cell growth promoter containing a compound having an activity of inhibiting the function of WNT-5A.

2. The dermal papilla cell growth promoter according to claim 1 wherein the compound inhibiting the function of WNT-5A is a WNT-5A production inhibitor.

3. A compound represented by the following formula (I): 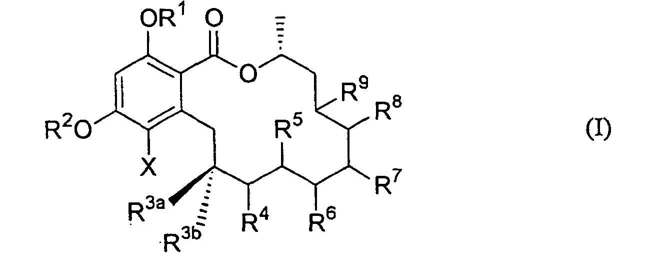 wherein R¹ and R² are the same or different and each represents a hydrogen atom, a C₁₋₆ alkyl group or a C₂₋₆ alkanoyl group;
X represents a hydrogen atom or a halogen atom;
R^{3a} and R^{3b} are the same or different and each represents a hydrogen atom or a hydroxyl group; and
R⁴, R⁵, R⁶, R⁷, R⁸ and R⁹ are the same or different and each represents a hydrogen atom, a hydroxyl group, a halogen atom or a C₂₋₆ alkanoyloxy group, or adjacent groups thereof are taken together to form a π bond or an ether bond, or R⁵ and R⁸ or R⁵ and R⁹ are taken together to form an ether bond.

4. A dermal papilla cell growth promoter containing a compound represented by the following formula (II): 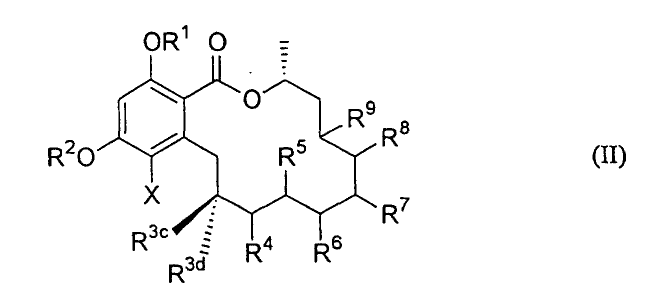 wherein R¹ and R² are the same or different and each represents a hydrogen atom, a C₁₋₆ alkyl group or a C₂₋₆ alkanoyl group; X represents a hydrogen atom or a halogen atom;
R^{3c} and R^{3d} are the same or different and each represents a hydrogen atom, a hydroxyl group or a C₁₋₆ alkoxy group, or R^{3c} and R^{3d} are taken together to form an oxo group, a hydroxyimino group or a C₁₋₆ alkoxyimino group; and
R⁴, R⁵, R⁶, R⁷, R⁸ and R⁹ are the same or different and each represents a hydrogen atom, a hydroxyl group, a halogen atom or a C₂₋₆ alkanoyloxy group, or adjacent groups thereof are taken together to form a π bond or an ether bond, or R⁵ and R⁸ or R⁵ and R⁹ are taken together to form an ether bond.

5. A dermal papilla cell growth promoter containing a compound represented by the following formula (IX): 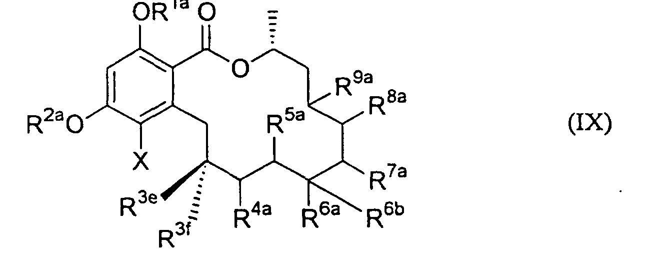 wherein R^{1a} and R^{2a} are the same or different and each represents a hydrogen atom, a C₁₋₆ alkyl group, a C₂₋₆ alkanoyl group,
a group represented by (CH₂)ₚCO-Y-R¹⁰, wherein Y represents an oxygen atom or a sulfur atom; R¹⁰ represents a hydrogen atom, a C₁₋₆ alkyl group or a substituted or unsubstituted aryl group; and p is 0 or 1,
a group represented by (CH₂)_{q}R¹¹, wherein R¹¹ represents a substituted or unsubstituted cycloalkyl group or a substituted or unsubstituted C₂₋₁₀ heterocycle; and q is 0 or 1, or
a group represented by COR¹², wherein R¹² represents a substituted or unsubstituted aryl group or a substituted or unsubstituted C₂₋₁₀ heterocycle;
X represents a hydrogen atom or a halogen atom;
R^{3e} and R^{3f} are the same or different and each represents a hydrogen atom, a hydroxyl group, a C₁₋₆ alkoxy group or a C₁₋₆ alkanoyloxy group, or R^{3e} and R^{3f} are taken together to form an oxo group, a hydroxyimino group or a C₁₋₆ alkoxyimino group; and
R^{4a}, R^{5a}, R^{6a}, R^{7a}, R^{8a} and R^{9a} are the same or different and each represents a hydrogen atom, a hydroxyl group, a halogen atom or
a group represented by Z-R¹³, wherein Z represents an oxygen atom or a sulfur atom; and R¹³ represents a C₁₋₆ alkyl group, a C₁₋₆ alkanoyl group or a substituted or unsubstituted aryl group, or
adjacent groups thereof are taken together to form a π bond or an ether bond, or
R^{5a} and R^{8a} or R^{5a} and R^{9a} are taken together to form an ether bond; and
R^{6b} represents a hydrogen atom, or is taken together with R^{3e} and R^{3f} to form an ether bond.

6. A hair growth stimulant or a hair growth tonic which comprises the dermal papilla cell growth promoter according to any one of claims 1, 2, 4 and 5 as an active ingredient.

7. A method of stimulating hair growth or promoting hair growth, which comprises administering a pharmaceutically effective amount of a compound having an activity of inhibiting the function of WNT-5A to human.

8. Use of a compound having an activity of inhibiting the function of WNT-5A in producing a hair growth stimulant or a hair growth tonic.

9. A method of screening a dermal papilla cell growth promoter, which comprises selecting a compound inhibiting the function of WNT-5A.

10. The method according to claim 9, which comprises the following steps (a) to (c):
(a) a step of culturing human WNT-5A-expressing cells in a medium to which a compound has been added;
(b) a step of lysing the human WNT-5A-expressing cells cultured in the step (a) to extract RNA, and determining the WNT-5A mRNA amount; and
(c) a step of comparing the WNT-5A mRNA amount determined in the step (b).


SEQUENCE LISTING
<110>TAISHO PHARMACEUTICAL CO., LTD.
<120>HAIR GROWTH PREPARATION
<130>P600
<160>61
<150>JP 2002-115529
<151>2002-04-17
<210>1
<211>365
<212>PROTEIN
<213>HOMO SAPIENS
<400>1
Met Ala Gly Ser Ala Met Ser Ser Lys Phe Phe Leu Val Ala Leu
                5                   10                  15
Ala Ile Phe Phe Ser Phe Ala Gln Val Val Ile Glu Ala Asn Ser
                20                  25                  30
Trp Trp Ser Leu Gly Met Asn Asn Pro Val Gln Met Ser Glu Val
                35                  40                  45
Tyr Ile Ile Gly Ala Gln Pro Leu Cys Ser Gln Leu Ala Gly Leu
                50                  55                  60
Ser Gln Gly Gln Lys Lys Leu Cys His Leu Tyr Gln Asp His Met
                65                  70                  75
Gln Tyr Ile Gly Glu Gly Ala Lys Thr Gly Ile Lys Glu Cys Gln
                80                  85                  90
Tyr Gln Phe Arg His Arg Arg Trp Asn Cys Ser Thr Val Asp Asn
                95                  100                 105
Thr Ser Val Phe Gly Arg Val Met Gln Ile Gly Ser Arg Glu Thr
                110                 115                 120

Ala Phe Thr Tyr Ala Val Ser Ala Ala Gly Val Val Asn Ala Met
                125                 130                 135
Ser Arg Ala Cys Arg Glu Gly Glu Leu Ser Thr Cys Gly Cys Ser
                140                 145                 150
Arg Ala Ala Arg Pro Lys Asp Leu Pro Arg Asp Trp Leu Trp Gly
                155                 160                 165
Gly Cys Gly Asp Asn Ile Asp Tyr Gly Tyr Arg Phe Ala Lys Glu
                170                 175                 180
Phe Val Asp Ala Arg Glu Arg Glu Arg Ile His Ala Lys Gly Ser
                185                 190                 195
Tyr Glu Ser Ala Arg Ile Leu Met Asn Leu His Asn Asn Glu Ala
                200                 205                 210
Gly Arg Arg Thr Val Tyr Asn Leu Ala Asp Val Ala Cys Lys Cys
                215                 220                 225
His Gly Val Ser Gly Ser Cys Ser Leu Lys Thr Cys Trp Leu Gln
                230                 235                 240
Leu Ala Asp Phe Arg Lys Val Gly Asp Ala Leu Lys Glu Lys Tyr
                245                 250                 255
Asp Ser Ala Ala Ala Met Arg Leu Asn Ser Arg Gly Lys Leu Val
                260                 265                 270
Gln Val Asn Ser Arg Phe Asn Ser Pro Thr Thr Gln Asp Leu Val
                275                 280                 285
Tyr Ile Asp Pro Ser Pro Asp Tyr Cys Val Arg Asn Glu Ser Thr
                290                 295                 300
Gly Ser Leu Gly Thr Gln Gly Arg Leu Cys Asn Lys Thr Ser Glu
                305                 310                 315
Gly Met Asp Gly Cys Glu Leu Met Cys Cys Gly Arg Gly Tyr Asp
                320                 325                 330

Gln Phe Lys Thr Val Gln Thr Glu Arg Cys His Cys Lys Phe His
                335                 340                 345
Trp Cys Cys Tyr Val Lys Cys Lys Lys Cys Thr Glu Ile Val Asp
                350                 355                 360
Gln Phe Val Cys Lys
                365
<210>2
<211>4428
<212>DNA
<213>HOMO SAPIENS
<400>2
   ttaaggaaat ccgggctgct cttccccatc tggaagtggc tttccccaca tcggctcgta 60
   aactgattat gaaacatacg atgttaattc ggagctgcat ttcccagctg ggcactctcg 120
   cgcgctggtc cccggggcct cgccccccac cccctgccct tccctcccgc gtcctgcccc 180
   catcctccac cccccgcgct ggccaccccg cctccttggc agcctctggc ggcagcgcgc 240
   tccactcgcc tcccgtgctc ctctcgccca tggaattaat tctggctcca cttgttgctc 300
   ggcccaggtt ggggagagga cggagggtgg ccgcagcggg ttcctgagtg aattacccag 360
   gagggactga gcacagcacc aactagagag gggtcagggg gtgcgggact cgagcgagca 420
   ggaaggaggc agcgcctggc accagggctt tgactcaaca gaattgagac acgtttgtaa 480
   tcgctggcgt gccccgcgca caggatccca gcgaaaatca gatttcctgg tgaggttgcg 540
   tgggtggatt aatttggaaa aagaaactgc ctatatcttg ccatcaaaaa actcacggag 600
   gagaagcgca gtcaatcaac agtaaactta agagaccccc gatgctcccc tggtttaact 660
   tgtatgcttg aaaattatct gagagggaat aaacatcttt tccttcttcc ctctccagaa 720
   gtccattgga atattaagcc caggagttgc tttggggatg gctggaagtg caatgtcttc 780
   caagttcttc ctagtggctt tggccatatt tttctccttc gcccaggttg taattgaagc 840
   caattcttgg tggtcgctag gtatgaataa ccctgttcag atgtcagaag tatatattat 900
   aggagcacag cctctctgca gccaactggc aggactttct caaggacaga agaaactgtg 960
   ccacttgtat caggaccaca tgcagtacat cggagaaggc gcgaagacag gcatcaaaga 1020
   atgccagtat caattccgac atcgacggtg gaactgcagc actgtggata acacctctgt 1080
   ttttggcagg gtgatgcaga taggcagccg cgagacggcc ttcacatacg ccgtgagcgc 1140
   agcaggggtg gtgaacgcca tgagccgggc gtgccgcgag ggcgagctgt ccacctgcgg 1200
   ctgcagccgc gccgcgcgcc ccaaggacct gccgcgggac tggctctggg gcggctgcgg 1260
   cgacaacatc gactatggct accgctttgc caaggagttc gtggacgccc gcgagcggga 1320
   gcgcatccac gccaagggct cctacgagag tgctcgcatc ctcatgaacc tgcacaacaa 1380
   cgaggccggc cgcaggacgg tgtacaacct ggctgatgtg gcctgcaagt gccatggggt 1440
   gtccggctca tgtagcctga agacatgctg gctgcagctg gcagacttcc gcaaggtggg 1500
   tgatgccctg aaggagaagt acgacagcgc ggcggccatg cggctcaaca gccggggcaa 1560
   gttggtacag gtcaacagcc gcttcaactc gcccaccaca caagacctgg tctacatcga 1620
   ccccagccct gactactgcg tgcgcaatga gagcaccggc tcgctgggca cgcagggccg 1680
   cctgtgcaac aagacgtcgg agggcatgga tggctgcgag ctcatgtgct gcggccgtgg 1740
   gtacgaccag ttcaagaccg tgcagacgga gcgctgccac tgcaagttcc actggtgctg 1800
   ctacgtcaag tgcaagaagt gcacggagat cgtggaccag tttgtgtgca agtagtgggt 1860
   gccacccagc actcagcccc gctcccagga cccgcttatt tatagaaagt acagtgattc 1920
   tggtttttgg tttttagaaa tattttttat ttttccccaa gaattgcaac cggaaccatt 1980
   ttttttcctg ttaccatcta agaactctgt ggtttattat taatattata attattattt 2040
   ggcaataatg ggggtgggaa ccacgaaaaa tatttatttt gtggatcttt gaaaaggtaa 2100
   tacaagactt cttttggata gtatagaatg aagggggaaa taacacatac cctaacttag 2160
   ctgtgtggga catggtacac atccagaagg taaagaaata cattttcttt ttctcaaata 2220
   tgccatcata tgggatgggt aggttccagt tgaaagaggg tggtagaaat ctattcacaa 2280
   ttcagcttct atgaccaaaa tgagttgtaa attctctggt gcaagataaa aggtcttggg 2340
   aaaacaaaac aaaacaaaac aaacctccct tccccagcag ggctgctagc ttgctttctg 2400
   cattttcaaa atgataattt acaatggaag gacaagaatg tcatattctc aaggaaaaaa 2460
   ggtatatcac atgtctcatt ctcctcaaat attccatttg cagacagacc gtcatattct 2520
   aatagctcat gaaatttggg cagcagggag gaaagtcccc agaaattaaa aaatttaaaa 2580
   ctcttatgtc aagatgttga tttgaagctg ttataagaat tgggattcca gatttgtaaa 2640
   aagaccccca atgattctgg acactagatt ttttgtttgg ggaggttggc ttgaacataa 2700
   atgaaatatc ctgtattttc ttagggatac ttggttagta aattataata gtagaaataa 2760
   tacatgaatc ccattcacag gtttctcagc ccaagcaaca aggtaattgc gtgccattca 2820
   gcactgcacc agagcagaca acctatttga ggaaaaacag tgaaatccac cttcctcttc 2880
   acactgagcc ctctctgatt cctccgtgtt gtgatgtgat gctggccacg tttccaaacg 2940
   gcagctccac tgggtcccct ttggttgtag gacaggaaat gaaacattag gagctctgct 3000
   tggaaaacag ttcactactt agggattttt gtttcctaaa acttttattt tgaggagcag 3060
   tagttttcta tgttttaatg acagaacttg gctaatggaa ttcacagagg tgttgcagcg 3120
   tatcactgtt atgatcctgt gtttagatta tccactcatg cttctcctat tgtactgcag 3180
   gtgtacctta aaactgttcc cagtgtactt gaacagttgc atttataagg ggggaaatgt 3240
   ggtttaatgg tgcctgatat ctcaaagtct tttgtacata acatatatat atatatacat 3300
   atatataaat ataaatataa atatatctca ttgcagccag tgatttagat ttacagctta 3360
   ctctggggtt atctctctgt ctagagcatt gttgtccttc actgcagtcc agttgggatt 3420
   attccaaaag ttttttgagt cttgagcttg ggctgtggcc ccgctgtgat cataccctga 3480
   gcacgacgaa gcaacctcgt ttctgaggaa gaagcttgag ttctgactca ctgaaatgcg 3540
   tgttgggttg aagatatctt tttttctttt ctgcctcacc cctttgtctc caacctccat 3600
   ttctgttcac tttgtggaga gggcattact tgttcgttat agacatggac gttaagagat 3660
   attcaaaact cagaagcatc agcaatgttt ctcttttctt agttcattct gcagaatgga 3720
   aacccatgcc tattagaaat gacagtactt attaattgag tccctaagga atattcagcc 3780
   cactacatag atagcttttt tttttttttt ttttttttaa taaggacacc tctttccaaa 3840
   caggccatca aatatgttct tatctcagac ttacgttgtt ttaaaagttt ggaaagatac 3900
   acatcttttc ataccccccc ttaggaggtt gggctttcat atcacctcag ccaactgtgg 3960
   ctcttaattt attgcataat gatatccaca tcagccaact gtggctcttt aatttattgc 4020
   ataatgatat tcacatcccc tcagttgcag tgaattgtga gcaaaagatc ttgaaagcaa 4080
   aaagcactaa ttagtttaaa atgtcacttt tttggttttt attatacaaa aaccatgaag 4140
   tacttttttt atttgctaaa tcagattgtt cctttttagt gactcatgtt tatgaagaga 4200
   gttgagttta acaatcctag cttttaaaag aaactattta atgtaaaata ttctacatgt 4260
   cattcagata ttatgtatat cttctagcct ttattctgta cttttaatgt acatatttct 4320
   gtcttgcgtg atttgtatat ttcactggtt taaaaaacaa acatcgaaag gcttattcca 4380
   aatggaagat agaatataaa ataaaacgtt acttgtaaaa aaaaaaaa 4428

<210>3

<211>2460

<212>DNA

<213>HOMO SAPIENS

<400>3

cgtggcacgc gcggaagatt ctcagtgtcc ttacagagtc atcttccctg agccccggaa 60

gtgttggaaa acatttagcc ccttctttgg gaaactcagt ttctgatcag aatttttgtt 120

ttaccctggg gttgacagtc tcgccagagg tctcatttca tactgtcttt tcggatctga 180

tcctcttggt aaacaggcgg ggatgtttta ccctacagag ccgatgtatg tgtgagttcg 240

ctgtgagttc tttgagtgtc tcaaacttgt ggggcctttt ctcggttgca ctgggattga 300

agagggaaga ggcccaaggt gtttccgggc aagcggcggg gttaagtgga gatgcgactc 360

gtgaggctct cctttccgat ccccctttgg gacaccctct gcctacctct accctggagc 420

cagggagacc caagtcttgg tgaccggatg ggcccgctct cagttggcct gggctctggg 480

aactggtgga ctctccctgg gggcttcggg ctgggagtgg gttcggtttg tgtggcttcg 540

gctctaacaa agagatccgc tgtaatccgc cgaatctgtt atcaatttct ctgctgcttg 600

agccccgccc cacgcgcccc gcccgccgcg aagcttggaa agtgcacgcg gccagcacca 660

atctgggccg ctgactcgga aacatgtcgc agcgtgtgtg tctatggacg cgtgtgagtg 720

tgtaaatgtg cacgagtgtg aatgtgtatg atgtgtgtgc acgcggcatc ggctgccctt 780

ggggagagtt gactttgcag cctgggctgc gcgagaagca gactttgcag cccactccct 840

cccctggagg aaatttgaca cttagggcgg gggtggggag atagccggag ccttctctct 900

cctagctggg gaaaccccag atttccattc tccaggatgc gccccccagc tttgcagcgt 960

cttggggaca actggcctgg tgttggagcc ctgcttagca ggcgctgggg accacataag 1020

cattcctctt tggagaagcc ccgaagcgtc caggccaaag ggggcggttc acggaagaaa 1080

aaccttgcac gcccttgagc gcatagcttt accagggctg cctaggtccc gcctcttgcc 1140

cttttacggc acaggttcca agccaggctc ttcccaccgc cttaaagagg ctcacctttc 1200

ttttcttttc tgtggaaggg gctccttcag ggctatgggg cgatgcagtg cggcagggtt 1260

agacttacgt gtaaggggat ttttaaaacc cgctcctccc acccgcaccc gccacctact 1320

cgctccgccg ccgcctacag gtggagaagt caccagtggg gaggaacggc agcggaagct 1380

tccaaggcca actcctaccc ctgaaattct tcaggaaggg aaccttcgcc gctggggggc 1440

tctttggcct ggaatcgatg cgcccagctg cggctcggaa gccagcgcct ctggccccgt 1500

ctggactcat ctgcaagggc tctggcctcg ccccgcaccc ccacctttcg ggactgaccg 1560

aaccaagtct gagttgggct ggagaggcta gactggaggc agggtggcag agttccaacg 1620

acaggctcgc agtgccgcga atggcaaagt gggccacaac cccagatcag gacccagaga 1680

aactggagtc tctctctggg cctcccatct cctccctccc tggcaactac caggttgtgg 1740

ggtgggaggg agagtgaaaa atcaagaatt tgggagaaag ctgtggggag ggcagggaag 1800

ggatccttct ccccggggaa gcgagaccca gactcccttc tttcctctag ggttccatcc 1860

cttctctcag tccgtggaag aggccacagg cgacgcgggc gagggtggca ctcttttcca 1920

gtttccttgg ttgggagacc cgacctctct ctccattatc ccctagggcc cccatctcct 1980

tctcccctcc ctagtctggc tgaagaacgt ccttaaggaa atccgggctg ctcttcccca 2040

tctggaagtg gctttcccca catcggctcg taaactgatt atgaaacata cgatgttaat 2100

tcggagctgc atttcccagc tgggcactct cgcgcgctgg tccccggggc ctcgcccccc 2160

accccctgcc cttccctccc gcgtcctgcc cccatcctcc accccccgcg ctggccaccc 2220

cgcctccttg gcagcctctg gcggcagcgc gctccactcg cctcccgtgc tcctctcgcc 2280

catggaatta attctggctc cacttgttgc tcggcccagg ttggtgagag gacggagggt 2340

gcccacagcg ggttcctgag tgaattaccc aggagggact gagcacagca ccaactagag 2400

gggggccagg gggtgcggga ctcgagcgag caggaaggag gcagcgcctg gcaccagggc 2460

<210>4

<211>585

<212>PROTEIN

<213>HOMO SAPIENS

<400>4

Met Ala Arg Pro Asp Pro Ser Ala Pro Pro Ser Leu Leu Leu Leu

                5                   10                  15

Leu Leu Ala Gln Leu Val Gly Arg Ala Ala Ala Ala Ser Lys Ala

                20                  25                  30
Pro Val Cys Gln Glu Ile Thr Val Pro Met Cys Arg Gly Ile Gly
                35                  40                  45
Tyr Asn Leu Thr His Met Pro Asn Gln Phe Asn His Asp Thr Gln
                50                  55                  60
Asp Glu Ala Gly Leu Glu Val His Gln Phe Trp Pro Leu Val Glu
                65                  70                  75
Ile Gln Cys Ser Pro Asp Leu Arg Phe Phe Leu Cys Thr Met Tyr
                80                  85                  90
Thr Pro Ile Cys Leu Pro Asp Tyr His Lys Pro Leu Pro Pro Cys
                95                  100                 105
Arg Ser Val Cys Glu Arg Ala Lys Ala Gly Cys Ser Pro Leu Met
                110                 115                 120
Arg Gln Tyr Gly Phe Ala Trp Pro Glu Arg Met Ser Cys Asp Arg
                125                 130                 135
Leu Pro Val Leu Gly Arg Asp Ala Glu Val Leu Cys Met Asp Tyr
                140                 145                 150
Asn Arg Ser Glu Ala Thr Thr Ala Pro Pro Arg Pro Phe Pro Ala
                155                 160                 165
Lys Pro Thr Leu Pro Gly Pro Pro Gly Ala Pro Ala Ser Gly Gly
                170                 175                 180
Glu Cys Pro Ala Gly Gly Pro Phe Val Cys Lys Cys Arg Glu Pro
                185                 190                 195
Phe Val Pro Ile Leu Lys Glu Ser His Pro Leu Tyr Asn Lys Val
                200                 205                 210
Arg Thr Gly Gln Val Pro Asn Cys Ala Val Pro Cys Tyr Gln Pro
                215                 220                 225
Ser Phe Ser Ala Asp Glu Arg Thr Phe Ala Thr Phe Trp Ile Gly

                230                 235                 240
Leu Trp Ser Val Leu Cys Phe Ile Ser Thr Ser Thr Thr Val Ala
                245                 250                 255
Thr Phe Leu Ile Asp Met Asp Thr Phe Arg Tyr Pro Glu Arg Pro
                260                 265                 270
Ile Ile Phe Leu Ser Ala Cys Tyr Leu Cys Val Ser Leu Gly Phe
                275                 280                 285
Leu Val Arg Leu Val Val Gly His Ala Ser Val Ala Cys Ser Arg
                290                 295                 300
Glu His Asn His Ile His Tyr Glu Thr Thr Gly Pro Ala Leu Cys
                305                 310                 315
Thr Ile Val Phe Leu Leu Val Tyr Phe Phe Gly Met Ala Ser Ser
                320                 325                 330
Ile Trp Trp Val Ile Leu Ser Leu Thr Trp Phe Leu Ala Ala Ala
                335                 340                 345
Met Lys Trp Gly Asn Glu Ala Ile Ala Gly Tyr Gly Gln Tyr Phe
                350                 355                 360
His Leu Ala Ala Trp Leu Ile Pro Ser Val Lys Ser Ile Thr Ala
                365                 370                 375
Leu Ala Leu Ser Ser Val Asp Gly Asp Pro Val Ala Gly Ile Cys
                380                 385                 390
Tyr Val Gly Asn Gln Asn Leu Asn Ser Leu Arg Arg Phe Val Leu
                395                 400                 405
Gly Pro Leu Val Leu Tyr Leu Leu Val Gly Thr Leu Phe Leu Leu
                410                 415                 420
Ala Gly Phe Val Ser Leu Phe Arg Ile Arg Ser Val Ile Lys Gln
                425                 430                 435
Gly Gly Thr Lys Thr Asp Lys Leu Glu Lys Leu Met Ile Arg Ile

440                 445                 450
Gly Ile Phe Thr Leu Leu Tyr Thr Val Pro Ala Ser Ile Val Val
                455                 460                 465
Ala Cys Tyr Leu Tyr Glu Gln His Tyr Arg Glu Ser Trp Glu Ala
                470                 475                 480
Ala Leu Thr Cys Ala Cys Pro Gly His Asp Thr Gly Gln Pro Arg
                485                 490                 495
Ala Lys Pro Glu Tyr Trp Val Leu Met Leu Lys Tyr Phe Met Cys
                500                 505                 510
Leu Val Val Gly Ile Thr Ser Gly Val Trp Ile Trp Ser Gly Lys
                515                 520                 525
Thr Val Glu Ser Trp Arg Arg Phe Thr Ser Arg Cys Cys Cys Arg
                530                 535                 540
Pro Arg Arg Gly His Lys Ser Gly Gly Ala Met Ala Ala Gly Asp
                545                 550                 555
Tyr Pro Glu Ala Ser Ala Ala Leu Thr Gly Arg Thr Gly Pro Pro
                560                 565                 570
Gly Pro Ala Ala Thr Tyr His Lys Gln Val Ser Leu Ser His Val
                575                 580                 585
<210>5
<211>2334
<212>DNA
<213>HOMO SAPIENS
<400>5
acccagggac ggaggaccca ggctggcttg gggactgtct gctcttctcg gcgggagccg   60
tggagagtcc tttccctgga atccgagccc taaccgtctc tccccagccc tatccggcga  120
ggagcggagc gctgccagcg gaggcagcgc cttcccgaag cagtttatct ttggacggtt  180
ttctttaaag gaaaaacgaa ccaacaggtt gccagccccg gcgccacaca cgagacgccg  240

gagggagaag ccccggcccg gattcctctg cctgtgtgcg tccctcgcgg gctgctggag 300
gcgaggggag ggagggggcg atggctcggc ctgacccatc cgcgccgccc tcgctgttgc 360
tgctgctcct ggcgcagctg gtgggccggg cggccgccgc gtccaaggcc ccggtgtgcc 420
aggaaatcac ggtgcccatg tgccgcggca tcggctacaa cctgacgcac atgcccaacc 480
agttcaacca cgacacgcag gacgaggcgg gcctggaggt gcaccagttc tggccgctgg 540
tggagatcca atgctcgccg gacctgcgct tcttcctatg cactatgtac acgcccatct 600
gtctgcccga ctaccacaag ccgctgccgc cctgccgctc ggtgtgcgag cgcgccaagg 660
ccggctgctc gccgctgatg cgccagtacg gcttcgcctg gcccgagcgc atgagctgcg 720
accgcctccc ggtgctgggc cgcgacgccg aggtcctctg catggattac aaccgcagcg 780
aggccaccac ggcgcccccc aggcctttcc cagccaagcc cacccttcca ggcccgccag 840
gggcgccggc ctcggggggc gaatgccccg ctgggggccc gttcgtgtgc aagtgtcgcg 900
agcccttcgt gcccattctg aaggagtcac acccgctcta caacaaggtg cggacgggcc 960
aggtgcccaa ctgcgcggta ccctgctacc agccgtcctt cagtgccgac gagcgcacgt 1020
tcgccacctt ctggataggc ctgtggtcgg tgctgtgctt catctccacg tccaccacag 1080
tggccacctt cctcatcgac atggacacgt tccgctatcc tgagcgcccc atcatcttcc 1140
tgtcagcctg ctacctgtgc gtgtcgctgg gcttcctggt gcgtctggtc gtgggccatg 1200
ccagcgtggc ctgcagccgc gagcacaacc acatccacta cgagaccacg ggccctgcac 1260
tgtgcaccat cgtcttcctc ctggtctact tcttcggcat ggccagctcc atctggtggg 1320
tcatcctgtc gctcacctgg ttcctggccg ccgcgatgaa gtggggcaac gaggccatcg 1380
cgggctacgg ccagtacttc cacctggctg cgtggctcat ccccagcgtc aagtccatca 1440
cggcactggc gctgagctcc gtggacgggg acccagtggc cggcatctgc tacgtgggca 1500
accagaacct gaactcgctg cggcgcttcg tgctgggccc gctggtgctc tacctgctgg 1560
tgggcacgct cttcctgctg gcgggcttcg tgtcgctctt ccgcatccgc agcgtcatca 1620
agcagggcgg caccaagacg gacaagctgg agaagctcat gatccgcatc ggcatcttca 1680
cgctgctcta cacggtcccc gccagcattg tggtggcctg ctacctgtac gagcagcact 1740
accgcgagag ctgggaggcg gcgctcacct gcgcctgccc gggccacgac accggccagc 1800
cgcgcgccaa gcccgagtac tgggtgctca tgctcaagta cttcatgtgc ctggtggtgg 1860
gcatcacgtc gggcgtctgg atctggtcgg gcaagacggt ggagtcgtgg cggcgtttca 1920

ccagccgctg ctgctgccgc ccgcggcgcg gccacaagag cgggggcgcc atggccgcag 1980
gggactaccc cgaggcgagc gccgcgctca caggcaggac cgggccgccg ggccccgccg 2040
ccacctacca caagcaggtg tccctgtcgc acgtgtagga ggctgccgcc gagggactcg 2100
gccggagagc tgaggggagg ggggcgtttt gtttggtagt tttgccaagg tcacttccgt 2160
ttaccttcat ggtgctgttg ccccctcccg cggcgacttg gagagaggga agaggggcgt 2220
tttcgaggaa gaacctgtcc caggtcttct ccaaggggcc cagctcacgt gtattctatt 2280
ttgcgtttct tacctgcctt ctttatggga accctctttt taatttatat gtat 2334
<210>6
<211>570
<212>PROTEIN
<213>RATTUS SP.
<400>6
Met Arg Ala Arg Ser Ala Leu Pro Arg Ser Ala Leu Pro Arg Leu
                5                   10                  15
Leu Leu Pro Leu Leu Leu Leu Pro Ala Ala Gly Pro Ala Gln Phe
                20                  25                  30
His Gly Glu Lys Gly Ile Ser Ile Pro Asp His Gly Phe Cys Gln
                35                  40                  45
Pro Ile Ser Ile Pro Leu Cys Thr Asp Ile Ala Tyr Asn Gln Thr
                50                  55                  60
Ile Met Pro Asn Leu Leu Gly His Thr Asn Gln Glu Asp Ala Gly
                65                  70                  75
Leu Glu Val His Gln Phe Tyr Pro Leu Val Lys Val Gln Cys Ser
                80                  85                  90
Pro Glu Leu Arg Phe Phe Leu Cys Ser Met Tyr Ala Pro Val Cys
                95                  100                 105
Thr Val Leu Glu Gln Ala Ile Pro Pro Cys Arg Ser Ile Cys Glu
                110                 115                 120

Arg Ala Arg Gln Gly Cys Glu Ala Leu Met Asn Lys Phe Gly Phe
                125                 130                 135
Gln Trp Pro Glu Arg Leu Arg Cys Glu His Phe Pro Arg His Gly
                140                 145                 150
Ala Glu Gln Ile Cys Val Gly Gln Asn His Ser Glu Asp Gly Thr
                155                 160                 165
Pro Ala Leu Leu Thr Thr Ala Pro Pro Ser Gly Leu Gln Pro Gly
                170                 175                 180
Ala Gly Gly Thr Pro Gly Gly Pro Gly Gly Gly Gly Ala Pro Pro
                185                 190                 195
Arg Tyr Ala Thr Leu Glu His Pro Phe His Cys Pro Arg Val Leu
                200                 205                 210
Lys Val Pro Ser Tyr Leu Ser Tyr Lys Phe Leu Gly Glu Arg Asp
                215                 220                 225
Cys Ala Ala Pro Cys Glu Pro Ala Arg Pro Asp Gly Ser Met Phe
                230                 235                 240
Phe Ser His His His Thr Arg Phe Ala Arg Leu Trp Ile Leu Thr
                245                 250                 255
Trp Ser Val Leu Cys Cys Ala Ser Thr Phe Phe Thr Val Thr Thr
                260                 265                 270
Ser Leu Val Ala Met Gln Arg Phe Arg Tyr Pro Glu Arg Pro Ile
                275                 280                 285
Ile Phe Leu Ser Gly Cys Tyr Thr Met Val Ser Val Ala Tyr Ile
                290                 295                 300
Ala Gly Phe Val Leu Gln Glu Arg Val Val Cys Asn Glu Arg Phe
                305                 310                 315
Ser Glu Asp Gly Tyr Arg Thr Val Gly Gln Gly Thr Lys Lys Glu
                320                 325                 330

Gly Cys Thr Ile Leu Phe Met Met Leu Tyr Phe Phe Ser Met Ala
                335                 340                 345
Ser Ser Ile Trp Trp Val Ile Leu Ser Leu Thr Trp Phe Leu Ala
                350                 355                 360
Ala Gly Met Lys Trp Gly His Ala Ala Ile Glu Ala Asn Ser Gln
                365                 370                 375
Tyr Phe His Leu Ala Ala Trp Ala Val Pro Ala Val Lys Thr Ile
                380                 385                 390
Thr Ile Leu Ala Met Gly Gln Ile Asp Gly Asp Leu Leu Ser Gly
                395                 400                 405
Val Cys Phe Val Gly Leu Asn Arg Leu Asp Pro Leu Arg Gly Phe
                410                 415                 420
Val Leu Ala Pro Leu Phe Val Tyr Leu Phe Ile Gly Thr Ser Phe
                425                 430                 435
Leu Leu Ala Gly Phe Val Ser Leu Phe Arg Ile Arg Thr Ile Met
                440                 445                 450
Lys His Asp Gly Thr Lys Thr Glu Pro Leu Glu Arg Leu Met Val
                455                 460                 465
Arg Ile Gly Val Phe Ser Val Leu Tyr Thr Val Pro Ala Thr Ile
                470                 475                 480
Val Ile Ala Cys Tyr Phe Tyr Glu Gln Ala Phe Arg Glu His Trp
                485                 490                 495
Glu Arg Ser Trp Val Ser Gln His Cys Lys Ser Leu Ala Ile Pro
                500                 505                 510
Cys Pro Ala His Tyr Thr Pro Arg Thr Ser Pro Asp Phe Thr Val
                515                 520                 525
Tyr Met Ile Lys Tyr Leu Met Thr Leu Ile Val Gly Ile Thr Ser
                530                 535                 540

Gly Phe Trp Ile Trp Ser Gly Lys Thr Leu His Ser Trp Arg Lys
                545                 550                 555
Phe Tyr Thr Arg Leu Thr Asn Ser Arg His Gly Glu Thr Thr Val
                560                 565                 570
<210>7
<211>1912
<212>DNA
<213>RATTUS SP.
<400>7
aggggaaggc gcgcggtctc tgggttgggg gcgggggctg gggggcgccc aggagccgag 60
tggggggcgg cggccagcat gcgggcccgc agcgccctgc cccgcagcgc cctgccccgc 120
ctgctgctgc cactgctgct gctgccggct gccgggccgg ctcagttcca cggggagaag 180
ggcatctcca tcccggacca cggcttctgc cagcccatct ccatcccgct gtgcacggac 240
atcgcctaca accagaccat catgcccaac cttcttgggc acacgaacca agaggacgcg 300
ggcctggagg tgcatcaatt ctacccgctg gtgaaggtgc agtgctcgcc cgagctgcgc 360
ttcttcctgt gctccatgta cgctccggtg tgcacggtgc tggagcaggc catcccgccg 420
tgccgctcca tctgcgaacg cgcgcgccaa ggctgcgagg cgctcatgaa caagttcggc 480
ttccagtggc ccgagcgcct ccgctgcgag catttcccgc gtcacggcgc ggagcagatc 540
tgcgtgggcc agaaccactc cgaggacgga actcctgcgc tactcaccac cgcgccaccg 600
tctgggctgc agcctggcgc tggtggcacc ccgggcggcc ctggcggtgg tggcgcgccc 660
ccgcgctacg ccactctgga gcaccctttc cactgtcccc gcgtcctcaa ggtgccgtcc 720
tatctcagct ataagtttct gggtgagcgc gattgtgccg cgccctgcga gcctgcacgg 780
cccgacggct ccatgttctt ctcgcaccac cacactcgtt ttgcccgtct ctggatcctc 840
acatggtcgg tgctgtgctg cgcttctact ttcttcacgg tcaccacctc tttagtggcc 900
atgcagcgat tccgctaccc agagcggccc atcatcttcc tgtccggttg ctacaccatg 960
gtgtcagtgg cctacattgc gggcttcgtg ctccaggagc gcgtggtgtg caacgagcgc 1020
ttctctgagg acggttatcg cacggtgggg cagggcacta agaaagaagg ctgtactata 1080
ctcttcatga tgctctactt cttcagtatg gccagctcca tctggtgggt gattctgtcc 1140

ctcacctggt tcctggcagc cggtatgaag tggggccacg cggccatcga ggccaattcg 1200
cagtacttcc acctggccgc ctgggcggtg ccggccgtca aaaccatcac catcctggcc 1260
atgggccaga tcgacggcga cctgctgagc ggcgtgtgct tcgtgggcct caacaggctg 1320
gacccgctgc gaggcttcgt gctggcgccg ctcttcgtgt acctgttcat cggcacatcc 1380
ttcctgctgg cgggcttcgt gtcactcttc cgcatccgca ccatcatgaa gcacgacggc 1440
accaagacgg agccgctgga gaggctcatg gtgcgtatcg gcgtcttctc cgtgctctac 1500
accgtaccgg ccaccatcgt catcgcctgc tacttctatg agcaggcctt ccgcgagcac 1560
tgggagcgct cgtgggtaag ccagcactgc aagagcctag ccatcccctg cccggcccac 1620
tacacgcccc gcacgtcgcc cgacttcaca gtctacatga tcaaatacct catgacgctc 1680
atcgtgggca tcacgtcggg cttctggatc tggtccggca agacgctgca ctcgtggagg 1740
aagttctaca cgcgtctcac caacagccgg catggagaga ccaccgtgtg aagcggtctc 1800
gctgctgggc gcccccctct cccaggtccg gactgcaacc gtgccctcct tcactcggga 1860
ggggggtgca ccctacggac tcctatttta tttttttaaa taaagaacag tg 1912
<210>8
<211>27
<212>DNA
<213>Artificial Sequence
<220>
<223>Designed nucleotide to act as a primer
<400>8
aatgtcttcc aagttcttcc tagtggc 27
<210>9
<211>22
<212>DNA
<213>Artificial Sequence
<220>
<223>Designed nucleotide to act as a primer
<400>9

gatgtcggaa ttgatactgg ca 22
<210>10
<211>20
<212>DNA
<213>Artificial Sequence
<220>
<223>Designed nucleotide to act as a primer
<400>10
accacagtcc atgccatcac 20
<210>11
<211>20
<212>DNA
<213>Artificial Sequence
<220>
<223>Designed nucleotide to act as a primer
<400>11
tccaccaccc tgttgctgta 20
<210>12
<211>38
<212>DNA
<213>Artificial Sequence
<220>
<223>Designed nucleotide to act as a probe
<400>12
gtcctgggag cggggctttt ttctcttgga aagaaagt 38
<210>13
<211>46
<212>DNA

<213>Artificial Sequence

<220>

<223>Designed nucleotide to act as a probe

<400>13

agagttctta gatggtaaca ggaaattttt ctcttggaaa gaaagt 46

<210>14

<211>47

<212>DNA

<213>Artificial Sequence

<220>

<223>Designed nucleotide to act as a probe

<400>14

tatttccccc ttcattctat actatctttt tctcttggaa agaaagt 47

<210>15

<211>48

<212>DNA

<213>Artificial Sequence

<220>

<223>Designed nucleotide to act as a probe

<400>15

catagaagct gaattgtgaa tagatttttt ttctcttgga aagaaagt 48

<210>16

<211>38

<212>DNA

<213>Artificial Sequence

<220>

<223>Designed nucleotide to act as a probe

<400>16

gccctgctgg ggaagggttt ttctcttgga aagaaagt 38
<210>17
<211>40
<212>DNA
<213>Artificial Sequence
<220>
<223>Designed nucleotide to act as a probe
<400>17
cctccctgct gcccaaattt ttttctcttg gaaagaaagt 40
<210>18
<211>44
<212>DNA
<213>Artificial Sequence
<220>
<223>Designed nucleotide to act as a probe
<400>18
atcccaattc ttataacagc ttctttttct cttggaaaga aagt 44
<210>19
<211>43
<212>DNA
<213>Artificial Sequence
<220>
<223>Designed nucleotide to act as a probe
<400>19
cttgttgctt gggctgagaa actttttctc ttggaaagaa agt 43
<210>20
<211>44
<212>DNA

<213>Artificial Sequence

<220>

<223>Designed nucleotide to act as a probe

<400>20

ggctcagtgt gaagaggaag gttttttctc ttggaaagaa agt 43

<210>21

<211>40

<212>DNA

<213>Artificial Sequence

<220>

<223>Designed nucleotide to act as a probe

<400>21

ccagtggagc tgccgtttgt ttttctcttg gaaagaaagt 40

<210>22

<211>49

<212>DNA

<213>Artificial Sequence

<220>

<223>Designed nucleotide to act as a probe

<400>22

cactgtactt tctataaata agcggttttt aggcatagga cccgtgtct 49

<210>23

<211>48

<212>DNA

<213>Artificial Sequence

<220>

<223>Designed nucleotide to act as a probe

<400>23

tttctaaaaa ccaaaaacca gaatttttta ggcataggac ccgtgtct 48
<210>24
<211>43
<212>DNA
<213>Artificial Sequence
<220>
<223>Designed nucleotide to act as a probe
<400>24
aaaaatggtt ccggttgcat ttttaggcat aggacccgtg tct 43
<210>25
<211>57
<212>DNA
<213>Artificial Sequence
<220>
<223>Designed nucleotide to act as a probe
<400>25
caaataataa ttataatatt aataataaac cactttttag gcataggacc cgtgtct 57
<210>26
<211>51
<212>DNA
<213>Artificial Sequence
<220>
<223>Designed nucleotide to act as a probe
<400>26
agatccacaa aataaatatt tttcgtgttt ttaggcatag gacccgtgtc t 51
<210>27
<211>50
<212>DNA

<213>Artificial Sequence

<220>

<223>Designed nucleotide to act as a probe

<400>27

ccacacagct aagttagggt atgtgttttt taggcatagg acccgtgtct 50

<210>28

<211>49

<212>DNA

<213>Artificial Sequence

<220>

<223>Designed nucleotide to act as a probe

<400>28

ttaccttctg gatgtgtacc atgtcttttt aggcatagga cccgtgtct 49

<210>29

<211>47

<212>DNA

<213>Artificial Sequence

<220>

<223>Designed nucleotide to act as a probe

<400>29

aacctaccca tcccatatga tggtttttag gcataggacc cgtgtct 47

<210>30

<211>45

<212>DNA

<213>Artificial Sequence

<220>

<223>Designed nucleotide to act as a probe

<400>30

ctaccaccct ctttcaactg gtttttaggc ataggacccg tgtct 45
<210>31
<211>47
<212>DNA
<213>Artificial Sequence
<220>
<223>Designed nucleotide to act as a probe
<400>31
ccagagaatt tacaactcat tttggttttt taggcatagg acccgtgtct 50
<210>32
<211>47
<212>DNA
<213>Artificial Sequence
<220>
<223>Designed nucleotide to act as a probe
<400>32
tcccaagacc ttttatcttg catttttagg cataggaccc gtgtct 46
<210>33
<211>49
<212>DNA
<213>Artificial Sequence
<220>
<223>Designed nucleotide to act as a probe
<400>33
aggtttgttt tgttttgttt tgtttttttt aggcatagga cccgtgtct 49
<210>34
<211>47
<212>DNA

<213>Artificial Sequence

<220>

<223>Designed nucleotide to act as a probe

<400>34

gtccttccat tgtaaattat cattttgttt ttaggcatag gacccgtgtc t 51

<210>35

<211>49

<212>DNA

<213>Artificial Sequence

<220>

<223>Designed nucleotide to act as a probe

<400>35

ttttccttga gaatatgaca ttcttttttt aggcatagga cccgtgtct 49

<210>36

<211>50

<212>DNA

<213>Artificial Sequence

<220>

<223>Designed nucleotide to act as a probe

<400>36

ggagaatgag acatgtgata tacctttttt taggcatagg acccgtgtct 50

<210>37

<211>47

<212>DNA

<213>Artificial Sequence

<220>

<223>Designed nucleotide to act as a probe

<400>37

tctgtctgca aatggaatat ttgattttta ggcataggac ccgtgtct 48
<210>38
<211>48
<212>DNA
<213>Artificial Sequence
<220>
<223>Designed nucleotide to act as a probe
<400>38
tcatgagcta ttagaatatg acggttttta ggcataggac ccgtgtct 48
<210>39
<211>48
<212>DNA
<213>Artificial Sequence
<220>
<223>Designed nucleotide to act as a probe
<400>39
aatttttaa tttctgggga cttttttta ggcataggac ccgtgtct 48
<210>40
<211>52
<212>DNA
<213>Artificial Sequence
<220>
<223>Designed nucleotide to act as a probe
<400>40
aaatcaacat cttgacataa gagttttatt tttaggcata ggacccgtgt ct 52
<210>41
<211>46
<212>DNA

<213>Artificial Sequence

<220>

<223>Designed nucleotide to act as a probe

<400>41

ggggtctttt tacaaatctg gatttttagg cataggaccc gtgtct 46

<210>42

<211>51

<212>DNA

<213>Artificial Sequence

<220>

<223>Designed nucleotide to act as a probe

<400>42

agaaaataca ggatatttca tttatgtttt ttaggcatag gacccgtgtc t 51

<210>43

<211>56

<212>DNA

<213>Artificial Sequence

<220>

<223>Designed nucleotide to act as a probe

<400>43

tactattata atttactaac caagtatccc tatttttagg cataggaccc gtgtct 56

<210>44

<211>51

<212>DNA

<213>Artificial Sequence

<220>

<223>Designed nucleotide to act as a probe

<400>44

ctgtgaatgg gattcatgta ttatttcttt ttaggcatag gacccgtgtc t 51
<210>45
<211>44
<212>DNA
<213>Artificial Sequence
<220>
<223>Designed nucleotide to act as a probe
<400>45
gctgaatggc acgcaattac tttttaggca taggacccgt gtct 44
<210>46
<211>46
<212>DNA
<213>Artificial Sequence
<220>
<223>Designed nucleotide to act as a probe
<400>46
   aggttgtctg ctctggtgca gttttttagg cataggaccc gtgtct 46
<210>47
<211>47
<212>DNA
<213>Artificial Sequence
<220>
<223>Designed nucleotide to act as a probe
<400>47
cacaacacgg aggaatcaga gagtttttag gcataggacc cgtgtct 47
<210>48
<211>45
<212>DNA

<213>Artificial Sequence

<220>

<223>Designed nucleotide to act as a probe

<400>48

gaaacgtggc cagcatcaca ttttttaggc ataggacccg tgtct 45

<210>49

<211>45

<212>DNA

<213>Artificial Sequence

<220>

<223>Designed nucleotide to act as a probe

<400>49

tgtcctacaa ccaaagggga ctttttaggc ataggacccg tgtct 45

<210>50

<211>46

<212>DNA

<213>Artificial Sequence

<220>

<223>Designed nucleotide to act as a probe

<400>50

gagctcctaa tgtttcattt cctttttagg cataggaccc gtgtct 46

<210>51

<211>46

<212>DNA

<213>Artificial Sequence

<220>

<223>Designed nucleotide to act as a probe

<400>51

agttttagga aacaaaaatc cctttttagg cataggaccc gtgtct 46
<210>52
<211>49
<212>DNA
<213>Artificial Sequence
<220>
<223>Designed nucleotide to act as a probe
<400>52
gaaaactact gctcctcaaa ataaattttt aggcatagga cccgtgtct 49
<210>53
<211>24
<212>DNA
<213>Artificial Sequence
<220>
<223>Designed nucleotide to act as a probe
<400>53
attcttgggg aaaaataaaa aata 24
<210>54
<211>20
<212>DNA
<213>Artificial Sequence
<220>
<223>Designed nucleotide to act as a probe
<400>54
gttcccaccc ccattattgc 20
<210>55
<211>28
<212>DNA

<213>Artificial Sequence

<220>

<223>Designed nucleotide to act as a probe

<400>55

caaaagaagt cttgtattac cttttcaa                                    28

<210>56

<211>29

<212>DNA

<213>Artificial Sequence

<220>

<223>Designed nucleotide to act as a probe

<400>56

catatttgag aaaaagaaaa tgtatttct                                   29

<210>57

<211>23

<212>DNA

<213>Artificial Sequence

<220>

<223>Designed nucleotide to act as a probe

<400>57

aaaatgcaga aagcaagcta gca                                         23

<210>58

<211>23

<212>DNA

<213>Artificial Sequence

<220>

<223>Designed nucleotide to act as a probe

<400>58

caaaaaatct agtgtccaga atcattg 27
<210>59
<211>19
<212>DNA
<213>Artificial Sequence
<220>
<223>Designed nucleotide to act as a probe
<400>59
tcaagccaac ctccccaaa 19
<210>60
<211>24
<212>DNA
<213>Artificial Sequence
<220>
<223>Designed nucleotide to act as a probe
<400>60
ggatttcact gtttttcctc aaat 24
<210>61
<211>25
<212>DNA
<213>Artificial Sequence
<220>
<223>Designed nucleotide to act as a probe
<400>61
taagtagtga actgttttcc aagca 25